# EUROPEAN PATENT SPECIFICATION

(11) **EP 1 358 353 B1**
(45) Date of publication and mention of the grant of the patent: **20.06.2007**
(21) Application number: 01994831.4
(22) Date of filing: 21.12.2001
(51) Int. Cl.: C12Q 1/68, C12N 15/00, C12N 5/10, C12N 9/02, C07K 16/00, C12Q 1/26, G01N 33/573, A61K 39/00, A61K 38/00, A61K 48/00

(54) **IDENTIFICATION OF GENETIC DETERMINANTS OF POLYMORPHIC CYP3A5 EXPRESSION**
IDENTIFIZIERUNG VON GENETISCHEN DETERMINANTEN DER POLYMORPHEN CYP3A5 EXPRESSION
IDENTIFICATION DES DETERMINANTS GENETIQUES DE L'EXPRESSION POLYMORPHE DE CYP3A5

(30) Priority: 28.12.2000 EP 00128627; 28.12.2000 US 258684 P; 29.12.2000 US 258952 P; 16.01.2001 EP 01100172; 18.01.2001 US 262859 P; 16.08.2001 EP 01118884; 16.08.2001 US 312825 P
(43) Date of publication of application: 05.11.2003
(73) Proprietor: Epidauros Biotechnologie AG, 82347 Bernried (DE)
(72) Inventor: WOJNOWSKI, Leszek, 80637 München (DE); HABERL, Michael, 82347 Bernried (DE); HUSTERT, Elisabeth, 82110 Germering (DE)
(74) Representative: Vossius & Partner
(86) International application number: PCT/EP2001/015290
(87) International publication number: WO 2002/053775

(56) References cited:
- WO-A-00/47771
- WO-A-98/44939
- PAULUSSEN A ET AL: "TWO LINKED MUTATIONS IN TRANSCRIPTIONAL REGULATORY ELEMENTS OF THE CYP3A5 GENE CONSTITUTE THE MAJOR GENETIC DETERMINANT OF POLYMORPHIC ACTIVITY IN HUMANS" PHARMACOGENETICS, CHAPMAN & HALL, LONDON, GB, vol. 10, no. 5, July 2000 (2000-07), pages 415-424, XP008015489 ISSN: 0960-314X
- HUSTERT E ET AL: "THE GENETIC DETERMINANTS OF THE CYP3A5 POLYMORPHISM" PHARMACOGENETICS, CHAPMAN & HALL, LONDON, GB, vol. 11, no. 9, December 2001 (2001-12), pages 773-779, XP008015487 ISSN: 0960-314X
- [Online] PMID: 12822676 Retrieved from the Internet: URL:http://www.ncbi.nlm.nih.gov/entrez/>
- PMID: 15454734 Retrieved from the Internet: URL:http://www.ncbi.nlm.nih.gov/entrez/>
- DATABASE NCBI Database accession no. AF280107
- DATABASE NCBI Database accession no. AC005020

## Description

The present invention relates to a polymorphic CYP3A5 polynucleotide. Moreover, the invention relates to vectors comprising the polynucleotides of the invention and to a host cell genetically engineered with the polynucleotide of the invention. Further, the invention relates to methods for producing a molecular variant polypeptide, methods for producing cells capable of expressing a molecular variant polypeptide and to a polypeptide encoded by the polynucleotide of the invention or which is obtainable by the method or from the cells produced by the method of the invention. Furthermore, the invention relates to an antibody which binds specifically the polypeptide of the invention. Moreover, the invention relates to a transgenic non-human animal comprising the polynucleotide or vector of the present invention. The invention also relates to a solid support comprising one or a plurality of the above mentioned polynucleotides, vectors, polypeptides, antibodies or host cells. Furthermore, methods of identifying a polymorphism, identifying and obtaining a pro-drug or drug are also encompassed by the present invention. In addition, the invention relates to methods of diagnosing a disease. Further, the invention relates to a method of detection of the polynucleotide of the invention. Furthermore, comprised by the present invention are a diagnostic and a pharmaceutical composition. Even more, the invention relates to uses of the polynucleotides, vectors, polypeptides or antibodies of the invention. Finally, the invention relates to a diagnostic kit.

The CYP3A enzymes play a particularly important role in drug metabolism. This is due to their abundant expression in the liver combined with a broad substrate spectrum. Indeed, it is estimated that CYP3A isozymes collectively comprise the largest portion of the liver CYP protein (Thummel, Annu Rev Pharmacol Toxicol 38 (1998), 389-430) and that they are involved in the metabolism of 45 % - 60 % of all currently used drugs (Li, Toxicology 104 (1995), 1-8; Evans, Science 286 (1999), 487-91). In addition to drugs, CYP3A isozymes metabolise a variety of other compounds including steroid hormones, toxins and carcinogens. For example, CYP3A isozymes metabolise aflatoxin B₁ (Wang, Biochemistry 37 (1998), 12536 - 45; Gillam, Arch Biochem Biophys 317 (1995), 374-84; Li, Cancer Res 57 (1997), 641-5), a mycotoxin strongly implicated in the etiology of liver cancer, which is a major cause of premature death in many areas of Africa and Asia (Henry, Science 286 (1999), 2453-4).

The hepatic expression and activity of CYP3A isozymes is inter-individually variable and this variability is the reason for harmful interactions frequently encountered in development and application of drugs that are CYP3A substrates. It has also been postulated that variable CYP3A expression could affect an individual's predisposition to cancers caused by environmental carcinogens which are metabolised by CYP3A: The elucidation of factors controlling an individual's CYP3A activity could permit personalised dose adjustments in therapies with its substrates and also lead to the identification of sub-populations at increased risk for several common cancers. However, despite considerable efforts, our understanding of factors governing CYP3A activity and expression is limited. There are several reasons for this: An average human liver may express products of up to four *CYP3A* genes (Gellner, Pharmacogenetics 11 (2001), 111 - 121), but their respective contributions to the hepatic CYP3A pool are still a matter of debate. The differentiation between the individual CYP3A proteins by enzymatic methods has proven difficult due to overlapping substrate specifities and due to the considerable effect of reconstitution conditions on their catalytic activities. RNA and protein analysis indicate that CYP3A4 forms the bulk of the hepatic CYP3A protein and its expression is highly variable (Thummel, Annu Rev Pharmacol Toxicol 38 (1998), 389-430). Less well understood are the contributions of the other *CYP3A* genes. CYP3A5 is widely considered the second most important CYP3A protein in the liver, but the available data are conflicting, since its expression has been reported to be present in 10 % to 97 % of human livers (Aoyama, J Biol Chem 264 (1989), 10388-95; Wrighton, Mol Pharmacol 38 (1990), 207-13; Schuetz, Pharmacogenetics 4 (1994), 11-20; Jounaidi, Biochem Biophys Res Commun 221 (1996), 466-70; Boobis, Br J Clin Pharmacol 42 (1996), 81-9). The possible reasons for these discrepancies include small sample sizes, interethnic differences and poor specificity of probes used to measure CYP3A5 expression. The third CYP3A, CYP3A7, was originally described in the human fetal liver where it accounts for about 50 % of the total CYP protein (Wrighton, Biochem Pharmacol 37 (1988), 3053-5). More recent studies indicate constitutive or induced expression of CYP3A7 in adult human livers, but its quantification has been hampered by the lack of specific antibodies. Similarly, no protein expression data are available for the recently identified fourth member of the family, CYP3A43 (Gellner, Pharmacogenetics 11 (2001), 111 - 121).

Clinical studies indicate that a major portion of the inter-individual CYP3A variability is caused by genetic factors (Ozdemir, Pharmacogenetics 10 (2000), 373-88), but the identities of the latter remain unknown. In respect of CYP3A5, a protein variant (Thr398Asn) has been found in 2 out of 5 individuals deficient in CYP3A5 expression (Jounaidi, Biochem Biophys Res Commun 221 (1996),466-70), but its significance has not been verified on a larger number of liver samples and in functional studies. In addition, a haplotype consisting of two linked polymorphisms has been described in the 5' flanking region of the CYP3A5 gene which is associated with increased expression and activity of the gene (Paulussen, Pharmacogenetics 10 (2000), 415-24). However, only a small sample set (n=29) was analysed for the genotype and the phenotype. Moreover, the single nucleotide polymorphisms (SNPs) which have been disclosed in said document are not suitable for a reliable prediction of CYP3A5 dysfunction and/or dysregulation and the problems caused thereby. This document does not suggest the existence of further haplotypes.

Thus, improved means and methods for diagnosing and treating a variety of diseases and disorders based on dysfunctions or dysregulations of drug metabolism were not available yet but are nevertheless highly desirable. Thus, the technical problem underlying the present invention is to comply with the above specified needs.

The solution to this technical problem is achieved by providing the embodiments characterized in the claims.

Accordingly, the present invention relates to a polynucleotide comprising a polynucleotide selected from the group consisting of:
(a) a polynucleotide hybridising under stringent conditions to a CYP3A5 gene and having the nucleic acid sequence of SEQ ID NO: 112;
(b) a polynucleotide hybridising under stringent conditions to a CYP3A5 gene and encoding a polypeptide having the amino acid sequence of SEQ ID NO: 141; and
(c) a polynucleotide hybridizing under stringent conditions to a part of a CYP3A5 gene comprising SEQ ID NO:111, wherein said polynucleotide is having an additional nucleotide at a position corresponding to position 27131/27132 of the CYP3A5 gene as defined by the joined sequences of Accession No: AF280107.1, wherein position 166220 has been numbered +1 and position 174832 has been numbered +8613, and Accession No: AC005020.2, wherein position 27341 has been numbered +8614.

In the context of the present invention the term "polynucleotides" or the term "polypeptides" refers to different variants of a polynucleotide or polypeptide. Said variants comprise a reference or wild type sequence of the polynucleotides or polypeptides of the invention as well as variants which differ therefrom in structure or composition. Reference or wild type sequences for the polynucleotides are Accession No: AF280107.1 and AC005020.2. Reference or wild type sequence for the polypeptides of the invention is Accession No: NP_000768.1. The differences in structure or composition usually occur by way of nucleotide or amino acid substitution(s), addition(s) and/or deletion(s). Said nucleotide substitution(s), addition(s) or deletion(s) result(s) in one or more changes of the corresponding amino acid(s) of the polypeptide of the invention. The polynucleotides and polypeptides of the present invention are characterized as being associated with a CYP3A5 dysfunction or dysregulation. Accordingly, said dysfunctions or dysregulations referred to in the present invention cause a disease or disorder or a prevalence for said disease or disorder.

The polynucleotides of the invention include polynucleotides that have at least 70%, preferably at least 75%, at least 80%, at least 85%, at least 90% or at least 95% sequence identity to a CYP3A5 gene, wherein said polynucleotide is having at least one additional nucleotide at a position corresponding to position 27131/27132 of the CYP3A5 gene as defined by the joined sequences of Accession No: AF280107.1, wherein position 166220 has been numbered +1 and position 174832 has been numbered +8613, and Accession No: AC005020.2, wherein position 27341 has been numbered +8614.

The term "hybridizing" as used herein refers to polynucleotides which are capable of hybridizing to the polynucleotides of the invention or parts thereof which are associated with a CYP3A5 dysfunction or dysregulation. Thus, said hybridizing polynucleotides are also associated with said dysfunctions and dysregulations. Therefore, said polynucleotides may be useful as probes in Northern or Southern Blot analysis of RNA or DNA preparations, respectively, or can be used as oligonucleotide primers in PCR analysis dependent on their respective size. Also comprised by the invention are hybridizing polynucleotides which are useful for analysing DNA-Protein interactions via, e.g., electrophoretic mobility shift analysis (EMSA). Preferably, said hybridizing polynucleotides comprise at least 10, more preferably at least 15 nucleotides in length while a hybridizing polynucleotide of the present invention to be used as a probe preferably comprises at least 100, more preferably at least 200, or most preferably at least 500 nucleotides in length.
It is well known in the art how to perform hybridization experiments with nucleic acid molecules, i.e. the person skilled in the art knows what hybridization conditions s/he has to use in accordance with the present invention. Such hybridization conditions are referred to in standard text , books such as Molecular Cloning A Laboratory Manual, Cold Spring Harbor Laboratory (1989) N.Y. Preferred in accordance with the present inventions are polynucleotides which are capable of hybridizing to the polynucleotides of the invention or parts thereof which are associated with a CYP3A5 dysfunction or dysregulation under stringent hybridization conditions, i.e. which do not cross hybridize to unrelated polynucleotides such as polynucleotides encoding a polypeptide different from the CYP3A5 polypeptides of the invention.
Nucleic acid hybridization will be affected by such conditions as salt concentration, temperature, or organic solvents, in addition to the base composition, length of the complementary strands and the number of nucleotide base mismatches between the hybridizing nucleic acids, as will be readily appreciated by those skilled in the art. Stringent temperature conditions will generally include temeratures in excess of 30°C, typically 37°C, and preferably in excess of 45°C. Stringent salt conditions will ordinarily be less than 1000 mM, typically less than 500 mM and preferably less than 200 mM. However, the combination of parameters is much more important than the measure of any single parameter. See, e.g., Wetmur and Davidson, 1968. Probe sequences may also hybridize specifically to duplex DNA under certain conditions to form triplex or higher order DNA complexes. The preparation of such probes and suitable hybridization conditions are well known in the art.

The term "percent sequence identity" or "identical" in the context of nucleic acid sequences refers to the residues in the two sequences which are the same when aligned for maximum correspondence. The length of sequence identity comparison may be over a strech of at least nine nucleotides, usually at least 20 nucleotides, more usually at least 24. nucleotides, typically at least 28 nucleotides, more typically at least 32 nucleotides, and preferably at least 36 nucleotides or more nucleotides. There are a number of different algorithms known in the art which can be used to measure nucleotide sequence identity. For instance, polynucleotide sequences can be compared using Fasta, a program in GCG Version 6.6, Fasta.provides alignments and percent sequence identity of the regions of the best overlap between the query and the search sequence (Pearson, 1980). For instance, percent sequence identity between nucleic acid sequences can be determined using Fasta with its default parameters (a word size of 6 and the NOPAMfactor for the scoring matrix) as provided in GCG Version 6.1.

The term "corresponding" as used herein means that a position is not only determined by the number of the preceding nucleotides and amino acids, respectively. The position of a given nucleotide or amino acid in accordance with the present invention which may be deleted, substituted or comprise one or more additional nucleotide(s) may vary due to deletions or additional nucleotides or amino acids elsewhere in the gene or the polypeptide. Thus, under a "corresponding position" in accordance with the present invention it is to be understood that nucleotides or amino acids may differ in the indicated number but may still have similar neighboring nucleotides or amino acids. Said nucleotides or amino acids which may be exchanged, deleted or comprise additional nucleotides or amino acids are also comprised by the term "corresponding position". Said nucleotides or amino acids may for instance together with their neighbors form sequences which may be involved in the regulation of gene expression, stability of the corresponding RNA or RNA editing, as well as encode functional domains or motifs of the protein of the invention.

By, e.g., "position 3709/3710" it is meant that said polynucleotide comprises one or more additional nucleotide(s) which are inserted between positions 3709 and position 3710 of the corresponding wild type version of said polynucleotide. The same applies mutatis mutandis to all other position numbers referred to in the above embodiment which are drafted in the same format, i.e. two consecutive position numbers separated by a slash (/). By, e.g., "position 7424 to 7427" is meant that said polynucleotide comprises one or more deleted nucleotides which are deleted between positions 7424 and position 7427 of the corresponding wild type version of said polynucleotide and/or one or more additional nucleotide(s) which are inserted between positions 7424 and position 7427 of the corresponding wild type version of said polynucleotide. The same applies mutatis mutandis to all other position numbers referred to in the above embodiment which are drafted in the same format.

The numbering of the polymorphisms refers to the aligned and joined genomic sequences AF280107.1 and AC005020.2, wherein the T at position 174832 (which has been numbered +8613) of the sequence AF280107.1 refers to position 27340 of the sequence AC005020.2. The nucleotide A at position 27341 of the sequence AC005020.2 has been numbered +8614. Numbering of polymorphisms to a position corresponding to a position up to +8613 refers to the genomic sequence AF280107.1, numbering of polymophisms to a position corresponding to position +8614 and greater refer to the genomic sequence AC005020.2.

In accordance with the present invention, the mode and population distribution of genetic variations in the CYP3A5 gene has been analyzed by sequence analysis of relevant regions of the human gene from many different individuals. It is a well known fact that genomic DNA of individuals, which harbor the individual genetic makeup of all genes, including the CYP3A5 gene, can easily be purified from individual blood samples. These individual DNA samples are then used for the analysis of the sequence composition of the alleles of the CYP3A5 gene that are present in the individual which provided the blood sample. The sequence analysis was carried out by PCR amplification of relevant regions of said genes, subsequent purification of the PCR products, followed by automated DNA sequencing with established methods (e.g. ABI dyeterminator cycle sequencing).
One important parameter that had to be considered in the attempt to determine the individual genotypes and identify novel variants of the CYP3A5 gene by direct DNA-sequencing of PCR-products from human blood genomic DNA is the fact that each human harbors (usually, with very few abnormal exceptions) two gene copies of each autosomal gene (diploidy). Because of that, great care had to be taken in the evaluation of the sequences to be able to identify unambiguously not only homozygous sequence variations but also heterozygous variations. The details of the different steps in the identification and characterization of novel polymorphisms in the CYP3A5 gene (homozygous and heterozygous) are described in the Examples below.

Over the past 20 years, genetic heterogeneity has been increasingly recognized as a significant source of variation in drug response. Many scientific communications (Meyer, Ann. Rev. Pharmacol. Toxicol. 37 (1997), 269-296 and West, J. Clin. Pharmacol. 37 (1997), 635-648) have clearly shown that some drugs work better or may even be highly toxic in some patients than in others and that these variations in patient's responses to drugs can be related to molecular basis. This "pharmacogenomic" concept spots correlations between responses to drugs and genetic profiles of patient's (Marshall, Nature Biotechnology, 15 (1997), 954-957; Marshall, Nature Biotechnology, 15 (1997), 1249-1252). In this context of population variability with regard to drug therapy, pharmacogenomics has been proposed as a tool useful in the identification and selection of patients which can respond to a particular drug without side effects. This identification/selection can be based upon molecular diagnosis of genetic polymorphisms by genotyping DNA from leukocytes in the blood of patient, for example, and characterization of disease (Bertz, Clin. Pharmacokinet. 32 (1997), 210-256; Engel, J. Chromatogra. B. Biomed. Appl. 678 (1996), 93-103). For the founders of health care, such as health maintenance organizations in the US and government public health services in many European countries, this pharmacogenomics approach can represent a way of both improving health care and reducing overheads because there is a large cost to unnecessary drugs, ineffective drugs and drugs with side effects.

The mutations in the variant gene of the invention may sometimes result in amino acid deletion(s), insertion(s) and in particular in substitution(s) either alone or in combination. It is of course also possible to genetically engineer such mutations in wild type genes or other mutant forms. Methods for introducing such modifications in the DNA sequence of said genes are well known to the person skilled in the art; see, e.g., Sambrook, Molecular Cloning A Laboratory Manual, Cold Spring Harbor Laboratory (1989) N.Y.

For the investigation of the nature of the alterations in the amino acid sequence of the polypeptide of the invention software programs may be used such as RASMOL that are obtainable from the Internet. Furthermore, folding simulations and computer redesign of structural motifs can be performed using other appropriate computer programs (Olszewski, Proteins 25 (1996), 286-299; Hoffman, Comput. Appl. Biosci. 11 (1995), 675-679). Computers can be used for the conformational and energetic analysis of detailed protein models (Monge, J. Mol. Biol. 247 (1995), 995-1012; Renouf, Adv. Exp. Med. Biol. 376 (1995), 37-45). These analysis can be used for the identification of the influence of a particular mutation on binding and/or processing of drugs.

Usually, said amino acid deletion, addition or substitution in the amino acid sequence of the protein encoded by the polynucleotide of the invention is due to one or more nucleotide substitution, insertion or deletion, or any combinations thereof. Preferably, an insertion may result in amino acid substitutions of TYD to YL. (with the period meaning termination) at a position corresponding to position 346 to 348 of the CYP3A5 polypeptide (Accession No: NP_000768.1).

The mutations in the CYP3A5 gene detected in accordance with the present invention are listed in Table 2A-E. The methods of the mutation analysis followed standard protocols and are described in detail in the Examples. In general such methods are to be used in accordance with the present invention for evaluating the phenotypic spectrum as well as the overlapping clinical characteristics of diseases or conditions related to dysfunctions and diseases related to the drug metabolism. Advantageously, the characterization of said mutants may form the basis of the development of improved drugs. Said methods encompass for example haplotype analysis, single-strand conformation polymorphism analysis (SSCA), PCR and direct sequencing, or TaqMan® analysis. On the basis of thorough clinical characterization of many patients the phenotypes can then be correlated to these mutations as well as to mutations that had been described earlier, for example in Jounaidi, Biochem Biophys Res Commun, 221, pp. 466-470, 1996.

Also comprised by the polynucleotides referred to in Table 2A-E are polynucleotides which comprise at least two, preferably at least three, of the polynucleotides specified hereinabove, i.e. polynucleotides having a nucleotide sequence which contains at least two, preferably three of the mutations listed in the tables below. Thus, the haplotype can be characterized by at least two, preferably three of said mutations in the CYP3A5 locus. Moreover, the polynucleotide of the present invention may further comprise at least one nucleotide deletion, addition and/or substitution other than those specified hereinabove, for example those described in the prior art; e.g., in Jounaidi, Biochem Biophys Res Commun, 221, pp. 466-470, 1996, in Paulussen, Pharmacogenetics 10, pp. 415-424, 2000, in Kuehl, 2001, Nature Genetics 27: 383-391, or in Chou, 2001, Drug Metab Dispos 29: 1205-1209. This allows the study of synergistic effects of said mutations in the CYP3A5 gene and/or a polypeptide encoded by said polynucleotide on the pharmacological profile of drugs in patients who bear such mutant forms of the gene or similar mutant forms that can be mimicked by the above described proteins. It is expected that the analysis of said synergistic effects provides deeper insights into the onset of dysfunctions or diseases related to drug metabolism as described supra. From said deeper insight the development of diagnostic and pharmaceutical compositions related to dysfunctions or diseases related to drug metabolism will greatly benefit.

Moreover, it has been surprisingly found that the so called positive predictive power for CYP3A5 dysfunctions or dysregulations can be significantly increased based on the polynucleotides of the present invention and thus allows a reliable prediction in contrast to positive predictive power based on the prior art. The increased CYP3A5 protein expression in all except one liver samples (17/18) identified in accordance with the present invention and described in detail in the examples below co-segregates with a haplotype which consists of at least three variants (ch-v-021, ch-v-026, ch-v-015) with distinct locations within or upstream of the gene locus. Genotyping these three variants has in no case led to the generation of false-positive predictions resulting in an estimated positive predictive power for the 3-variant genotype of about 99.95 %. This is in striking contrast to the positive predictive power determined for the haplotype described by Paulussen, Pharmacogenetics 10, pp. 415-424, 2000 which is about 65 %. Moreover, based on the polynucleotides of the invention and as described in the examples below, it has been found that the SNPs described by Paulussen, Pharmacogenetics 10, pp. 415-424, 2000 are located in contrary to what is reported in said document approximately 20 kb upstream of the transcriptional start site of the CYP3A5 gene in a sequence 5' to a CYP3A5 pseudogene locus.
Therefore, the haplotypes characterized on the basis of the polynucleotides of the present invention fulfil the criteria expected from a reliable marker of CYP3A5 expression. As is evident to the person skilled in the art, the genetic knowledge deduced from the present invention can now be used to exactly and reliably characterize the genotype of a patient. Advantageously, diseases or a prevalence for a disease which are associated with CYP3A5 dysfunction or dysregulation, such as cancer can be predicted and preventive or therapeutical measures can be applied accordingly. Moreover in accordance with the foregoing, in cases where a given drug takes an unusual effect, a suitable individual therapy can be designed based on the knowledge of the individual genetic makeup of a subject with respect to the polynucleotides of the invention and improved therapeutics can be developed as will be further discussed below.
Finally, the polynucleotides and polypeptides referred to in accordance with the present invention are also useful as forensic markers, which improve the identification of subjects which have been murdered or killed by, for example, a crime of violence or any other violence and can not be identified by the well known conventional forensic methods. The application of forensic methods based on the detection of the polymorphisms comprised by the polynucleotides of this invention in the genome of a subject are particularly well suited in cases where a (dead) body is disfigured in a severe manner such that identification by other body characteristics such as the features of the face is not possible. This is the case, for example, for corpse found in water which are usually entirely disfigured. Advantageously methods which are based on the provision of the polynucleotides of the invention merely require a minimal amount of tissues or cells in order to be carried out. Said tissues or cells may be blood droplets, hair roots, epidermal scales, saliva droplets, sperms etc. Since only such a minimal amount of tissues or cells are required for the identification of a subject, the polymorphisms comprised by the polynucleotides of this invention can be also used as forensic markers in order to prove someone guilt of a crime, such as a violation or a ravishment. Moreover, the polymorphisms comprised by the polynucleotides of this invention can be used to proof paternity. In accordance with the forensic methods referred to herein the presence or absence of the polynucleotides of the invention is determined and compared with a reference sample which is unambiguously derived from the subject to be identified. The forensic methods which require detection of the presence or absence of the polynucleotides of the invention in a sample of a subject the polymorphisms comprised by the polynucleotides of this invention can be for example PCR-based techniques which are particularly well suited in cases where only a minimal amount of tissues or cells are available as forensic samples. On the other hand, where enough tissue or cells are available, hybridization based techniques may be performed in order to detect the presence or absence of a polynucleotide of this invention. These techniques are well known by the person skilled in the art and can be adopted to the individual purposes referred to herein without further ado. In conclusion, thanks to the present invention forensic means which allow improved and reliable predictions as regards the aforementioned aspects are now available.

The term "cancer" used herein is very well known and characterized in the art. Several variants of cancer exist and are comprised by said term as meant in accordance with the invention. For a detailed list of symptoms which are indicative for cancer it is referred to text book knowledge, e.g. Pschyrembel.

In a further embodiment the present invention relates to a polynucleotide which is DNA or RNA.

The polynucleotide of the invention may be, e.g., DNA, cDNA, genomic DNA, RNA or synthetically produced DNA or RNA or a recombinantly produced chimeric nucleic acid molecule comprising any of those polynucleotides either alone or in combination. Preferably said polynucleotide is part of a vector, particularly plasmids, cosmids, viruses and bacteriophages used conventionally in genetic engineering that comprise a polynucleotide of the invention. Such vectors may comprise further genes such as marker genes which allow for the selection of said vector in a suitable host cell and under suitable conditions.

Also disclosed herein is a gene comprising the polynucleotide of the invention.

It is well known in the art that genes comprise structural elements which encode an amino acid sequence as well as regulatory elements which are involved in the regulation of the expression of said genes. Structural elements are represented by exons which may either encode an amino acid sequence or which may encode for RNA which is not encoding an amino acid sequence but is nevertheless involved in RNA function, e.g. by regulating the stability of the RNA or the nuclear export of the RNA.
Regulatory elements of a gene may comprise promoter elements or enhancer elements both of which could be involved in transcriptional control of gene expression. It is very well known in the art that a promoter is to be found upstream of the structural elements of a gene. Regulatory elements such as enhancer elements, however, may be found distributed over the entire locus of the gene. Said elements could be reside, e.g., in introns, regions of genomic DNA which separate the exons of a gene. Said introns may comprise further regulatory elements which are required for proper gene expression. Introns are usually transcribed together with the exons of a gene resulting in a nascent RNA transcript which contains both, exon and intron sequences. The intron encoded RNA sequences are usually removed by a process known as RNA splicing. However, said process also requires regulatory sequences present on a RNA transcript, said regulatory sequences may be encoded by the introns.
In addition, besides their function in transcriptional control and control of proper RNA processing and/or stability, regulatory elements of a gene could be also involved in the control of genetic stability of a gene locus. Said elements control, e.g., recombination events or serve to maintain a certain structure of the DNA or the arrangement of DNA in a chromosome.
Therefore, polymorphisms can occur in exons of a gene which encode an amino acid sequence as discussed supra as well as in regulatory regions which are involved in the above discussed process. The analysis of the nucleotide sequence of a gene locus in its entirety including, e.g., introns is in light of the above desirable. It has been found based on the polymorphisms comprised by the polynucleotides of the present invention that the mechanism of the increased expression of CYP3A5 protein in most Caucasians livers described in the examples below may involve enhanced transcription and stabilisation of the gene's transcripts.

Therefore, in a gene a nucleotide deletion, addition and/or substitution may result in altered expression of the variant gene compared to the corresponding wild type gene.

In another embodiment the present invention relates to a vector comprising the polynucleotide of the invention:
Said vector may be, for example, a phage, plasmid, viral or retroviral vector. Retroviral vectors may be replication competent or replication defective. In the latter case, viral propagation generally will occur only in complementing host/cells.
   The polynucleotide of the invention may be joined to a vector containing selectable markers for propagation in a host. Generally, a plasmid vector is introduced in a precipitate such as a calcium phosphate precipitate, or in a complex with a charged lipid or in carbon-based clusters: Should the vector be a virus, it may be packaged in vitro using an appropriate packaging cell line prior to application to host cells.
In a more preferred embodiment of the vector of the invention the polynucleotide is operatively linked to expression control sequences allowing expression in prokaryotic or eukaryotic cells or isolated fractions thereof.
Expression of said polynucleotide comprises transcription of the polynucleotide, preferably into a translatable mRNA. Regulatory elements ensuring expression in eukaryotic cells, preferably mammalian cells, are well known to those skilled in the art. They usually comprise regulatory sequences ensuring initiation of transcription and optionally poly-A signals ensuring termination of transcription and stabilization of the transcript. Additional regulatory elements may include transcriptional as well as translational enhancers. Possible regulatory elements permitting expression in prokaryotic host cells comprise, e.g., the *lac, trp* or *tac* promoter in *E*. *coli,* and examples for regulatory elements permitting expression in eukaryotic host cells are the *AOX1* or *GAL1* promoter in yeast or the CMV-, SV40- , RSV-promoter (Rous sarcoma virus), CMV-enhancer, SV40-enhancer or a globin intron in mammalian and other animal cells. Beside elements which are responsible for the initiation of transcription such regulatory elements may also comprise transcription termination signals, such as the SV40-poly-A site or the tk-poly-A site, downstream of the polynucleotide. In this context, suitable expression vectors are known in the art such as Okayama-Berg cDNA expression vector pcDV1 (Pharmacia), pCDM8, pRc/CMV, pcDNA1, pcDNA3 (In-vitrogene), pSPORT1 (GIBCO BRL). Preferably, said vector is an expression vector and/or a gene transfer or targeting vector. Expression vectors derived from viruses such as retroviruses, vaccinia virus, adeno-associated virus, herpes viruses, or bovine papilloma virus, may be used for delivery of the polynucleotides or vector of the invention into targeted cell population. Methods which are well known to those skilled in the art can be used to construct recombinant viral vectors; see, for example, the techniques described in Sambrook, Molecular Cloning A Laboratory Manual, Cold Spring Harbor Laboratory (1989) N.Y. and Ausubel, Current Protocols in Molecular Biology, Green Publishing Associates and Wiley Interscience, N.Y. (1994). Alternatively, the polynucleotides and vectors of the invention can be reconstituted into liposomes for delivery to target cells.

The term "isolated fractions thereof" refers to fractions of eukaryotic or prokaryotic cells or tissues comprising said cells which are capable of transcribing or transcribing and translating RNA from the vector of the invention. Said fractions comprise proteins which are required for transcrition of RNA or transcription of RNA and translation of said RNA into a polypeptide. Said isolated fractions may be, e.g., nuclear and cytoplasmic fractions of eukaryotic cells such as of reticulocytes.

The present invention furthermore relates to a host cell genetically engineered with the polynucleotide or the vector of the invention.

Said host cell may be a prokaryotic or eukaryotic cell; see supra. The polynucleotide or vector of the invention which is present in the host cell may either be integrated into the genome of the host cell or it may be maintained extrachromosomally. In this respect, it is also to be understood that the recombinant DNA molecule of the invention can be used for "gene targeting" and/or "gene replacement", for restoring a mutant gene or for creating a mutant gene via homologous recombination; see for example Mouellic, Proc. Natl. Acad. Sci. USA, 87 (1990), 4712-4716; Joyner, Gene Targeting, A Practical Approach, Oxford University Press.
The host cell can be any prokaryotic or eukaryotic cell, such as a bacterial, insect, fungal, plant, animal or human cell. Preferred fungal cells are, for example, those of the genus Saccharomyces, in particular those of the species S. cerevisiae. The term "prokaryotic" is meant to include all bacteria which can be transformed or transfected with a polynucleotide for the expression of a variant polypeptide of the invention. Prokaryotic hosts may include gram negative as well as gram positive bacteria such as, for example, *E. coli, S. typhimurium, Serratia marcescens* and *Bacillus subtilis.* A polynucleotide coding for a mutant form of variant polypeptides of the invention can be used to transform or transfect the host using any of the techniques commonly known to those of ordinary skill in the art.

Methods for preparing fused, operably linked genes and expressing them in bacteria or animal cells are well-known in the art (Sambrook, supra). The genetic constructs and methods described therein can be utilized for expression of variant polypeptides of the invention in, e.g., prokaryotic hosts. In general, expression vectors containing promoter sequences which facilitate the efficient transcription of the inserted polynucleotide are used in connection with the host. The expression vector typically contains an origin of replication, a promoter, and a terminator, as well as specific genes which are capable of providing phenotypic selection of the transformed cells. The transformed prokaryotic hosts can be grown in fermentors and cultured according to techniques known in the art to achieve optimal cell growth. The proteins of the invention can then be isolated from the grown medium, cellular lysates, or cellular membrane fractions. The isolation and purification of the microbially or otherwise expressed polypeptides of the invention may be by any conventional means such as, for example, preparative chromatographic separations and immunological separations such as those involving the use of monoclonal or polyclonal antibodies.

Thus, in a further embodiment the invention relates to a method for producing a molecular variant polypeptide comprising culturing the above described host cell; and recovering said protein from the culture.

In another embodiment the present invention relates to a method for producing cells capable of expressing a molecular variant polypeptide comprising genetically engineering cells with the polynucleotide of the invention or the vector of the invention.

The cells obtainable by the method of the invention can be used, for example, to test drugs according to the methods described in D. L. Spector, R. D. Goldman, L. A. Leinwand, Cells, a Lab manual, CSH Press 1998. Furthermore, the cells can be used to study known drugs and unknown derivatives thereof for their ability to complement the deficiency caused by mutations in the CYP3A5 gene. For these embodiments the host cells preferably lack a wild type allele, preferably both alleles of the CYP3A5 gene and/or have at least one mutated from thereof. Ideally, the gene comprising an allele as comprised by the polynucleotides of the invention could be introduced into the wild type locus by homologous replacement. Alternatively, strong overexpression of a mutated allele over the normal allele and comparison with a recombinant cell line overexpressing the normal allele at a similar level may be used as a screening and analysis system. The cells obtainable by the above-described method may also be used for the screening methods referred to herein below.

Furthermore, the invention relates to a polypeptide encoded by the polynucleotide of the invention or obtainable by the method described above or from cells produced by the method described above.

In this context it is also understood that the variant polypeptide of the invention can be further modified by conventional methods known in the art. By providing said variant proteins according to the present invention it is also possible to determine the portions relevant for their biological activity or inhibition of the same. The terms "polypeptide" and "protein" as used herein are exchangeable. Moreover, what is comprised by said terms is standard textbook knowledge.

The present invention furthermore relates to an antibody which binds specifically to the polypeptide of the invention.

Advantageously, the antibody specifically recognizes or binds an epitope containing one or more amino acid substitution(s) as defined above. Antibodies against the variant polypeptides of the invention can be prepared by well known, methods using a purified protein according to the invention or a (synthetic) fragment derived therefrom as an antigen. Monoclonal antibodies can be prepared, for example, by the techniques as originally described in Köhler and Milstein, Nature 256 (1975), 495, and Galfré, Meth. Enzymol. 73 (1981), 3, which comprise the fusion of mouse myeloma cells to spleen cells derived from immunized mammals. In a preferred embodiment of the invention; said antibody is a monoclonal antibody, a polyclonal antibody, a single chain antibody, human or humanized antibody, primatized, chimerized or fragment thereof that specifically binds said peptide or polypeptide also including bispecific antibody, synthetic antibody, antibody fragment, such as Fab, Fv or scFv fragments etc., or a chemically modified derivative of any of these. Furthermore, antibodies or fragments thereof to the aforementioned polypeptides can be obtained by using methods which are described, e.g., in Harlow and Lane "Antibodies, A Laboratory Manual", CSH Press, Cold Spring Harbor, 1988. These antibodies can be used, for example, for the immunoprecipitation and immunolocalization of the variant polypeptides of the invention as well as for the monitoring of the presence of said variant polypeptides, for example, in recombinant organisms, and for the identification of compounds interacting with the proteins according to the invention. For example, surface plasmon resonance as employed in the BIAcore system can be used to increase the efficiency of phage antibodies which bind to an epitope of the protein of the invention (Schier, Human Antibodies Hybridomas 7 (1996), 97-105; Malmborg, J. Immunol. Methods 183 (1995), 7-13).

In a preferred embodiment the antibody of the present invention specifically recognizes an epitope containing one or more amino acid substitution(s) resulting from a nucleotide exchange as defined supra.

Antibodies which specifically recognize modified amino acids such as phospho-Tyrosine residues are well known in the art. Similarly, in accordance with the present invention antibodies which specifically recognize even a single amino acid exchange in an epitope may be generated by the well known methods described supra.

In light of the foregoing, in a more preferred embodiment the antibody of the present invention is monoclonal or polyclonal.

The invention also relates to a transgenic non-human animal comprising the polynucleotide of the invention or the vector of the invention as described supra.

The present invention also encompasses a method for the production of a transgenic non-human animal comprising introduction of a polynucleotide or vector of the invention into a germ cell, an embryonic cell, stem cell or an egg or a cell derived therefrom. The non-human animal can be used in accordance with the method of the invention described below and may be a non-transgenic healthy animal, or may have a disease or disorder, preferably a disease caused by at least one mutation in the gene of the invention. Such transgenic animals are well suited for, e.g., pharmacological studies of drugs in connection with variant forms of the above described variant polypeptides since these polypeptides or at least their functional domains are conserved between species in higher eukaryotes, particularly in mammals. Production of transgenic embryos and screening of those can be performed, e.g., as described by A. L. Joyner Ed., Gene Targeting, A Practical Approach (1993), Oxford University Press. The DNA of the embryos can be analyzed using, e.g., Southern blots with an appropriate probe or based on PCR techniques.

A transgenic non-human animal in accordance with the invention may be a transgenic mouse, rat, hamster, dog, monkey, rabbit, pig, frog, nematode such as Caenorhabditis elegans, fruitfly such as Drosophila melanogaster or fish such as torpedo fish or zebrafish comprising a polynucleotide or vector of the invention or obtained by the method described above, preferably wherein said polynucleotide or vector is stably integrated into the genome of said non-human animal, preferably such that the presence of said polynucleotide or vector leads to the expression of the variant polypeptide of the invention. It may comprise one or several copies of the same or different polynucleotides or genes of the invention. This animal has numerous utilities, including as a research model for cancer or any other disease caused by as dysfunction or dysregulation of the polynucleotides or polypeptides of the invention research and therefore, presents a novel and valuable animal in the development of therapies, treatment, etc. for cancer diseases or any other disease caused by as dysfunction or dysregulation of the polynucleotides or polypeptides of the invention. Accordingly, in this instance, the mammal is preferably a laboratory animal such as a mouse or rat.

Thus, in a preferred embodiment the transgenic non-human animal of the invention is a mouse, a rat or a zebrafish.

Numerous reports revealed that said animals are particularly well suited as model organisms for the investigation of the drug metabolism and its deficiencies or cancer. Advantageously, transgenic animals can be easily created using said model organisms, due to the availability of various suitable techniques well known in the art.

The invention also relates to a solid support comprising one or a plurality of the polynucleotide, the vector, the polypeptide, the antibody or the host cell of the invention in immobilized form.

The term "solid support" as used herein refers to a flexible or non-flexible support that is suitable for carrying said immobilized targets. Said solid support may be homogenous or inhomogeneous. For example, said solid support may consist of different materials having the same or different properties with respect to flexibility and immobilization, for instance, or said solid support may consist of one material exhibiting a plurality of properties also comprising flexibility and immobilization properties. Said solid support may comprise glass-polypropylene- or silicon-chips, membranes, oligonucleotide-conjugated beads or bead arrays.

The term "immobilized" means that the molecular species of interest is fixed to a solid support, preferably covalently linked thereto. This covalent linkage can be achieved by different means depending on the molecular nature of the molecular species. Moreover, the molecular species may be also fixed on the solid support by electrostatic forces, hydrophobic or hydrophilic interactions or Van-der-Waals forces. The above described physico-chemical interactions typically occur in interactions between molecules. For example, biotinylated polypeptides may be fixed on a avidin-coated solid support due to interactions of the above described types. Further, polypeptides such as antibodies, may be fixed on an antibody coated solid support. Moreover, the immobilization is dependent on the chemical properties of the solid support. For example, the nucleic acid molecules can be immobilized on a membrane by standard techniques such as UV-crosslinking or heat.

In a preferred embodiment of the invention said solid support is a membrane, a glass- or poylpropylene- or silicon-chip, are oligonucleotide-conjugated beads or a bead array, which is assembled on an optical filter substrate.

Moreover, the present invention relates to an in vitro method for identifying a polymorphism said method comprising the step of identifying a polymorphism by comparing the nucleic acid sequence of the polynucleotide of the invention obtained from a subgroup of individuals, wherein the subgroup has no prevalence for cancer with the nucleic acid sequence of the polynucleotide of the invention obtained from a plurality of subgroups of individuals, wherein at least one or more further subgroup(s) do have prevalence for cancer.

The term "prevalence" as used herein means that individuals are susceptible for one or more disease(s) which are associated with CYP3A5 dysfuntion or dysregulation or could already have one or more of said disease(s). Thereby, one CYP3A5 associated disease can be used to determine the susceptibility for another CYP3A5 associated disease, e.g. impaired drug metabolism may be indicative for a prevalence for, e.g. cancer. Moreover, symptoms which are indicative for a prevalence for developing said diseases are very well known in the art and have been sufficiently described in standard textbooks such as Pschyrembel.

Advantageously, polymorphisms according to the present invention which are associated with CYP3A5 dysfunction or dysregulation or one or more disease(s) based thereon should be enriched in subgroups of individuals which have a prevalence for said diseases versus subgroups which have no prevalence for said diseases. Thus, the above described method allows the rapid and reliable detection of polymorphisms which are indicative for one or more CYP3A5 associated disease(s) or a susceptibility therefor. Advantageously, due to the phenotypic preselection a large number of individuals having no prevalence might be screened for polymorphisms in general. Thereby, a reference sequences comprising polymorphisms which do not correlate to one or more CYP3A5 associated disease(s) can be obtained. Based on said reference sequences it is possible to efficiently and reliably determine the relevant polymorphisms.

In a further embodiment the present invention relates to a method for identifying and obtaining a pro-drug or a drug capable of modulating the activity of a molecular variant of a CYP3A5 polypeptide comprising the steps of:
(a) contacting the polypeptide, the solid support of the invention, a cell expressing a molecular variant gene comprising a polynucleotide of the invention or the vector of the invention in the presence of components capable of providing a detectable signal in response to drug activity with a compound to be screened for pro-drug or drug activity; and
(b) detecting the presence or absence of a signal or increase or decrease of a signal generated from the pro-drug or the drug activity, wherein the absence, presence, increase or decrease of the signal is indicative for a putative pro-drug or drug.

The term "compound" in a method of the invention includes a single substance or a plurality of substances which may or may not be identical.

Said compound(s) may be chemically synthesized or produced via microbial fermentation but can also be comprised in, for example, samples, e.g., cell extracts from, e.g., plants, animals or microorganisms. Furthermore, said compounds may be known in the art but hitherto not known to be useful as an inhibitor, respectively. The plurality of compounds may be, e.g., added to the culture medium or injected into a cell or non-human animal of the invention.
If a sample containing (a) compound(s) is identified in the method of the invention, then it is either possible to isolate the compound from the original sample identified as containing the compound in question or one can further subdivide the original sample, for example, if it consists of a plurality of different compounds, so as to reduce the number of different substances per sample and repeat the method with the subdivisions of the original sample. It can then be determined whether said sample or compound displays the desired properties, for example, by the methods described herein or in the literature (Spector et al., Cells manual; see supra). Depending on the complexity of the samples, the steps described above can be performed several times, preferably until the sample identified according to the method of the invention only comprises a limited number of or only one substance(s). Preferably said sample comprises substances of similar chemical and/or physical properties, and most preferably said substances are identical. The methods of the present invention can be easily performed and designed by the person skilled in the art, for example in accordance with other cell based assays described in the prior art or by using and modifying the methods as described herein. Furthermore, the person skilled in the art will readily recognize which further compounds may be used in order to perform the methods of the invention, for example, enzymes, if necessary, that convert a certain compound into a precursor. Such adaptation of the method of the invention is well within the skill of the person skilled in the art and can be performed without undue experimentation.

Compounds which can be used in accordance with the present invention include peptides, proteins, nucleic acids, antibodies, small organic compounds, ligands, peptidomimetics, PNAs and the like. Said compounds may act as agonists or antagonists of the invention. Said compounds can also be functional derivatives or analogues of known drugs. Methods for the preparation of chemical derivatives and analogues are well known to those skilled in the art and are described in, for example, Beilstein, Handbook of Organic Chemistry, Springer edition New York Inc., 175 Fifth Avenue, New York, N.Y. 10010 U.S.A. and Organic Synthesis, Wiley, New York, USA. Furthermore, said derivatives and analogues can be tested for their effects according to methods known in the art or as described. Furthermore, peptide mimetics and/or computer aided design of appropriate drug derivatives and analogues can be used, for example, according to the methods described below. Such analogs comprise molecules may have as the basis structure of known CYP3A5 substrates and/or inhibitors and/or modulators; see infra.
Appropriate computer programs can be used for the identification of interactive sites of a putative inhibitor and the polypeptides of the invention by computer assistant searches for complementary structural motifs (Fassina, lmmunomethods 5 (1994), 114-120). Further appropriate computer systems for the computer aided design of protein and peptides are described in the prior art, for example, in Berry, Biochem. Soc. Trans. 22 (1994), 1033-1036; Wodak, Ann. N. Y. Acad. Sci. 501 (1987), 1-13; Pabo, Biochemistry 25 (1986), 5987-5991. The results obtained from the above-described computer analysis can be used in combination with the method of the invention for, e.g., optimizing known inhibitors, analogs, antagonists or agonists. Appropriate peptidomimetics and other inhibitors can also be identified by the synthesis of peptidomimetic combinatorial libraries through successive chemical modification and testing the resulting compounds, e.g., according to the methods described herein. Methods for the generation and use of peptidomimetic combinatorial libraries are described in the prior art, for example in Ostresh, Methods in Enzymology 267 (1996), 220-234 and Domer, Bioorg. Med. Chem. 4 (1996), 709-715. Furthermore, the three-dimensional and/or crystallographic structure of said compounds and the polypeptides of the invention can be used for the design of peptidomimetic drugs (Rose, Biochemistry 35 (1996), 12933-12944; Rutenber, Bioorg. Med. Chem. 4 (1996), 1545-1558). It is very well known how to obtain said compounds, e.g. by chemical or biochemical standard techniques. Thus, also comprised by the method of the invention are means of making or producing said compounds. In summary, the present invention provides methods for identifying and obtaining compounds which can be used in specific doses for the treatment of specific forms of CYP3A5 associated diseases, e.g. dysfunctions of the drug metabolism or cancer.

The above definitions apply mutatis mutandis to all of the methods described in the following.

Also disclosed herein is a method for identifying and obtaining an inhibitor of the activity of a molecular variant of a CYP3A5 polypeptide comprising the steps of:
(a) contacting the protein, the solid support of the invention or a cell expressing a molecular variant gene comprising a polynucleotide or the gene of the invention in the presence of components capable of providing a detectable signal in response to drug activity with a compound to be screened for inhibiting activity; and
(b) detecting the presence or absence of a signal or increase or decrease of a signal generated from the inhibiting activity, wherein the absence or decrease of the signal is indicative for a putative inhibitor.

In a preferred embodiment of the method of the invention said cell is a cell, obtained by the method of the invention or can be obtained from the transgenic non-human animal as described supra.

In a still further embodiment the present invention relates to a method of identifying and obtaining a pro-drug or drug capable of modulating the activity of a molecular variant of a CYP3A5 polypeptide comprising the steps of:
(a) contacting the host cell, the cell obtained by the method of the invention, the polypeptide or the solid support of the invention with the first molecule known to be bound by a CYP3A5 polypeptide to form a first complex of said polypeptide and said first molecule;
(b) contacting said first complex with a compound to be screened, and
(c) measuring whether said compound displaces said first molecule from said first complex.

Advantageously, in said method said measuring step comprises measuring the formation of a second complex of said protein and said inhibitor candidate. Preferably, said measuring step comprises measuring the amount of said first molecule that is not bound to said protein.

In a particularly preferred embodiment of the above-described method of said first molecule is a agonist or antagonist or a substrate and/or a inhibitor and/or a modulator of the polypeptide of the invention, e.g., with a radioactive or fluorescent label.

Further disclosed herein is a method of identifying and obtaining an inhibitor capable of modulating the activity of a molecular variant of a CYP3A5 polypeptide comprising the steps of:
(a) contacting the host cell or the cell obtained by the method of the invention, the protein or the solid support of the invention with the first molecule known to be bound by a CYP3A5 polypeptide to form a first complex of said protein and said first molecule;
(b) contacting said first complex with a compound to be screened, and
(c) measuring whether said compound displaces said first molecule from said first complex.

In a preferred embodiment of the method of the invention said measuring step comprises measuring the formation of a second complex of said protein and said compound.

In another preferred embodiment of the method of the invention said measuring step comprises measuring the amount of said first molecule that is not bound to said protein.

In a more preferred embodiment of the method of the invention said first molecule is labeled.

Additionally disclosed herein is a method for the production of a pharmaceutical composition comprising the steps of the method as described supra; and the further step of formulating the compound identified and obtained or a derivative thereof in a pharmaceutically acceptable form.

The therapeutically useful compounds identified according to the methods of the invention can be formulated and administered to a patient as discussed above. For uses and therapeutic doses determined to be appropriate by one skilled in the art and for definitions of the term "pharmaceutical composition" see infra.

Furthermore, herein is disclosed a method for the preparation of a pharmaceutical composition comprising the steps of the above-described methods; and formulating a drug or pro-drug in the form suitable for therapeutic application and preventing or ameliorating the disorder of the subject diagnosed in the method of the invention.

Drugs or pro-drugs after their *in vivo* administration are metabolized in order to be eliminated either by excretion or by metabolism to one or more active or inactive metabolites (Meyer, J. Pharmacokinet. Biopharm. 24 (1996), 449-459). Thus, rather than using the actual compound or inhibitor identified and obtained in accordance with the methods of the present invention a corresponding formulation as a pro-drug can be used which is converted into its active in the patient. Precautionary measures that may be taken for the application of pro-drugs and drugs are described in the literature; see, for review, Ozama, J. Toxicol. Sci. 21 (1996), 323-329).

In a preferred embodiment of the method of the present invention said drug or prodrug is a derivative of a medicament as defined hereinafter.

The present invention also relates to
an in vitro method of diagnosing liver cancer or a susceptibility to liver cancer related to the presence of a molecular variant of a CYP3A5 gene comprising determining the presence of
   (a) a polynucleotide selected from the group consisting of:
      (i) a polynucleotide as described herein; and
      (ii) a polynucleotide having an additional nucleotide at a position corresponding to position 27131/27132 of the CYP3A5 gene as defined by the joined sequences of Accession No: AF280107.1, wherein position 166220 has been numbered +1 and position 174832 has been numbered +8613, and Accession No: AC005020.2, wherein position 27341 has been numbered +8614; or
   (b) the polypeptide as described herein in a sample from a subject.

In accordance with this embodiment of the present invention, the method of testing the status of a disorder or susceptibility to such a disorder can be effected by using a polynucleotide or nucleic acid of the invention, e.g., in the form of a Southern or Northern blot or *in situ* analysis. Said nucleic acid sequence may hybridize to a coding region of either of the genes or to a non-coding region, e.g. intron. In the case that a complementary sequence is employed in the method of the invention, said nucleic acid molecule can again be used in Northern blots. Additionally, said testing can be done in conjunction with an actual blocking, e.g., of the transcription of the gene and thus is expected to have therapeutic relevance. Furthermore, a primer or oligonucleotide can also be used for hybridizing to one of the above mentioned CYP3A5 gene or corresponding mRNAs. The nucleic acids used for hybridization can, of course, be conveniently labeled by incorporating or attaching, e.g., a radioactive or other marker. Such markers are well known in the art. The labeling of said nucleic acid molecules can be effected by conventional methods.
Additionally, the presence or expression of variant CYP3A5 gene can be monitored by using a primer pair that specifically hybridizes to either of the corresponding nucleic acid sequences and by carrying out a PCR reaction according to standard procedures. Specific hybridization of the above mentioned probes or primers preferably occurs at stringent hybridization conditions. The term "stringent hybridization conditions" is well known in the art; see, for example, Sambrook et al., "Molecular Cloning, A Laboratory Manual" second ed., CSH Press, Cold Spring Harbor, 1989; "Nucleic Acid Hybridisation, A Practical Approach", Hames and Higgins eds., IRL Press, Oxford, 1985. Furthermore, the mRNA, cRNA, cDNA or genomic DNA obtained from the subject may be sequenced to identify mutations which may be characteristic fingerprints of mutations in the polynucleotide or the gene of the invention. The present invention further comprises methods wherein such a fingerprint may be generated by RFLPs of DNA or RNA obtained from the subject, optionally the DNA or RNA may be amplified prior to analysis, the methods of which are well known in the art. RNA fingerprints may be performed by, for example, digesting an RNA sample obtained from the subject with a suitable RNA-Enzyme, for example RNase T₁, RNase T₂ or the like or a ribozyme and, for example, electrophoretically separating and detecting the RNA fragments as described above.
Further modifications of the above-mentioned embodiment of the invention can be easily devised by the person skilled in the art, without any undue experimentation from this disclosure; see, e.g., the examples. An additional embodiment of the present invention relates to a method wherein said determination is effected by employing an antibody of the invention or fragment thereof. The antibody used in the method of the invention may be labeled with detectable tags such as a histidine flags or a biotin molecule.

Also disclosed herein is a method of diagnosing a disorder related to the presence of a molecular variant of a CYP3A5 gene or susceptibility to such a disorder comprising determining the presence of a polypeptide or the antibody of the invention in a sample from a subject.

In a preferred embodiment of the present invention, the above described method is comprising PCR, ligase chain reaction, restriction digestion, direct sequencing, nucleic acid amplification techniques, hybridization techniques or immunoassays.
Said techniques are very well known in the art.

Moreover, the invention relates to a method of detection of the polynucleotide or the gene of the invention in a sample comprising the steps of
(a) contacting the solid support described supra with the sample under conditions allowing interaction of the polynucleotide of the invention with the immobilized targets on a solid support and;
(b) determining the binding of said polynucleotide or said gene to said immobilized targets on a solid support.

The invention also relates to an in vitro method for diagnosing a disease comprising the steps of the method described supra, wherein binding of said polynucleotide or gene to said immobilized targets on said solid support is indicative for the presence or the absence of said disease or a prevalence for said disease.

The invention furthermore relates to a diagnostic composition comprising the polynucleotide, the vector, the polypeptide or the antibody of the invention.

In addition, the invention relates to a pharmaceutical composition comprising the polynucleotide, the vector, the polypeptide or the antibody of the invention.

These pharmaceutical compositions comprising, e.g., the antibody may conveniently be administered by any of the routes conventionally used for drug administration, for instance, orally, topically, parenterally or by inhalation. Acceptable salts comprise acetate, methylester, HCl, sulfate, chloride and the like. The compounds may be administered in conventional dosage forms prepared by combining the drugs with standard pharmaceutical carriers according to conventional procedures. These procedures may involve mixing, granulating and compressing or dissolving the ingredients as appropriate to the desired preparation. It will be appreciated that the form and character of the pharmaceutically acceptable character or diluent is dictated by the amount of active ingredient with which it is to be combined, the route of administration and other well-known variables. The carrier(s) must be "acceptable" in the sense of being compatible with the other ingredients of the formulation and not deleterious to the recipient thereof. The pharmaceutical carrier employed may be, for example, either a solid or liquid. Exemplary of solid carriers are lactose, terra alba, sucrose, talc, gelatin, agar, pectin, acacia, magnesium stearate, stearic acid and the like. Exemplary of liquid carriers are phosphate buffered saline solution, syrup, oil such as peanut oil and olive oil, water, emulsions, various types of wetting agents, sterile solutions and the like. Similarly, the carrier or diluent may include time delay material well known to the art, such as glyceryl mono-stearate or glyceryl distearate alone or with a wax.
The dosage regimen will be determined by the attending physician and other clinical factors; preferably in accordance with any one of the above described methods. As is well known in the medical arts, dosages for any one patient depends upon many factors, including the patient's size, body surface area, age, the particular compound to be administered, sex, time and route of administration, general health, and other drugs being administered concurrently. Progress can be monitored by periodic assessment.
Furthermore, the use of pharmaceutical compositions which comprise antisense-oligonucleotides which specifically hybridize to RNA encoding mutated versions of the polynucleotitde or gene according to the invention or which comprise antibodies specifically recognizing a mutated polypeptide of the invention but not or not substantially the functional wild-type form is conceivable in cases in which the concentration of the mutated form in the cells should be reduced.
Thanks to the present invention the particular drug selection, dosage regimen and corresponding patients to be treated can be determined in accordance with the present invention. The dosing recommendations will be indicated in product labeling by allowing the prescriber to anticipate dose adjustments depending on the considered patient group, with information that avoids prescribing the wrong drug to the wrong patients at the wrong dose.

A gene encoding a functional and expressible polypeptide of the invention can be introduced into the cells which in turn produce the protein of interest. Gene therapy, which is based on introducing therapeutic genes into cells by *ex-vivo* or *in-vivo* techniques is one of the most important applications of gene transfer. Suitable vectors and methods for *in-vitro* or *in-vivo* gene therapy are described in the literature and are known to the person skilled in the art; see, e.g., Giordano, Nature Medicine 2 (1996), 534-539; Schaper, Circ. Res. 79 (1996), 911-919; Anderson, Science 256 (1992), 808-813; Isner, Lancet 348 (1996), 370-374; Muhlhauser, Circ. Res. 77 (1995), 1077-1086; Wang, Nature Medicine 2 (1996), 714-716; WO94/29469; WO 97/00957 or Schaper, Current Opinion in Biotechnology 7 (1996), 635-640, and references cited therein. The gene may be designed for direct introduction or for introduction via liposomes, or viral vectors (e.g. adenoviral, retroviral) into the cell. Preferably, said cell is a germ line cell, embryonic cell, or egg cell or derived therefrom, most preferably said cell is a stem cell.As is evident from the above, it is preferred that in the use of the invention the nucleic acid sequence is operatively linked to regulatory elements allowing for the expression and/or targeting of the polypeptides of the invention to specific cells. Suitable gene delivery systems that can be employed in accordance with the invention may include liposomes, receptor-mediated delivery systems, naked DNA, and viral vectors such as herpes viruses, retroviruses, adenoviruses, and adeno-associated viruses, among others. Delivery of nucleic acids to a specific site in the body for gene therapy may also be accomplished using a biolistic delivery system, such as that described by Williams (Proc. Natl. Acad. Sci. USA 88 (1991), 2726-2729). Standard methods for transfecting cells with recombinant DNA are well known to those skilled in the art of molecular biology, see, e.g., WO 94/29469; see also supra. Gene therapy may be carried out by directly administering the recombinant DNA molecule or vector of the invention to a patient or by transfecting cells with the polynucleotide or vector of the invention ex vivo and infusing the transfected cells into the patient.
A polynucleotide comprising SEQ ID No: 104 and a polypeptide comprising SEQ ID No: 145 have already been described in Jounaidi *et al.* (Jounaidi, Biochem Biophys Res Commun 221 (1996), 466-70). However, Jounaidi *et al.* have merely disclosed the respective amino acid and nucleotide sequences without making any suggestion towards the pharmaceutic and diagnostic value of said polynucleotide or polypeptide, in particular for those disorders and diseases referred to infra.

In another embodiment the present invention encompasses the use of a polynucleotide as defined herein for the preparation of a diagnostic composition for diagnosing a disease in a subject having a genome comprising a variant allele of the CYP3A5 gene, wherein said allele is having an A at a position corresponding to position 6986 of the CYP3A5 gene as defined by the joined sequences of Accession No: AF280107.1, wherein position 166220 has been numbered +1 and position 174832 has been numbered +8613, and Accession No: AC005020.2, wherein position 27341 has been numbered +8614.

The definitions of the terms referred to in this specification apply mutatis mutandis to the aforementioned use.
The term "subject" inter alia refers to animals. Preferably, said animals belong to the animal species referred to above. Moreover, the term "subject" encompasses humans. The humans in accordance with the use of the present invention are selected from all existing ethnical groups and subgroups, e.g. Caucasians, African Americans or Asians. However, particular well suited for the use of the invention are African Americans for which it could be demonstrated that diagnosing a disease or a prevalence for a disease based on monitoring the presence or absence of an CYP3A5 allele having at a position corresponding to position 6986 of the CYP3A5 gene (Accession No: AF280107.1, wherein position 166220 has been numbered +1 and position 174832 has been numbered +8613, and Accession No: AC005020.2, wherein position 27341 has been numbered +8614) an A results in a false positive prediction for CYP3A5 expression in a considerable number of subjects. This allele of CYP3A5 has been described in detail in Kuehl, 2001, Nature Genetics 27: 383-391 as CYP3A5*1 allele. The CYP3A5*1 allele is characterized by the presence of an A at position 22893 of the CYP3A5 nucleic acid sequence referred to in Kuehl, loc.cit. The allelic frequency of said allele is particularly high in African Americans although it is also present in other ethnical groups or subgroups. However, it was found in accordance with the present invention that the CYP3A5 expression of subjects for which a false positive result was obtained in diagnostic studies based on the CYP3A5*1 allele could be correctly predicted by further diagnosing the presence or absence of a polynucleotide as defined under (a) to (d) in accordance with the use of the present invention. For example, a polynucleotide having an additional nucleotide at a position corresponding to position 27131/27132 of the CYP3A5 gene as defined supra has been found in accordance with this invention to be present in approximately 10% of the African Americans resulting in a frameshift mutation in exon 11. The present invention provides means and methods to distinguish between the haplotype resulting in improved expression of CYP3A5 comprising the polymorphism(s) of the CYP3A5*1 allele and the haplotype resulting in decreased expression, wherein said haplotype as set forth above comprises in addition to the polymorphism(s) of the CYP3A5*1 allele co-segregating polymorphisms comprised by a polynucleotide referred to under (a) to (d) supra. Thus, based on the aforementioned use of the present invention a reliable diagnosis of the CYP3A5 activity of a subject is achieved.

The definitions of the terms referred to in this specification apply mutatis mutandis to the aforementioned use.
In accordance with the present invention it could also be shown that the polymorphism(s) constituting the CYP3A5*1 and the CYP3A5*6 allele as described in Kuehl, loc.cit., co-segregate in a considerable number of subjects and thereby constitute another haplotype resulting in decreased CYP3A5 expression. It has been shown that the CYP3A5*6 allele results in inappropriate splicing of exon 7 of CYP3A5, a frameshift and a premature termination at position 184 of the CYP3A5 protein. Consequently, false positive result as regards the expression level of CYP3A5 in a subject can be obtained in diagnostic studies based on the CYP3A5*1 allele. Said false positive results, however, can be avoided according to the use of this invention by further diagnosing the presence or absence of the polymorphism(s) of the CYP3A5*6 allele. Thus, based on the aforementioned use of the present invention a reliable diagnosis of the CYP3A5 activity of a subject is achieved.

In light with the foregoing, in a preferred embodiment of the aforementioned use said subject is an African American.
As has been discussed above, the number of subjects which are diagnosed false positive is due to the high allelic frequency of CYP3A5 alleles such as those comprising a polynucleotide as defined under (a) to (d) above resulting in a frame shift mutation or those comprised by the CYP3A5*6 allele. Said allelic frequency is particularly high within the group of African Americans. In accordance with the present invention it has been found that the CYP3A5*6 allele is present in about 13.3% of the African Americans. Thus within this ethnic group the problems emerging from a wrong prediction of CYP3A5 expression are more severe than for other ethnical groups.

Finally, the present invention relates to a diagnostic kit for detection of a polymorphism comprising the polynucleotide, the vector, the polypeptide, the antibody, the host cell, the transgenic non-human animal or the solid support of the invention.

The kit of the invention may contain further ingredients such as selection markers and components for selective media suitable for the generation of transgenic cells and animals. The kit of the invention can be used for carrying out a method of the invention and could be, inter alia, employed in a variety of applications, e.g., in the diagnostic field or as research tool. The parts of the kit of the invention can be packaged individually in vials or in combination in suitable containers or multicontainer units. Manufacture of the kit follows preferably standard procedures which are known to the person skilled in the art. The kit may be used for methods for detecting expression of a mutant form of the polypeptides, genes or polynucleotides in accordance with any one of the above-described methods of the invention, employing, for example, : immunoassay techniques such as radioimmunoassay or enzymeimmunoassay or preferably nucleic acid hybridization and/or amplification techniques such as those described herein before and in the Examples as well as pharmacokinetic studies when using non-human transgenic animals of the invention.

The figures illustrate the invention.

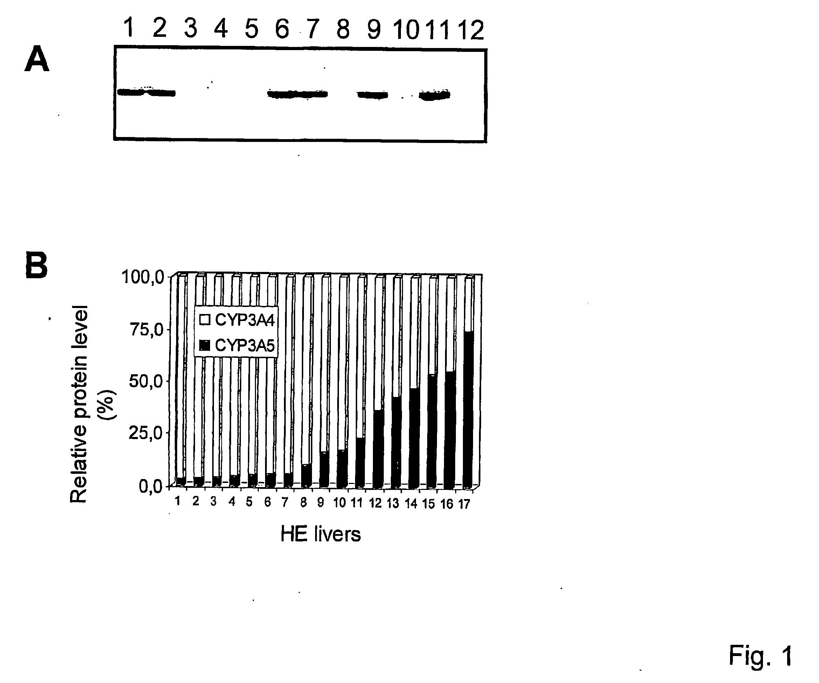

**Figure 1: *A*.** Western blot analysis of CYP3A5 protein expression in microsomes prepared from 6 LE (low expressing) and 6 HE (high expressing) Caucasian livers. ***B***. The relative contributions of CYP3A5 and CYP3A4 to the combined CYP3A5/CYP3A4 protein pool in 17 HE livers as determined by Western blot.

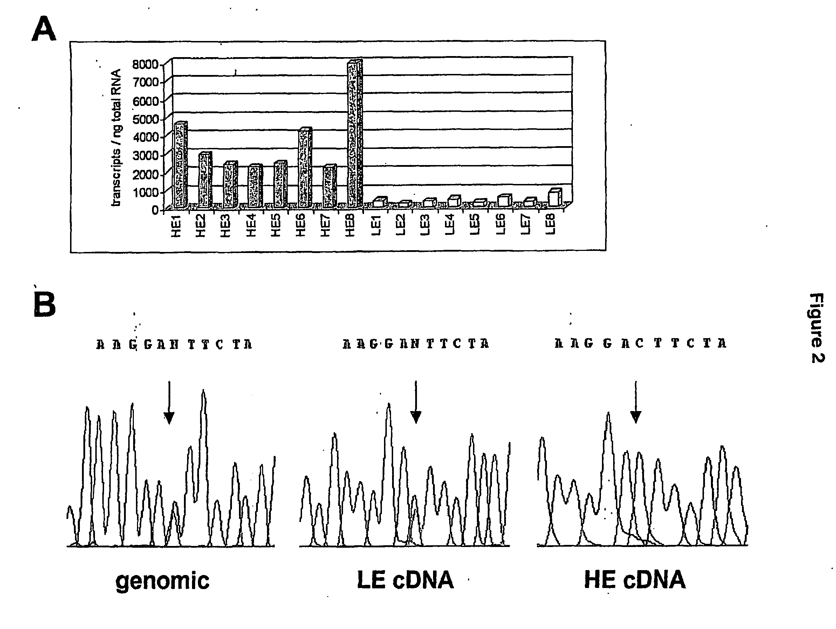

**Figure 2:** Expression levels and the allelic source of *CYP3A5* transcripts in LE and HE Caucasian livers. ***A.*** TaqMan analysis of *CYP3A5* mRNA in 8 LE (low expressing, white bars) and 8 HE (high expressing, grey bars) liver samples. ***B.*** Sequences of a portion of the 3'-UTR of *CYP3A5* in samples heterozygous for variant ch-v-015 (Table 2A). Templates used for PCR were genomic DNA (left panel), cDNA from a LE liver (middle panel) and cDNA from an HE liver (right panel).

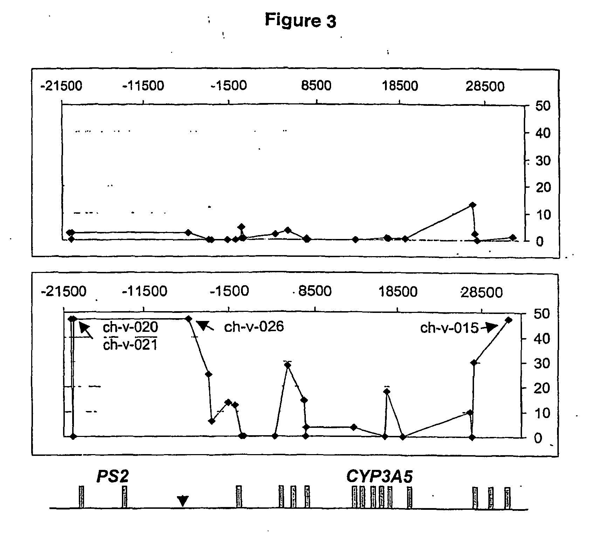

**Figure 3:** Allelic frequencies of *CYP3A5* genetic variants in Caucasians. ***A.*** in DNA samples derived from 8-168 LE individuals ***B.*** in DNA samples derived from 7-18 HE individuals. The bars at the bottom of the figure indicate schematically the localisation of the pseudoexons *PS2* exon 1 and 2 and exons 1 - 13 of the *CYP3A5* gene. The arrowhead marks the duplication boundary (Gellner, Pharmacogenetics 11 (2001), 111 - 121).

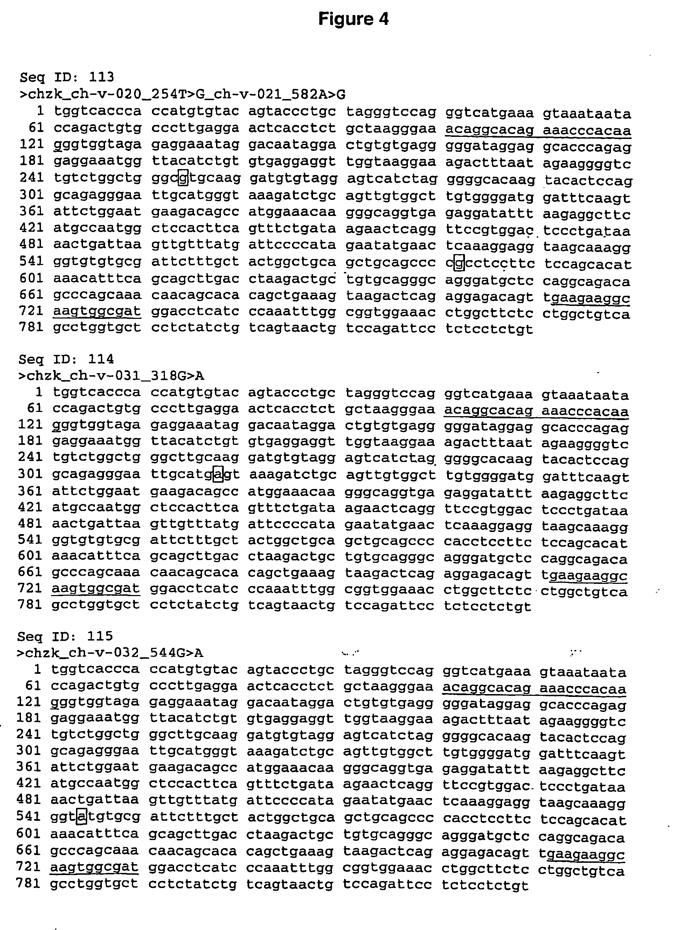

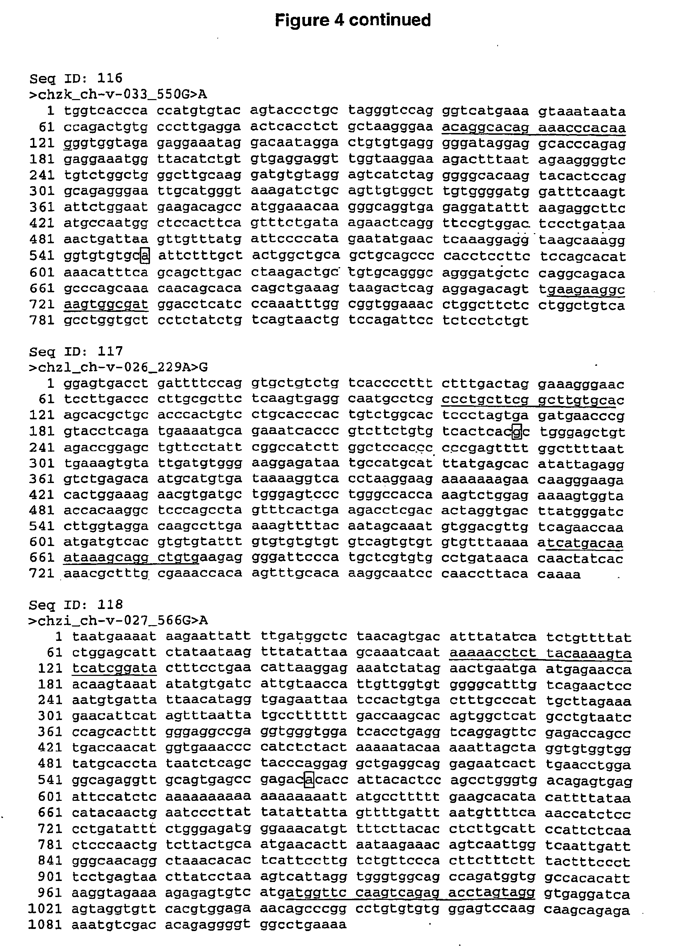

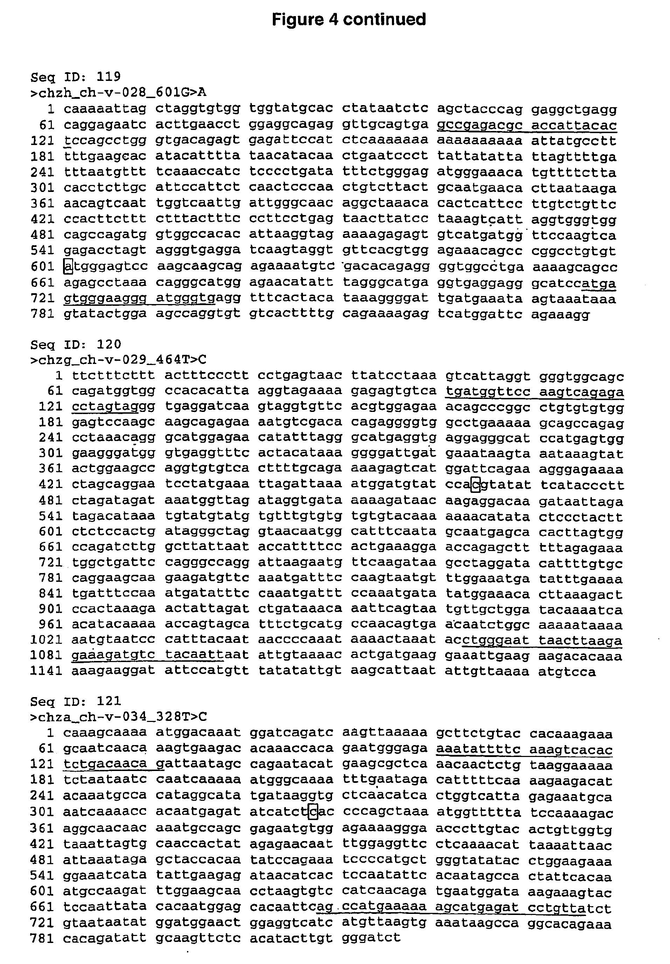

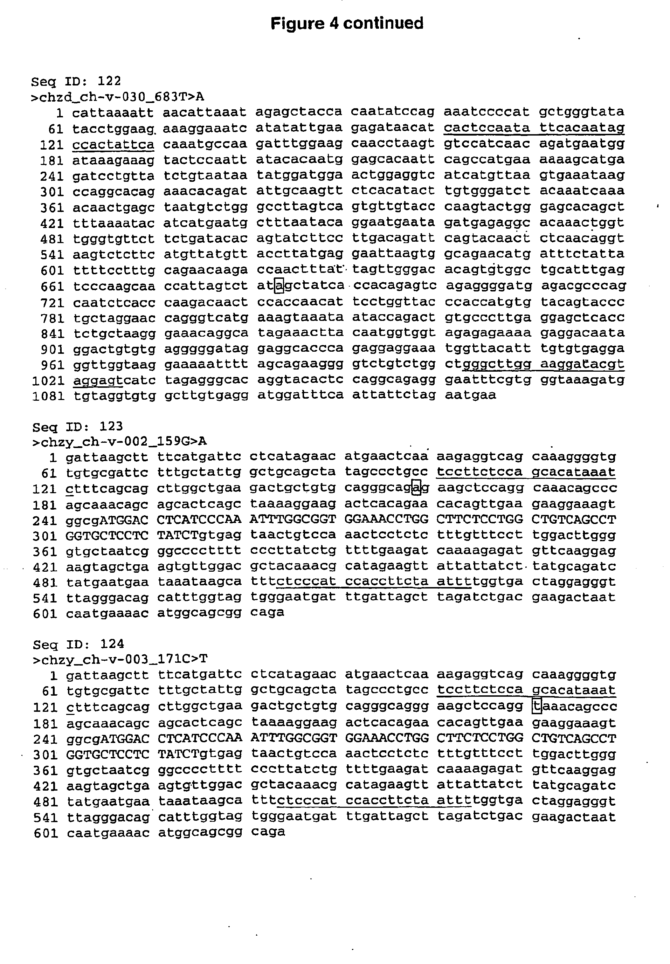

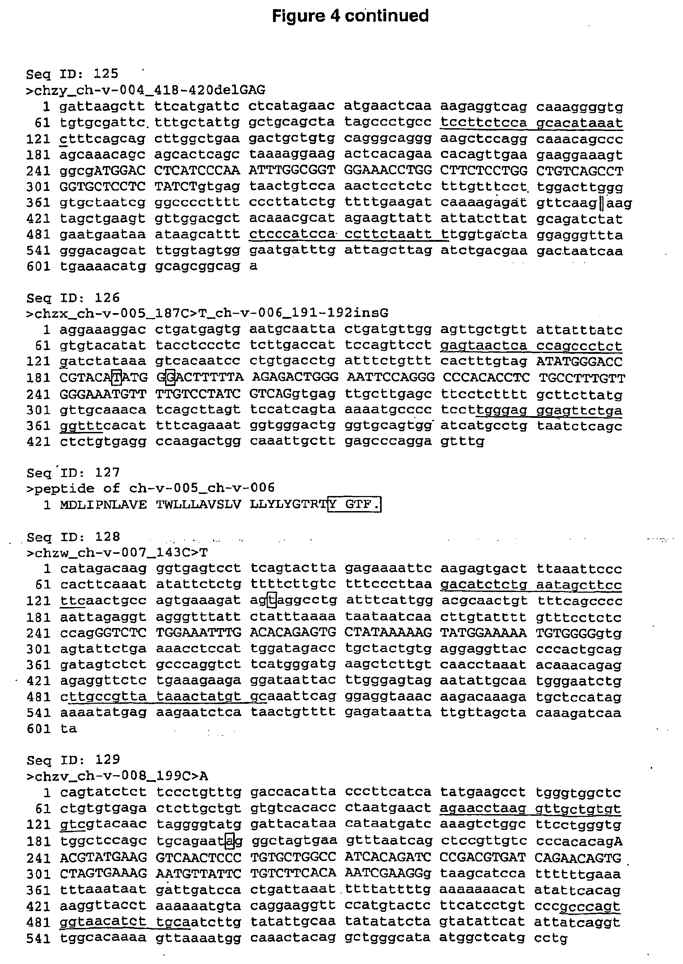

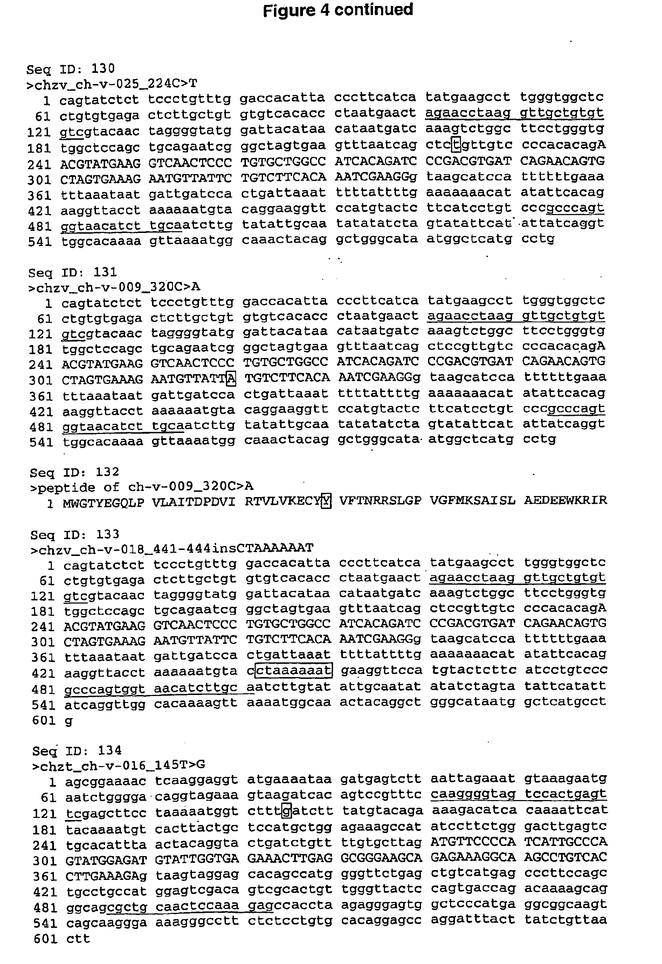

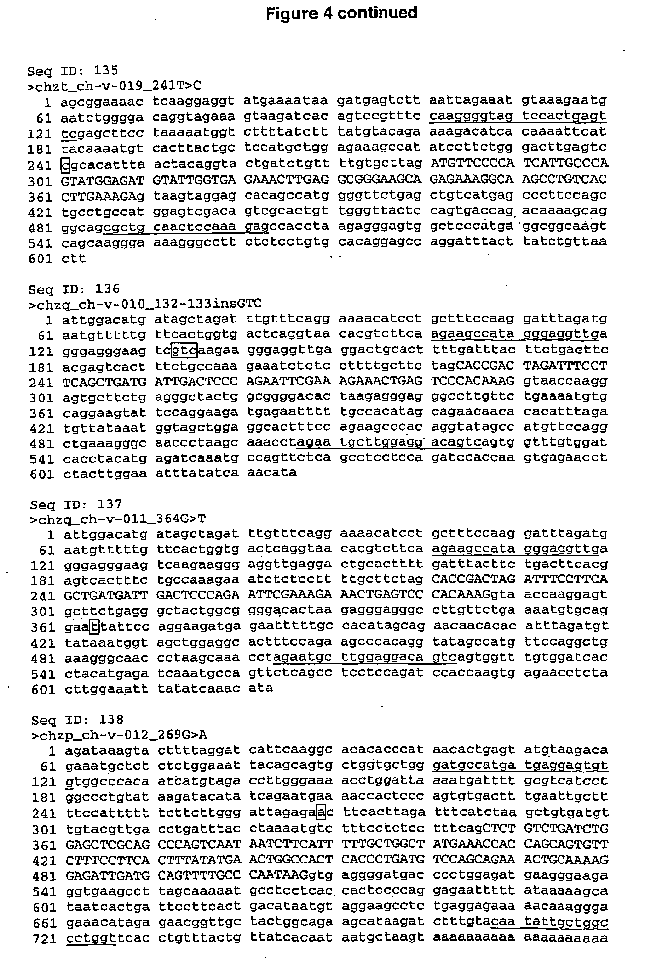

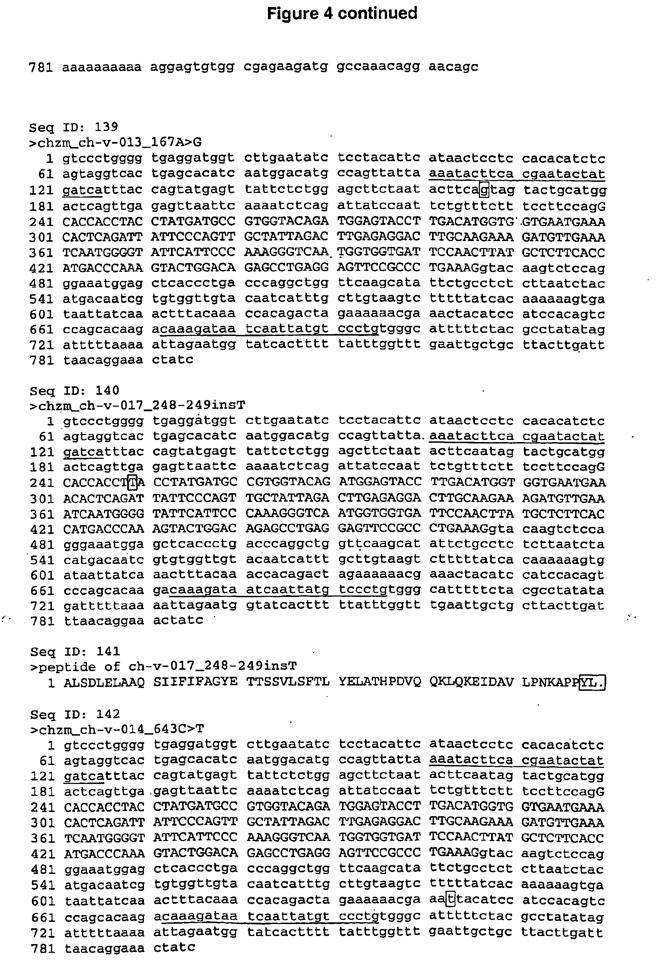

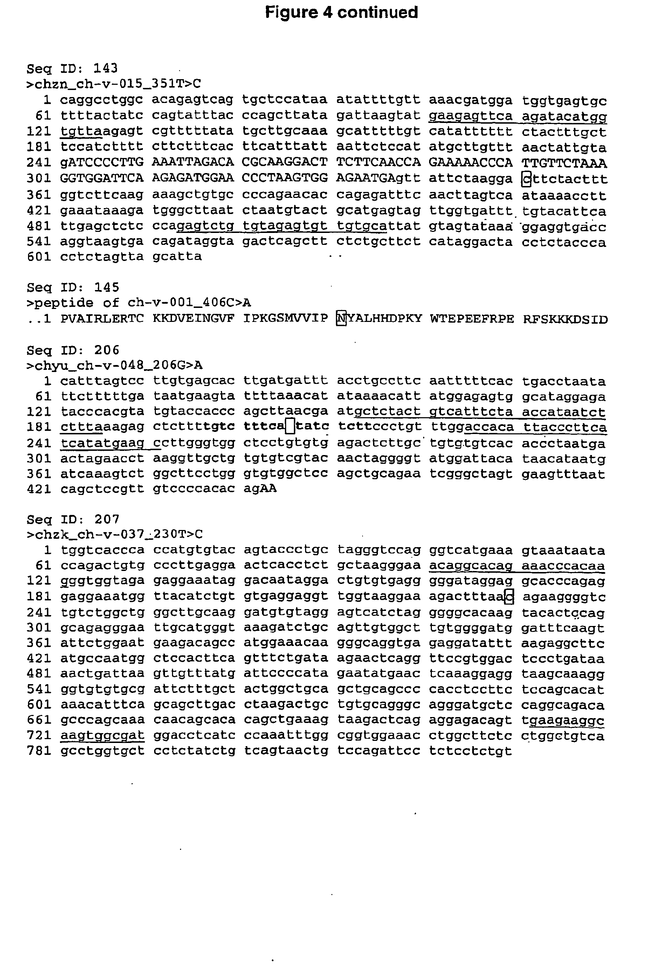

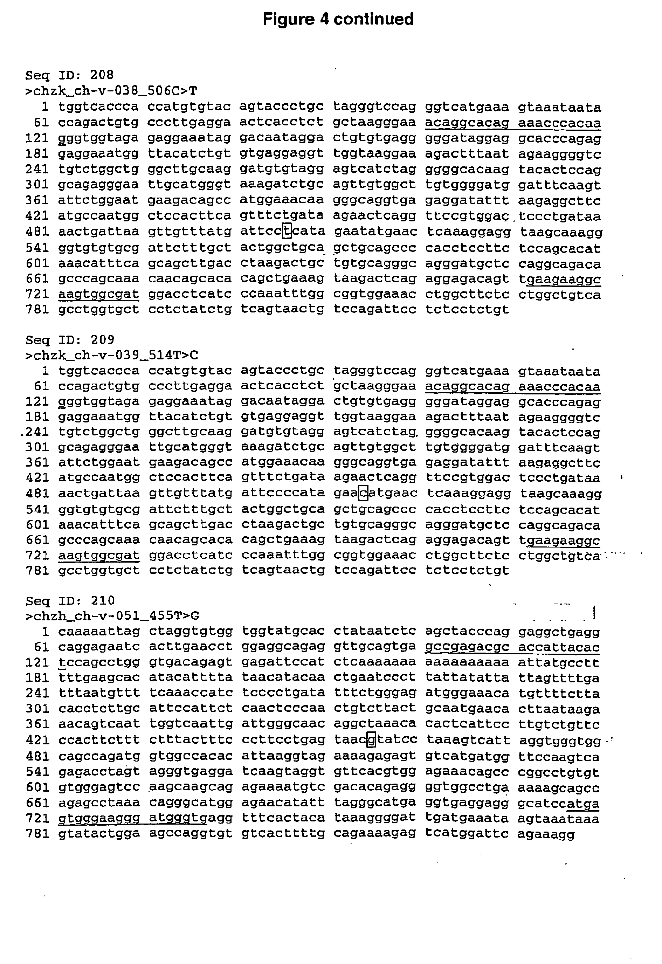

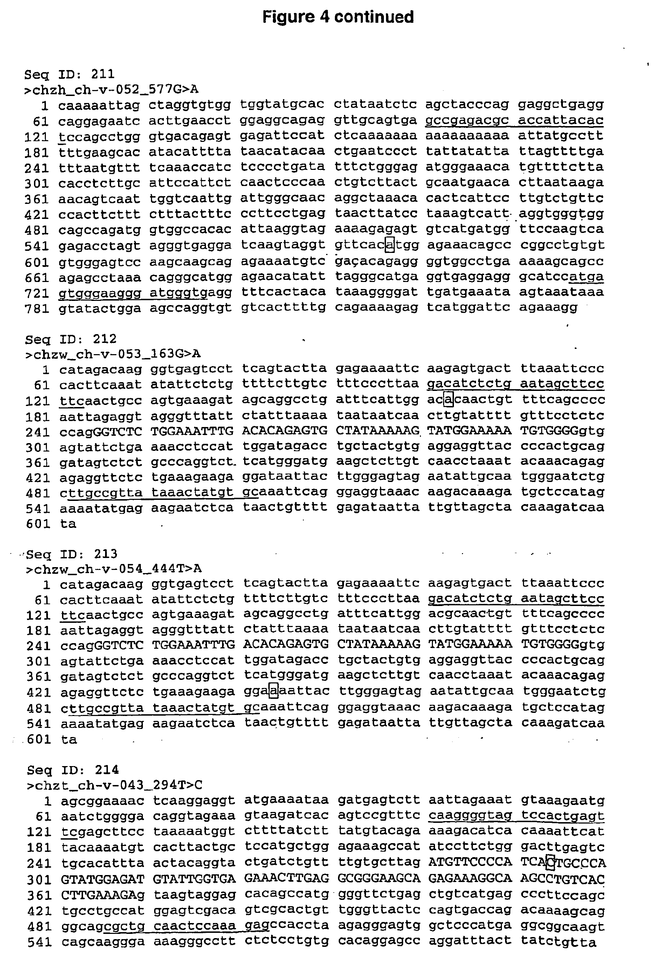

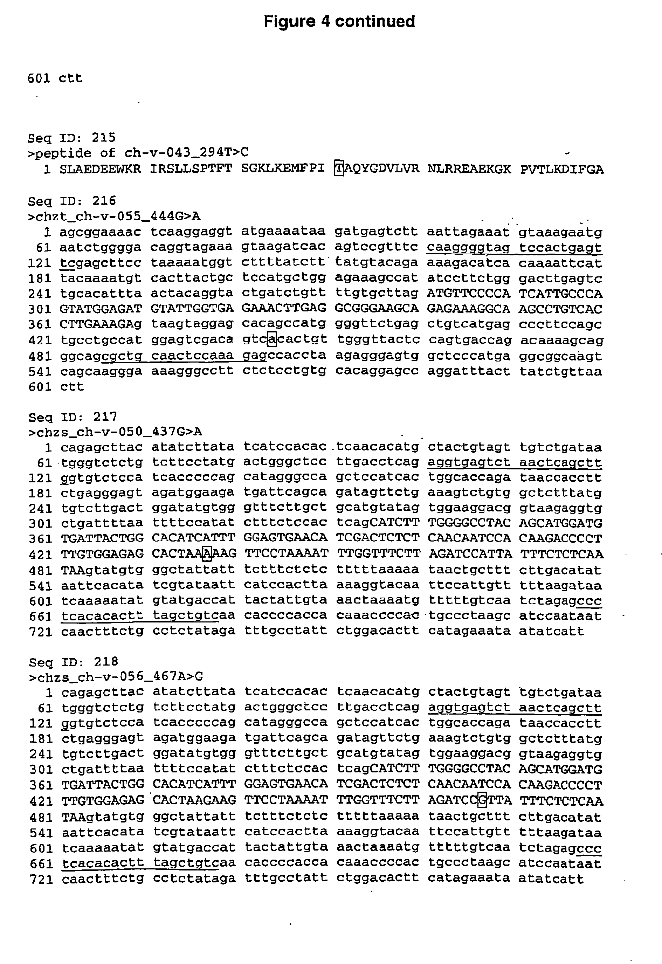

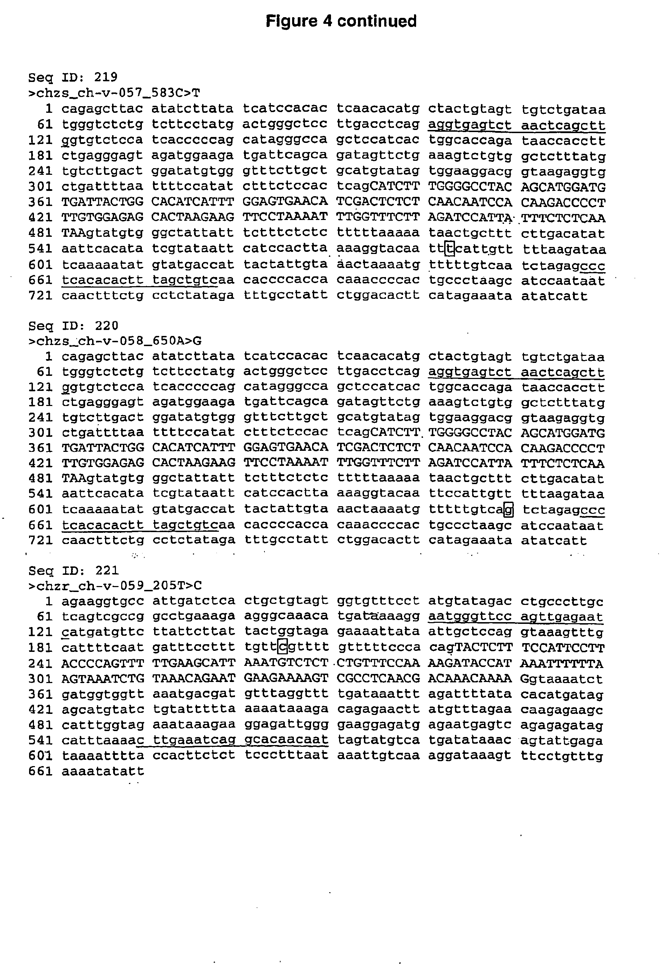

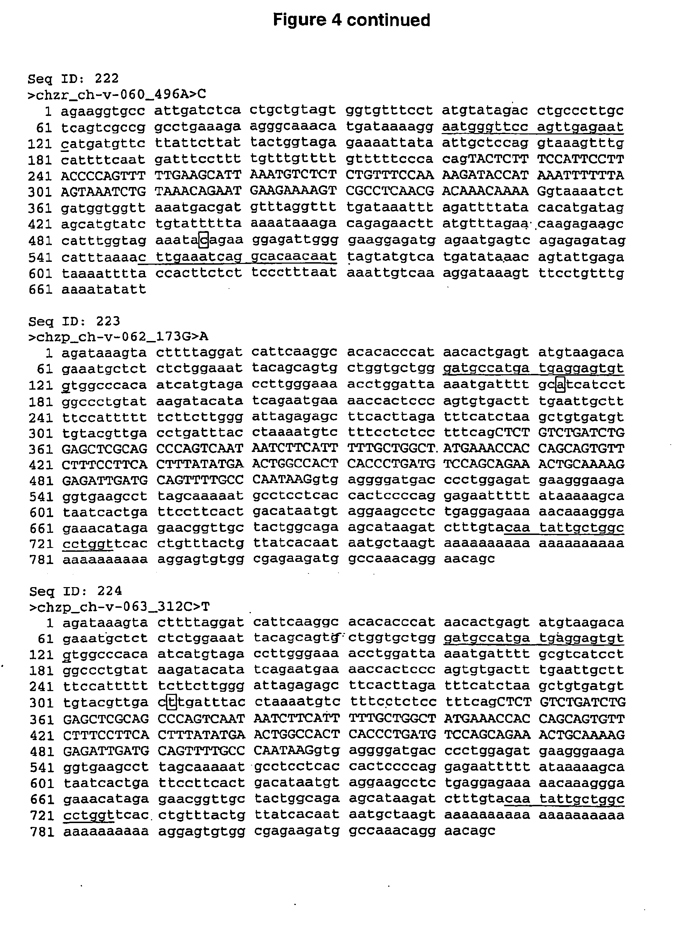

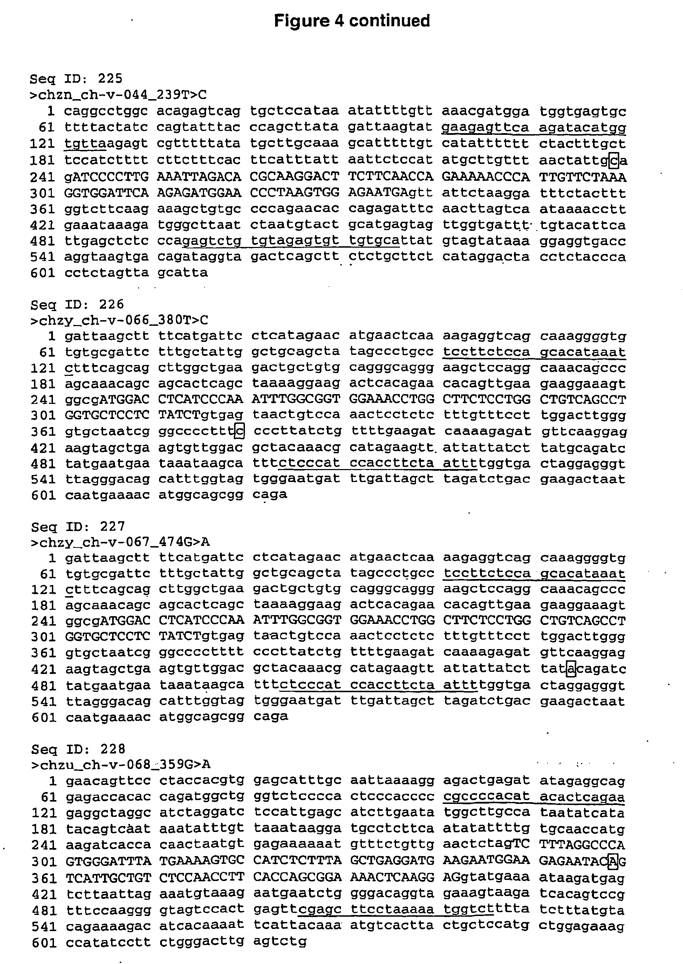

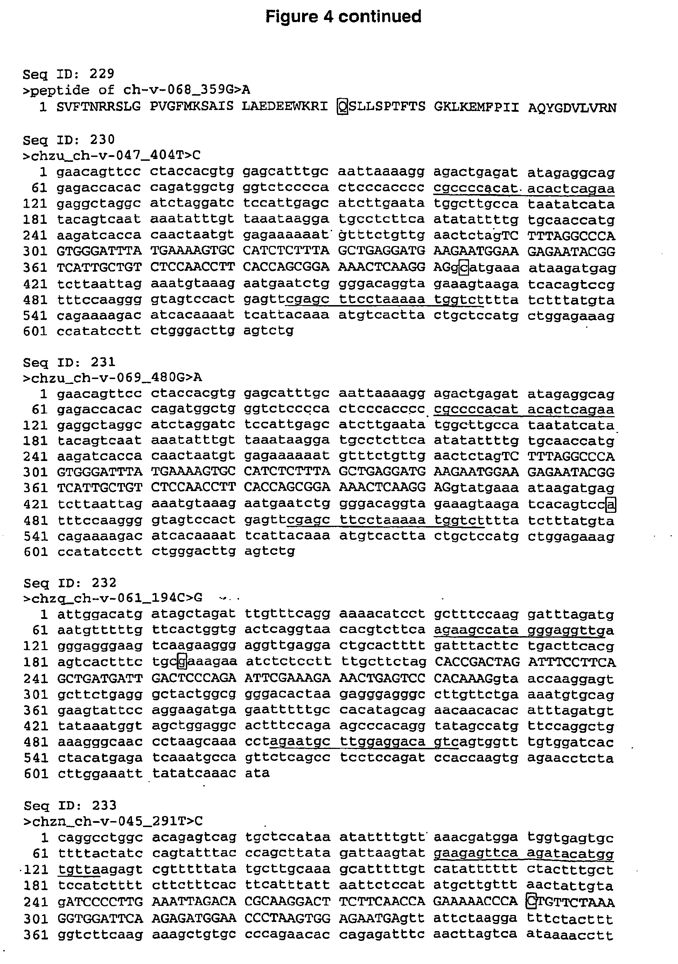

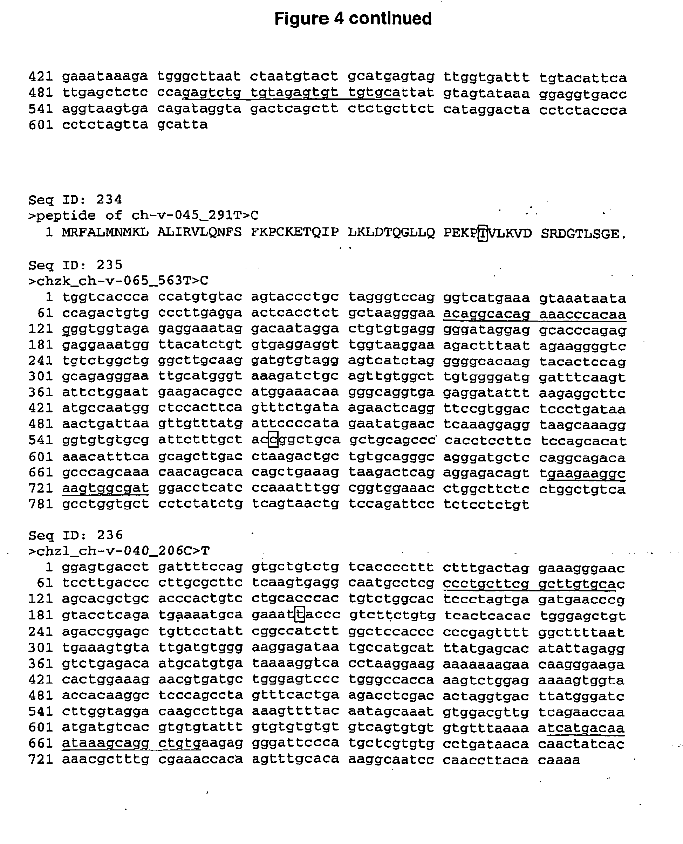

**Figure 4:** Genomic and peptide sequences: genomic DNA sequences containing the amplified regions in which polymorphisms were detected and polypeptide sequences with amino acid substitutions. Nucleotide sequences are listed in 5' - 3' orientation. Letters in lowercase indicate non-coding sequences, letters in uppercase indicate coding sequences. Primer regions are underlined. Variant sites are shown framed. Peptide sequences are shown in one letter code. ∥ marks a site where a deletion has occurred. In Seq ID 198, the hybridzing site of the TaqMan® probe is shown in bold.

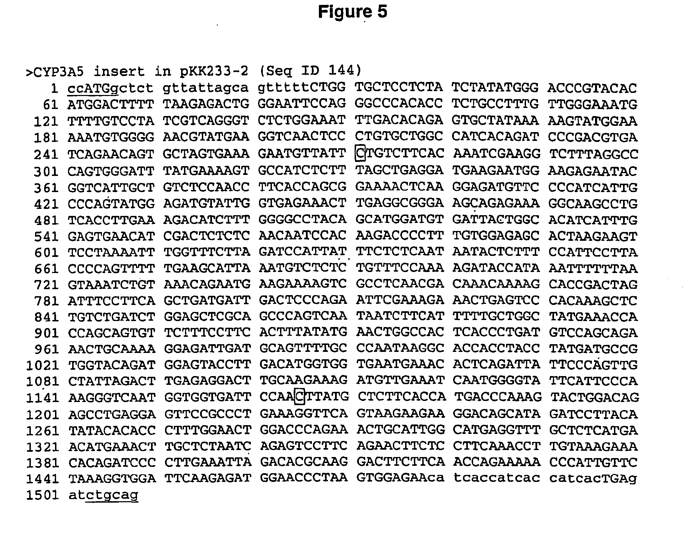

**Figure 5:** CYP3A5 cDNA insert region of the plasmid that was used as starting material for in vitro mutagenesis. Cloning sites are shown underlined. Modified 5' and 3' regions of the CYP3A5 cDNA are shown in lowercase letters. The 5' modification, a MALLLAVF amino acid sequence on protein level, has been introduced in order to increase expression in *E. coli* (Gillam, Arch Biochem Biophys 317 (1995), 374-84). The 3' modification, a His₆ tag on protein level, has been introduced in order to enhance subsequent purification. The unmodified part of the CYP3A5 insert was verified to be identical to the CYP3A5 cDNA corresponding to accession no. NM_000777.1 by sequencing, which is the underlying nucleotide sequence for NP_000768.1. Sites corresponding to ch-v-009 and ch-v-001 are shown framed.

The invention will now be described by reference to the following biological Examples which are merely illustrative and are not constructed as a limitation of the scope of the present invention.

### Example 1: Isolation of genomic DNA from human blood, generation and purification of CYP3A5 gene fragments

Genomic DNA was isolated from blood or liver samples using Qiagen blood and tissue DNA isolation kits. Oligonucleotides used in the screen were designed based on the recently determined sequence and organisation of the human *CYP3A* locus (Gellner, Pharmacogenetics 11 (2001), 111 - 121). Primer sequences and PCR fragment lengths are given in Table 1A. Amplified fragments were processed through PCR purification columns (Qiagen) and sequenced on PE ABI 3700 DNA Analysers using the same primers as in PCR. The sequences were analysed for the presence of polymorphisms using the PHRED/PHRAP/POLYPHRED/CONSED software package (University of Washington, Seattle, WA, USA).

Total RNA was isolated from liver samples using the RNeasy kit (Qiagen) according to the manufacturers instructions except that an additional DNase I digestion was performed directly on the column. cDNA pools were generated from 1 *µ*g of total RNA using random hexamer primers and Superscript reverse transcriptase (Life Technologies). The cDNA used for one TaqMan assay was derived from 40 ng total RNA. *CYP3A5* mRNA expression levels were quantified by real time quantitative PCR using the ABI 7700 Sequence Detection System (PE Biosystems). Oligonucleotides and probes were designed with the Primer Express (PE Biosystems) programme. Oligonucleotides used for the quantitative PCR were: forward 5'- TTG TTG GGA AAT GTT TTG TCC TAT C -3' (Seq ID: 237) and reverse 5'- ACA GGG AGT TGA CCT TCA TAC GTT -3' (Seq ID: 238). The TaqMan probe (5'- TCA GGG TCT CTG GAA ATT TGA CAC AGA GTG CTA-3'; Seq ID: 239) was labelled with the 5' reporter dye 6-carboxy-fluorescein (FAM) and the 3' quencher 6-carboxy-tetramethylrhodamin (TAMRA). The experiments were performed according to a standard protocol developed by PE Biosystems. The specificity of the assay for *CYP3A5* was determined using equal amounts of *CYP3A4, CYP3A5, CYP3A7* and *CYP3A43* cDNA species expressed *in vitro.* The specificity of the probe was 10⁴ times higher for *CYP3A5* than for *CYP3A7* cDNA whereas *CYP3A4* and *CYP3A43* cDNAs were not detectable at all. The linear range of the *CYP3A5* assay was determined to be between 10 and 10⁶ target molecules. *CYP3A5* expression levels were normalised using the expression of *18S* mRNA species determined with pre-developed TaqMan assays (PE Biosystems).

### Example 2: Determination of genetic variations within the CYP3A5 locus

Sequence diversity within the *CYP3A5* locus was determined by PCR amplification from genomic DNA (fragment size: 264 - 997 bp) and sequencing each PCR-product of 19 - 217 samples of Caucasian origin, 36 - 45 samples of African American origin, 34 - 47 samples of Chinese origin, 41 - 50 samples of Japanese origin, and 31 - 47 samples of Korean origin. The PCR fragments encompass the entire protein-coding region of *CYP3A5,* a portion of the 3'-UTR, the entire 5'-UTR as well as 6203 bp sequence between the *CYP3A5* transcriptional start site and a L1_5'UTR_ORF repeat located upstream of the gene (Fig. 3). In addition, we genotyped two linked single nucleotide polymorphisms (SNPs, ch-v-020, ch-v-021, Table 2A-E) located in a sequence originally described as *CYP3A5* promoter that were recently reported to co-segregate with increased CYP3A5 protein expression (Paulussen, Pharmacogenetics 10 (2000), 415-24). The results also indicate co-segregation of both variants. Using the recently determined sequence of the entire *CYP3A* locus (Gellner, Pharmacogenetics 11 (2001), 111 - 121), we place these variants approximately 20 kb upstream of the first exon of *CYP3A5,* in a sequence 5' adjacent to a *CYP3A* pseudogene (PS2 in Fig. 3). Furthermore, we additionally genotyped a single nucleotide polymorphism located in intron 3 of the *CYP3A5* gene (ch-v-048; Kuehl, 2001, Nature Genetics 27: 383-391) by TaqMan® assay using the primers and probes listed in Table 1 B. The analysis were performed on a Sequence Detection System (PE Biosystems).

A total of 29 variants including the two linked SNPs described by Paulussen *et al.* (Paulussen, Pharmacogenetics 10 (2000), 415-24) were detected in the screen of Caucasian samples and their allelic frequencies were estimated to be between 0.3 % and 11.9 % (Table 2A). 6 variants are located within the 6 kb sequence upstream of the transcriptional start site of *CYP3A5.* 14 variants are located in introns, or in the 5'-UTR or 3'-UTR, whereas 4 have been found in the protein-coding sequence. Among the latter ones, three variants result in amino acid substitutions and one in a premature termination of the CYP3A5 protein (Table 2A). The g.7303C>A variant (ch-v-009, Table 2A) results in a S100Y amino acid exchange in exon 4. The g.3705C>T variant (ch-v-005) leads to a H30Y amino acid exchange in exon 2. Cloning and sequencing revealed a physical linkage of this variant to the g.3709-3710insG variant (ch-v-006). The latter variant results in a shift of the open reading frame leading to a truncation of the protein sequence at position 34 (K34.). The T398N variant (ch-v-001, Table 2A), originally described by Jounaidi (Jounaidi, Biochem Biophys Res Commun 221 (1996), 466-70), was found in 3 out of 80 individuals tested.

Neither of the four protein altering variants found in Caucasians have been found in the African-Americans, Chinese, Japanese or Korean samples (Table 2A-E). However, among others we have found 4 new variants in these samples that result in an altered CYP3A5 amino acid sequence (ch-v-017, ch-v-043, ch-v-045, ch-v-068, Table 2B-E). The g.27131-27132insT (ch-v-017) variant in exon 11 (ch-v-017, Table 2B, 2D) has been found in 9 out of 45 African-American samples and in one out of 50 Japanese samples. The variant results in a shift of the open reading frame which leads to a truncation of the protein sequence at position 348 (D348.). Variants ch-v-043, ch-v-045 and ch-v-068 lead to amino acid exchanges.

### Example 3: Identification of genetic determinants of CYPA5 protein expression

In the following, the frequencies of Caucasian *CYP3A5* gene variants have been analyzed as a function of CYP3A5 protein expression. For this purpose, allelic frequencies of variants shown in Table 2A were calculated separately for HE and LE livers (Fig. 3). The frequencies of 9 variants (ch-v-020, ch-v-021, ch-v-026, ch-V-034, ch-v-007, ch-v-008, ch-v-011, ch-v-014 and ch-v-015) were significantly increased in HE livers (all χ² > 13.3, df = 1, p < 0.01, Bonferroni corrected). Except one, all tested HE livers (17/18, 94 %) were heterozygous for three variants (ch-v-021, ch-v-026 and ch-v-015). 16 of those samples were heterozygous for ch-v-020 as well. One HE sample could not be genotyped for this variant. In contrast, LE livers were either wildtype (155/168, 92.3 %), heterozygous for variants ch-v-021 and ch-v-26 (9/168, 5.4 %) or heterozygous for the variant ch-v-015 (4/168, 2.4 %) only. However, in LE livers all three variants never occurred simultaneously (Table 3). These results defined either of the three variants as useful but imperfect marker of increased CYP3A5 expression. The variants ch-v-034, ch-v-008, ch-v-011 and ch-v-14 only occurred in a subset of the samples heterozygous for the above three variants (ch-v-021, ch-v-026 and ch-v-015).

The distribution of variants ch-v-021, ch-v-026 and ch-v-015 in the samples screened strongly suggest that they constitute a haplotype. In the following, the hypotheses whether these three variants recombine independently or not has been tested. Assuming their independent inheritance, the expected 3-loci-genotype frequencies for all combinations of variants and compared them with the observed frequencies have been calculated. The difference is highly significant (χ² = 93.6; classes 'all wildtype', 'single variant hetero- or homozygous', 'two or three variants hetero- or homozygous'; df = 1; p << 0.001). There were more individuals with two or three of the variants than expected and less individuals with only one of the variants. This result suggests linkage among the three variants. The degree of linkage with the linkage disequilibrium parameter D for the three pairs of variants was estimated. Using maximum likelihood estimates for haplotype frequencies, D was calculated to be 0.041 for the variant pairs ch-v-021/ch-v-015 and ch-v-026/ch-v-015, which is 80 % of its theoretical maximum, and 0.065 for variants ch-v-021 and ch-v-026 which corresponds to 100 % of its theoretical maximum.
The probability that individuals showing the respective variant genotype are HE (positive predictive value) is estimated to be 65 % for variants ch-v-021 and ch-v-026, respectively, and 81 % for the ch-v-015 variant. For the combination of all three variants the positive predictive value is 100 % in our sample set. However, assuming that these variants need to be located in *cis* for increased protein expression, it is clear that there is some probability for individuals showing all three variants to be LE. The results show that at least the allele ch-v-021/ch-v-026 and the allele ch-v-015 actually exist (see genotype 2 and 3, Table 3) and therefore the existence of a genotype with a combination of these two alleles has to be postulated. The maximum likelihood estimate for the frequency of these 3-fold heterozygotes having not all three variants in cis is 0.05 % of all samples screened or 0.61 % of samples hetero- or homozygote for all three variants. In other words, of 100 Caucasians screened statistically about 9 of them will be hetero- or homozygous for all three variants and about 0.05 of these will have not all three variants in cis. Therefore, it can be expected that the positive predictive power of the 3-variant genotype to be about 99.95 %. Of course, the same values would be achieved for a combination of only two variants, either ch-v-021/ch-v-015 or ch-v-26/ch-v-15.

In a single HE liver none of the above 9. variants that were found in the other 17 HE samples could be detected. A closer examination of variants found in this sample revealed a variant within intron 4 (ch-v-018) and one within intron 5 (ch-v-019), respectively. These variants were unique to this sample, since they were not found in any other of the samples screened. Neither were they found in any of the other ethnic groups screened. It remains to be shown whether these variants are themselves causative for transcriptional activation or whether they are linked to another, so far undetected variant.

### Example 4: Determination of CYP3A5 protein expression

Protein expression of CYP3A4 and CYP3A5 in Caucasian liver samples was determined by Western blotting using CYP3A4- and CYP3A5-specific antibodies (Gentest). Liver microsomes were prepared as previously described (Zanger, Biochemistry 27 (1988), 5447-54). To obtain total protein homogenate, powdered liver tissue was homogenised in 0.1 M Tris-Cl pH 7.4, 1 mM EDTA, 1 mM Pefa Bloc SC, 1 *µ*g/ml leupeptin, 1 *µ*g/ml pepstatin with a Potter Elvehjem homogenisator (glass/Teflon) for 2 min at 1000 rpm. Homogenates were then sonified with a Bandelin Sonoplus HD 200 and stored at -80°C. For Western blotting, 12.5 *µ*g microsomal protein homogenate or 40 *µ*g total protein homogenate were separated in a 10 % SDS-polyacrylamide gel. Electrophoretic transfer onto PVDF membranes was carried out in a TankBlot Cell (BioRad) for 1.5 hours at constant voltage (100 V) and at 10 °C. Following the transfer, the membranes were incubated for 60 min in 5 % milk, TBS, 0.1 % Tween 20 to reduce the unspecific antibody binding. Incubations with either primary antibody (Gentest, dilution 1:500) were performed in 1 % milk, TBS, 0.1 % Tween 20 for 60 min, those with the secondary antibody (anti-rabbit IgG-POD Fab-fragments, Dianova, dilution 1:10000 in the same solution for 30 min. CYP3A4 or CYP3A5 protein bands were detected with Supersignal Dura (Pierce) and a digital CCD-camera (LAS-1000, Fuji). Signal quantification was performed with AIDA (Raytest). Protein expression levels were calculated based on calibration curves obtained with microsomes expressing recombinant CYP3A4 and CYP3A5 proteins (Gentest).

Homogenates or microsomal fractions were prepared from 186 human livers and investigated by Western blotting using a CYP3A5-specific antibody. CYP3A5 protein was detected in all samples analysed and its expression showed a clear bimodality (Fig. 1 A). 168 livers (- 90 %), further referred to as LE (low-expressing), showed expression close to or below the lower limit of quantification (LLOQ) of the assay (0.3 pmol/mg homogenate protein and 1.0 pmol/mg microsomal protein). Eighteen samples (~10 %), further referred to as HE (high-expressing), exhibited much higher CYP3A5 expression levels. The expression was in the range between 1.6 and 2.9 pmol/mg homogenate protein (2.3 ± 0.5; n = 6) and between 3.9 and 15.5 pmol/mg microsomal protein (9.7 ± 4.1; n = 12). Taking the LLOQ of the assay as the expression level of CYP3A5 in LE livers, HE livers express on average 8 to 10 times more CYP3A5 protein than LE livers.

In the following, the contribution of CYP3A5 to the combined CYP3A5 and CYP3A4 protein expression in HE livers was investigated. CYP3A4 expression in these livers was between 0.9 and 82.6 pmol/mg homogenate protein (n = 6) and between 4.5 and 295 pmol/mg microsomal protein (n = 11). The levels and the range of CYP3A4 variability in HE livers were similar to those in LE livers (not shown). Fig. 1B shows the share of CYP3A5 in the combined CYP3A5 and CYP3A4 protein pool in 17 HE livers. CYP3A5 contribution varies between 3 % and 74 %. In an average HE liver, the share of CYP3A5 in the combined pool of both proteins is 24 %. Taking the LLOQ of the CYP3A5 assay as the actual expression level of the protein in LE livers, the corresponding value in these livers is approximately 1.6 %.

### Example 5: Determination of CYP3A5 mRNA expression

The expression of *CYP3A5* mRNA in 8 Caucasian HE and 8 LE livers was investigated using a CYP3A5-specific TaqMan probe. As illustrated in Fig. 2A, the distribution of *CYP3A5* mRNA levels exhibited a bimodality which was in complete agreement with that observed in the expression of CYP3A5 protein (Fisher's exact test, p = << 0.001). The number of 3A5 transcripts per ng of total RNA in HE livers (n = 8) was on average 8.5 times higher than those in LE livers (n = 8).

In the following, the allelic origin of *CYP3A5* transcripts in HE and LE livers was investigated. To this end, by PCR a portion of the 3'-UTR (untranslated region) of the gene was amplified and sequenced using genomic or cDNA samples as templates which were heterozygous for a T>C variant located in this region (variant ch-v-015 in Table 2A;). As expected, both alleles are represented in the sequence using genomic DNAs as template (Fig. 2B). Both alleles were also equally represented in a sequence of PCR-amplified cDNA from a LE (homozygous wildtype for ch-v-021 and ch-v-26, heterozygous for ch-v-015) liver. In contrast, only the C allele was found in the same portion of *CYP3A5* 3'-UTR cDNA from a HE liver. This indicates an overrepresentation of transcripts derived from the chromosome harbouring the C allele in the total pool of *CYP3A5* transcripts in HE livers.

### Example 6: In vitro mutagenesis and expression of recombinant CYP3A5 proteins

Five polymorphisms in Caucasians have been detected that lead to changes in the protein sequence. Two of them, ch-v-006 and ch-v-017, lead to a truncation of the protein and therefore are unlikely to code for a functional protein. As ch-v-005 has only been found physically linked to ch-v-006, the resulting protein variant is not likely to be functional as well. To determine the effect of the protein variants ch-v-009 and ch-v-001 these variants were analysed in a heterologous bacterial expression system.
A modified CYP3A5 cDNA in the prokaryotic expression vector pKK233-2 (Pharmacia) was used as starting material for in vitro mutagenesis (Fig. 5). Variants ch-v-009 and ch-v-001, respectively, were introduced into the plasmid by in vitro mutagenesis using the QuikChange mutagenesis kit (Stratagene). The successful introduction of the mutations and the absence of other, undesired mutations was confirmed by sequencing. The original plasmid as well as the two mutagenised plasmids were used to transform *E. coli* TOPP3 cells, a strain in which optimal expression of CYP3A proteins has previously been obtained. A total of 8 separate colonies of each mutant plasmid were chosen for expression studies. The bacteria were grown and induced as described in Eiselt *et al.* (Eiselt, Pharmacogenetics 11 (2001), 447-58.). Expression was analysed 48 h and 72 h after induction with IPTG/δ-ALA. Cells were harvested as described in Domanski *et al.* (Domanski, Arch Biochem Biophys 350 (1998), 223-32). The final P450 content was measured by reduced carbon monoxide (CO) difference spectra (Omura, J. Biol. Chem. 239 (1964), 2370-2378).

Whereas 30 to 50 nmol solubilised CYP3A5 could be recovered per litre culture of the non-mutagenised CYP3A5, expression in the two CYP3A5 variants S100Y and T398N was determined to be lower than 3 nmol P450 protein per litre culture. In many instances, the "P450" peak was shifted to 454 - 458 nm rather than the typical 448 - 450 nm peak expected. The low level of expression in mutagenised colonies made any attempts at protein purification futile. Previous experiments suggest that expression levels as low as those demonstrated by these CYP3A5 variants can not be significantly improved by utilising other bacterial strains or adjusting growth temperature. Therefore, the results strongly suggest that the CYP3A5 protein variants comprising the S100Y or the T398N substitutions are unstable in an *E. coli* expression system and that the variants comprising ch-v-009 or ch-v-001 may not code for functional proteins. The result of negative expression for variant ch-v-001 (T398N) is in agreement with the study in which this polymorphism was initially detected in two of five CYP3A5 deficient individuals (Jounaidi, Biochem Biophys Res Commun 221 (1996), 466-70).

### Example 7: Prediction of expected drug metabolism by CYP3A5 genotypes

The CYP3A5 protein degrades many drugs by oxidation so that they are not therapeutically active anymore. Therefore, drugs that are CYP3A5 substrates might not reach therapeutically active plasma concentrations for an adequate time span in patients with enhanced CYP3A5 activity. In these patients these drugs have to be dosed higher. On the other side, in patients with reduced CYP3A5 activity, these drugs have to be administered at lower dosage in order to avoid toxic drug levels. Table 4 gives an assignment for CYP3A5 genotypes and recommended dosages.
In cases in which CYP3A5 metabolism leads to the formation of pharmacologically active substance, enhanced enzyme activity has to be counteracted by reduced dosage whereas reduced CYP3A5 activity has to be met by increased dosage.

**Table 1A: Primers used to screen for polymorphisms within the CYP3A5 upstream regions and the CYP3A5 gene.**

| **Ref.** | **Primer** | | | **Position (nt)** | **(bp)** |
|---|---|---|---|---|---|
| | **ID** | **Name** | **Sequence (5'-3')** | | |
| chzk | 001 | 694 | ACAGGCACAGAAACCCACAAG | 145448-145468 ¹ | 630 |
| | 002 | 711 | ATCGCCACTTGCCTTCTTC | 146077-146059 ¹ | |
| chzl | 003 | 794 | CCCTGCTTCGGCTTGTGCA | 159915-159933 ¹ | 575 |
| | 004 | 750 | CACAGCCTGCTTTATTTGTCATGA | 160489-160466 ¹ | |
| chzj | 005 | 751 | GATCCTTGGTAGGACAAGCCT | 160351-160371 ¹ | 844 |
| | 006 | 754 | CAAGCACTGATTTGGTCACTTCCT | 161194-161171 ¹ | |
| chzb | 007 | 819 | GGGATGGGACCGTAAGTGGAAC | 160951-160972 ¹ | 618 |
| | 008 | 820 | TAATCACATTGGAGTTCTGACAAATG | 161568-161543 ¹ | |
| chzi | 009 | 736 | AAAAACCTCTTACAAAAGTATCATCGGATA | 161419-161448 ¹ | 910 |
| | 010 | 737 | CCTACTAGGTCTCTGACTTGGAACCAT | 162328-162302 ¹ | |
| chzh | 011 | 784 | GCCGAGACGCACCATTACACT | 161876-161896 ¹ | 637 |
| | 012 | 785 | CACCCATCCCTTCCCACTCAT | 162512-162492 ¹ | |
| chzg | 013 | 740 | TGATGGTTCCAAGTCAGAGACCTAGTAG | 162300-162327 ¹ | 997 |
| | 014 | 741 | AATTGTAGACATCTTTCTCTTAAGTTAATTCCCAG | 163296-163262 ¹ | |
| chzf | 015 | 786 | TCTGCATGCCAACAGTGAACAATCT | 163182-163206 ¹ | 824 |
| | 016 | 789 | GGCACGCACCAGCATGTCC | 164005-163987 ¹ | |
| chze | 017 | 790 | CTGGCTGAGTGCCGTGGCT | 163845-163863 ¹ | 591 |
| | 018 | 791 | TGAGCGCTTCATGTATTCTGGCTAT | 164435-164411 ¹ | |
| chza | 019 | 824 | AAATATTTTCAAAGTCACACTCTGACAACAG | 164376-164406¹ | 617 |
| | 020 | 822 | TAACAGGATCTCATGCTTTTTTCATGGCT | 164992-164964 ¹ | |
| chzd | 021 | 747 | CACTCCAATATTCACAATAGCCACTATTCA | 164843-164872 ¹ | 926 |
| | 022 | 748 | ACTCCTACGTATCCTTCCAAGCCC | 165768-165745 ¹ | |
| chzc | 023 | 728 | GCTAAGGGAAACAGGCATAGAAACTTAC | 165586-165613 ¹ | 557 |
| | 024 | 729 | GGAGCTTCCCTGCCCTGC | 166142-166125 ¹ | |
| chzy | 025 | 323 | TCCTTCTCCAGCACATAAATC | 166076-166096 ¹ | 424 |
| | 026 | 325 | AAATTAGAAGGTGGATGGGAG | 166499-166479 ¹ | |
| chzx | 027 | 335 | GAGTAACTCACCAGCCCTCTG | 169838-169858 ¹ | 264 |
| | 028 | 336 | AAACCTCAGAACTCCCTCCCA | 170101-170081 ¹ | |
| chzw | 029 | 338 | GACATCTCTGAATAGCTTCCTTC | 171392-171414 ¹ | 402 |
| | 030 | 341 | GCACATAGTTTATAACGGCAA | 171793-171773 ¹ | |
| chzv | 031 | 346 | AGAACCTAAGGTTGCTGTGTGTC | 173303-173325 ¹ | 394 |
| | 032 | 348 | TGCAAGATGTTACCACTGGGC | 173696-173676 ¹ | |
| chzu | 033 | 354 | CGCCCCACATACACTCAGAA | 31376-31395 ² | 426 |
| | 034 | 357 | AGACCATTTTTAGGAAGCTCG | 31801-31781 ² | |
| chzt | 035 | 379 | CAAGGGGTAGTCCACTGAGTTC | 31760-31781 ² | 403 |
| | 036 | 381 | CTCTTTGGAGTTGCAGCG | 32162-32145 ² | |
| chzs | 037 | 362 | AGGTGAGTCTAACTCAGCTTG | 33081-33101 ² | 578 |
| | 038 | 365 | GACAGCTAAAGTGTGTGAGGG | 33658-33638 ² | |
| chzr | 039 | 371 | AATGGGTTCCAGTTGAGAATC | 34411-34431 ² | 470 |
| | 040 | 373 | ATTGTTGTGCCTGATTTCAAG | 34880-34860 ² | |
| chzq | 041 | 387 | AGAAGCCATAGGGAGGTTG | 35627-35645 ² | 423 |
| | 042 | 389 | GACTGTCCTCCAAGCATTCT | 36049-36030 ² | |
| chzp | 043 | 394 | GATGCCATGATGAGGAGTGTG | 37724-37744 ² | 626 |
| | 044 | 397 | ACCAGGGCCAGCAATATTG | 38349-38331 ² | |
| chzm | 045 | 403 | AAATACTTCACGAATACTATGATCA | 45711-45735 ² | 595 |
| | 046 | 405 | CAGGGACATAATTGATTATCTTTG | 46305-46282 ² | |
| chzo | 047 | 411 | TACTGGTTGGGAGGTGGAG | 48290-48308 ² | 456 |
| | 048 | 412 | CACTGATGTTCTTAATGCTACAGG | 48745-48723 ² | |
| chzn | 049 | 419 | GAAGAGTTCAAGATACATGGTGTTA | 50088-50112 ² | 416 |
| | 050 | 420 | TGCACAACACTCTACACAGACTC | 50503-50481 ² | |

| | | | | | |
|---|---|---|---|---|---|
| *Ref*.: Reference sequence. The positions of primers refer to GenBank sequences with accession numbers (1) AF280107.1 and (2) AC005020.2. | | | | | |

**Table 1B: Primers (Seq IDs 202 and 203) and probes (Seq IDs 204 and 205) used determine the nucleotide status at the polymorphic site ch-v-048 (g.6986G>A) by TaqMan assay.**

| **Ref.** | **Primer** | | | **Position (nt)** | **(bp)** |
|---|---|---|---|---|---|
| | **ID** | **Name** | **Sequence (5'-3')** | | |
| chyu | 202 | TQPi_ch-v-048_F | GCTCTACTGTCATTTCTAACCATAATCTCTTTA | 173152-173184 | 99 |
| | 204 | TQPo_ch-v-048_Al1_G_VIC | VIC-TGTCTTTCAGTATCTCTTMGB-DQ | 173196-173213 | |
| | 205 | TQPo_Ch-v-048_Al2_A_FAM | FAM-TGTCTTTCAATATCTCTTCMGB-DQ | 173196-173214 | |
| | 203 | TQPi_ch-v-048_R | GCTTCATATGATGAAGGGTAATGTGGT | 173250-173224 | |

| | | | | | |
|---|---|---|---|---|---|
| *Ref.:* Reference sequence. The positions of the primers refer to the GenBank sequence with accession number AF280107.1. Probes are labelled with a fluorenscent dye at the 5' end and labelled with a dark quencher (DQ) and using a minor groove binder (MGB). | | | | | |

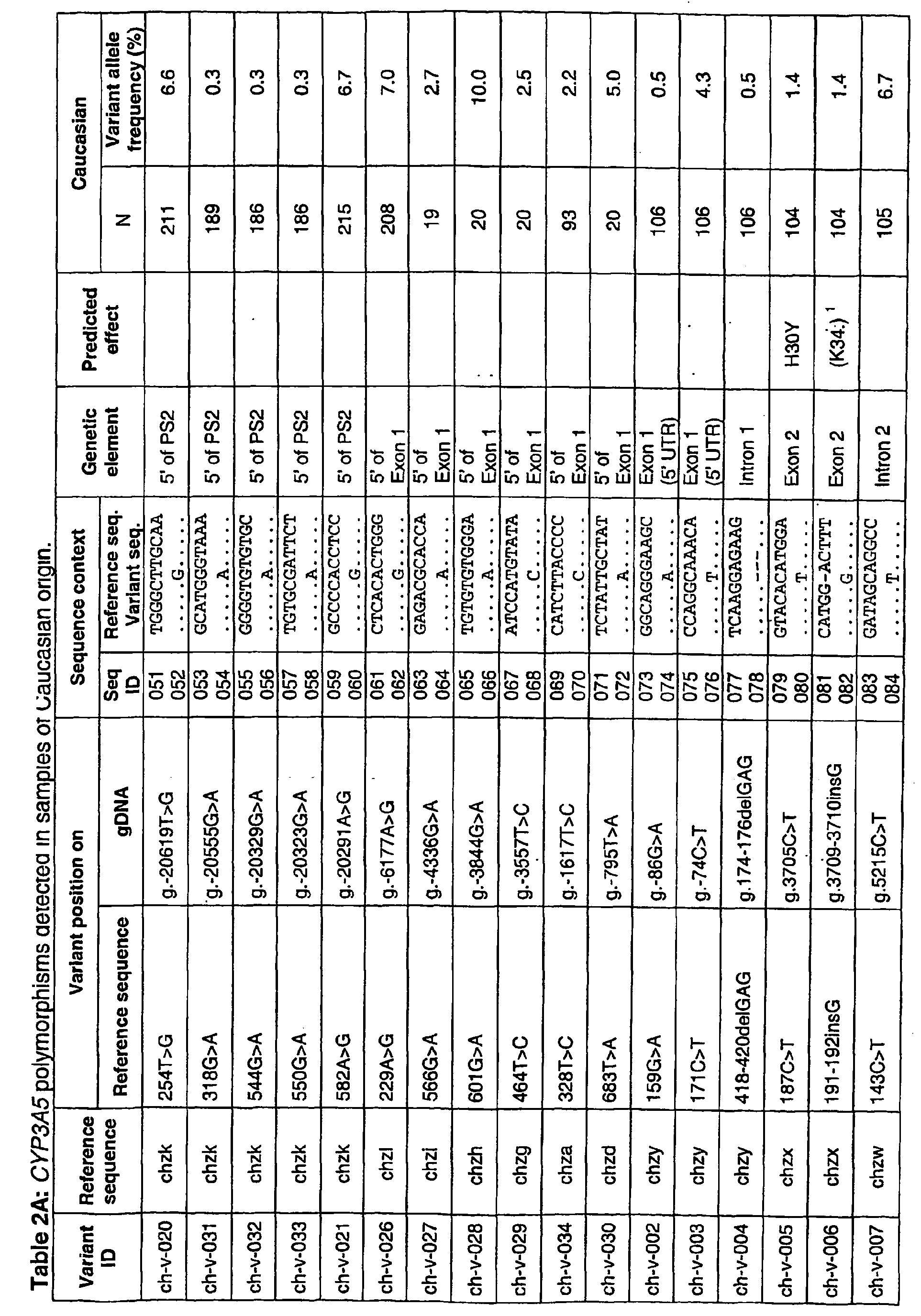

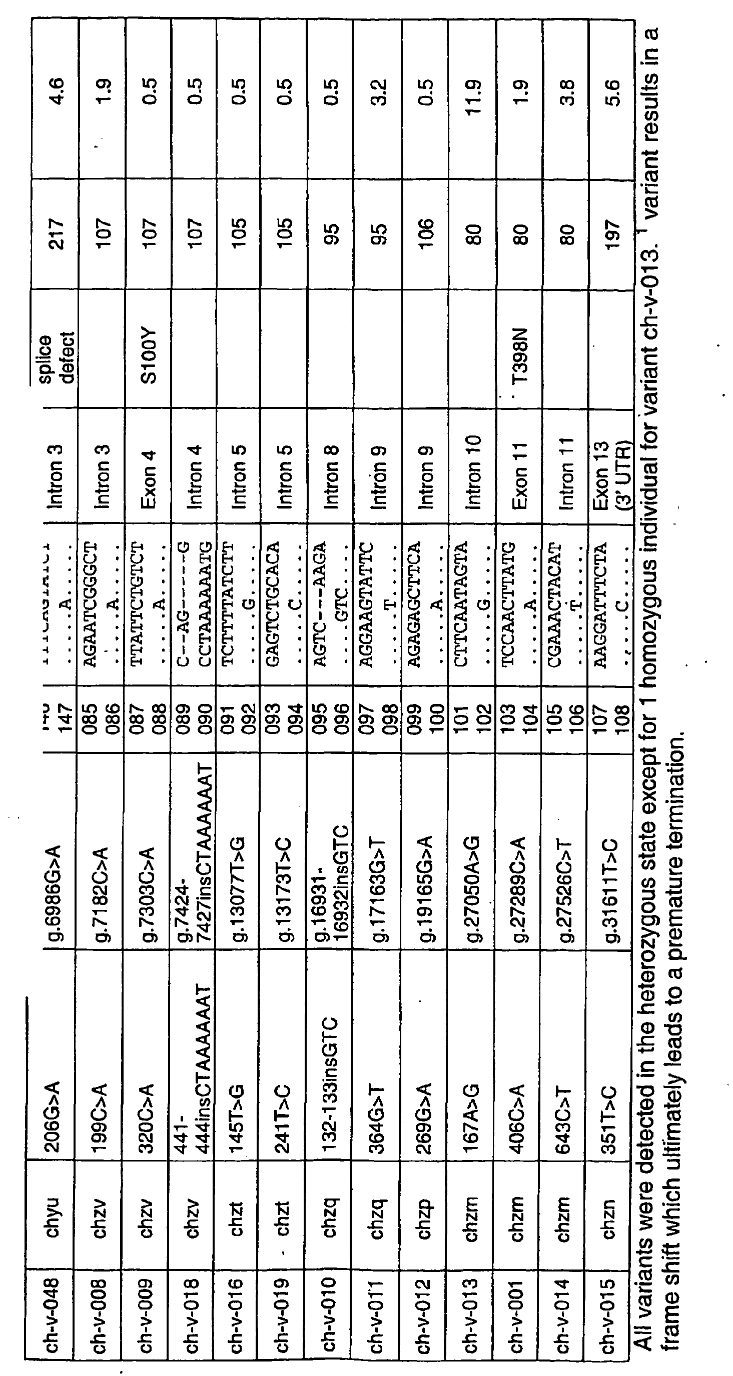

**Table 2B: CYP3A5 polymorphisms detected in samples of African-American origin.**

| **Variant ID** | **Reference sequence** | **Variant position on** | | **Sequence context** | | **Genetic element** | **Predicted effect** | **N** | **Variant allele frequency (%)** |
|---|---|---|---|---|---|---|---|---|---|
| | | **Reference sequence** | **gDNA** | **Seq ID** | **reference seq. variant seq.** | | | | |
| ch-v-037 | chzk | 230T>C | g.-20643T>C | 148 | TTTAATAGAAG | 5' of PS2 | | 42 | 3.6 |
| | | | | 149 | .....C..... | | | | |
| ch-v-020 | chzk | 254T>G | g.-20619T>G | 051 | TGGGCTTGCAA | 5' of PS2 | | 41 | 69.5 |
| | | | | 052 | .....G..... | | | | |
| ch-v-038 | chzk | 506C>T | g.-20367C>T | 150 | ATTCCCCATAG | 5' of PS2 | | 44 | 1.1 |
| | | | | 151 | .....T..... | | | | |
| ch-v-039 | chzk | 514T>C | g.-20359T>C | 152 | TAGAATATGAA | 5' of PS2 | | | 1.1 |
| | | | | 153 | .....C..... | | | | |
| ch-v-021 | chzk | 582A>G | g.-20291A>G | 059 | GCCCCACCTCC | 5' of PS2 | | 45 | 66.7 |
| | | | | 060 | .....G..... | | | | |
| ch-v-026 | chzl | 229A>G | g.-6177A>G | 061 | CTCACACTGGG | 5' of Exon 1 | | 43 | 65.1 |
| | | | | 062 | .....G..... | | | | |
| ch-v-051 | chzh | 455T>G | g.-3990T>G | 154 | GTAACTTATCC | 5' of Exon 1 | | 44 | 2.3 |
| | | | | 155 | .....G..... | | | | |
| ch-v-052 | chzh | 577G>A | g.-3868G>A | 156 | TTCACGTGGAG | 5' of Exon 1 | | 44 | 3.4 |
| | | | | 157 | .....A..... | | | | |
| ch-v-028 | chzh | 601G>A | g.-3844G>A | 065 | TGTGTGTGGGA | 5' of Exon 1 | | 44 | 17.1 |
| | | | | 066 | .....A..... | | | | |
| ch-v-034 | chyz | 328T>C | g.-1617T>C | 069 | CATCTTACCCC | 5' of Exon 1 | | 45 | 42.2 |
| | | | | 070 | .....C..... | | | | |
| ch-v-002 | chzy | 159G>A | g.-86G>A | 073 | GGCAGGGAAGC | Exon 1 (5' UTR) | | 45 | 1.1 |
| | | | | 074 | .....A..... | | | | |
| ch-v-003 | chzy | 171C>T | g.-74C>T | 075 | CCAGGCAAACA | Exon 1 (5' UTR) | | 45 | 1.1 |
| | | | | 076 | .....T..... | | | | |
| ch-v-007 | chzw | 143C>T | g.5215C>T | 083 | GATAGCAGGCC | Intron 2 | | 44 | 3.4 |
| | | | | 084 | .....T..... | | | | |
| cfi-v-053 | chzw | 163G>A | g.5235G>A | 158 | TGGACGCAACT | Intron 2 | | 45 | 2.2 |
| | | | | 159 | .....A...... | | | | |
| ch-v-054 | chzw | 444T>A | g.5516T>A | 160 | GAGGATAATTA | Intron 3 | | 43 | 3.5 |
| | | | | 161 | .....A..... | | | | |
| ch-v-048 | chyu | 206G>A | g.6986G>A | 146 | TTTCAGTATCT | Intron 3 | | 45 | 73.3 |
| | | | | 147 | .....A..... | | | | |
| ch-v-025 | chzv | 224C>T | g.7207C>T | 109 | AGCTCCGTTGT | Intron 3 | | 43 | 7.0 |
| | | | | 110 | .....T..... | | | | |
| ch-v-043 | chzt | 294T>C | g.13226T>C | 162 | CATCATTGCCC | Exon 6 | I149T | 45 | 1.1 |
| | | | | 163 | .....C..... | | | | |
| ch-v-055 | chzt | 444G>A | g.13376G>A | 164 | CAGTCGCACTG | Intron 6 | | 45 | 1.1 |
| | | | | 165 | .....A..... | | | | |
| ch-v-050 | chzs | 437G>A | g.14690G>A | 166 | ACTAAGAAGTT | Exon 7 | splice defect | 45 | 13.3 |
| | | | | 167 | .....A..... | | | | |
| ch-v-056 | chzs | 467A>G | g.14720A>G | 168 | GATCCATTATT | Exon 7 | P218P | 45 | 6.7 |
| | | | | 169 | .....G..... | | | | |
| ch-v-057 | chzs | 583C>T | g.14836C>T | 170 | CAATTCCATTG | Intron 7 | | 45 | 1.1 |
| | | | | 171 | .....T..... | | | | |
| ch-v-058 | chzs | 650A>G | g.14903A>G | 172 | TGTCAATCTAG | Intron 7 | | 45 | 6.7 |
| | | | | 173 | .....G..... | | | | |
| ch-v-059 | chzr | 205T>C | g.15788T>C | 174 | TTGTTTGTTTT | Intron 7 | | 44 | 3.4 |
| | | | | 175 | .....C..... | | | | |
| ch-v-060 | chzr | 496A>C | g.16079A>C | 176 | AAATAAAGAAG | Intron 8 | | 44 | 1.1 |
| | | | | 177 | .....C..... | | | | |
| ch-v-011 | chzq | 364G>T | g.17163G>T | 097 | AGGAAGTATTC | Intron 9 | | 43 | 7.0 |
| | | | | 098 | .....T..... | | | | |
| ch-v-062 | chzp | 173G>A | g.19069G>A | 178 | TTTGCGTCATC | Intron 9 | | 45 | 1.1 |
| | | | | 179 | .....A..... | | | | |
| ch-v-063 | chzp | 312C>T | g.19208C>T | 180 | TTGACCTGATT | Intron 9 | | 45 | 2.2 |
| | | | | 181 | .....T..... | | | | |
| ch-v-013 | chzm | 167A>G | g.27050A>G | 101 | CTTCAATAGTA | Intron 10 | | 36 | 1.4 |
| | | | | 102 | ......G ..... | | | | |
| ch-v-017 | chzm | 248-249insT | g.27131-27132insT | 111 | CACCT-ACCTA | Exon 11 | (D348.)¹ | 45 | 10.0 |
| | | | | 112 | .....T..... | | | | |
| ch-v-014 | chzm | 643C>T | g.27526C>T | 105 | CGAAACTACAT | Intron 11 | | 42 | 11.9 |
| | | | | 106 | .....T..... | | | | |
| ch-v-044 | chzn | 239T>C | g.31499T>C | 182 | TATTGTAGATC | Intron 12 | | 45 | 5.6 |
| | | | | 183 | .....C..... | | | | |
| ch-v-015 | chzn | 351T>C | g.31611T>C | 107 | AAGGATTTCTA | Exon 13 (3' UTR) | | 44 | 68.2 |
| | | | | 108 | .....C..... | | | | |

| | | | | | | | | | |
|---|---|---|---|---|---|---|---|---|---|
| ¹ variant results in a frame shift which ultimately leads to premature termination. | | | | | | | | | |

**Table 2C: CYP3A5 polymorphisms detected in samples of Chinese origin.**

| **Variant ID** | **Reference sequence** | **Variant position on** | | **Sequence context** | | **Genetic element** | **Predicted effect** | **N** | **Variant allele frequency (%)** |
|---|---|---|---|---|---|---|---|---|---|
| | | **Reference sequence** | **gDNA** | **Seq ID** | **reference seq. variant seq.** | | | | |
| ch-v-020 | chzk | 254T>G | g.-20619T>G | 051 | TGGGCTTGCAA | 5' of PS2 | | 42 | 23.8 |
| | | | | 052 | .....G..... | | | | |
| ch-v-021 | chzk | 582A>G | g.-20291A>G | 059 | GCCCCACCTCC | 5' of PS2 | | 45 | 26.7 |
| | | | | 060 | .....G..... | | | | |
| ch-v-026 | chzl | 229A>G | g.-6177 A>G | 061 | CTCACACTGGG | 5' of Exon 1 | | 47 | 26.6 |
| | | | | 062 | .....G..... | | | | |
| ch-v-034 | chyz | 328T>C | g.-1617T>C | 069 | CATCTTACCCC | 5' of | | 47 | 21.3 |
| | | | | 070 | .....C..... | | | | |
| ch-v-066 | chzy | 380T>C | g.136T>C | 184 | CCTTTTCCCTT | Intron 1 | | 45 | 1.1 |
| | | | | 185 | .....C..... | | | | |
| ch-v-067 | chzy | 474G>A | g.230G>A | 186 | CTTATGCAGAT | Intron 1 | | 44 | 1.14 |
| | | | | 187 | .....A..... | | | | |
| ch-v-048 | chyu | 206G>A | g.6986G>A | 146 | TTTCAGTATCT | Intron 3 | | 47 | 26.6 |
| | | | | 147 | .....A..... | | | | |
| ch-v-018 | chzv | 441-444insCTAAAAAAT | g.7424-7427insCTAAAAAAT | 089 | C--AG-----G | Intron 4 | | 47 | 2.1 |
| | | | | 090 | CCTAAAAAATG | | | | |
| ch-v-068 | chzu | 359G>A | g.12907G>A | 188 | AATACGGTCAT | Exon 5 | R130Q | 45 | 3.3 |
| | | | | 189 | .....A..... | | | | |
| ch-v-047 | chzu | 404T>C | g.12952T>C | 190 | GGAGGTATGAA | Intron 5 | splice | 44 | 1.1 |
| | | | | 191 | .....C..... | | | | |
| ch-v-069 | chzu | 480G>A | g.13028G>A | 192 | AGTCCGTTTCC | Intron 5 | | 44 | 1.1 |
| | | | | 193 | .....A..... | | | | |
| ch-v-011 | chzq | 364G>T | g.17163G>T | 097 | AGGAAGTATTC | Intron 9 | | 40 | 21.3 |
| | | | | 098 | .....T..... | | | | |
| ch-v-014 | chzm | 643C>T | g.27526C>T | 105 | CGAAACTACAT | Intron 11 | | 47 | 5.3 |
| | | | | 106 | .....T..... | | | | |
| ch-v-015 | chzn | 351T>C | g.31611T>C | 107 | AAGGATTTCTA | Exon 13 | | 47 | 26.6 |
| | | | | 108 | .....C..... | | | | |

**Table 2D: CYP3A5 polymorphisms detected in samples of Japanese origin.**

| **Variant ID** | **Referenc sequence** | **Variant position on** | | **Sequence context** | | **Genetic element** | **Predicted effect** | **N** | **Variant allele frequency (%)** |
|---|---|---|---|---|---|---|---|---|---|
| | | **Reference sequence** | **gDNA** | **Seq ID** | **reference seq. variant seq.** | | | | |
| ch-v-020 | chzk | 254T>G | g.-20619T>G | 051 | TGGGCTTGCAA | 5' of PS2 | | 42 | 29.8 |
| | | | | 052 | .....G..... | | | | |
| ch-v-021 | chzk | 582A>G | g.-20291 A>G | 059 | GCCCCACCTCC | 5' of PS2 | | 49 | 28.6 |
| | | | | 060 | .....G..... | | | | |
| ch-v-026 | chzl | 229A>G | g.-6177A>G | 061 | CTCACACTGGG | 5' of Exon1 1 | | 46 | 28.3 |
| | | | | 062 | .....G..... | | | | |
| ch-v-028 | chzh | 601 G>A | g.-3844G>A | 065 | TGTGTGTGGGA | 5' of Exon 1 | | 50 | 2.0 |
| | | | | 066 | .....A..... | | | | |
| ch-v-034 | chyz | 328T>C | g.-161TT>C | 069 | CATCTTACCCC | 5' of Exon 1 | | 50 | 26.0 |
| | | | | 070 | .....C..... | | | | |
| ch-v-007 | chzw | 143C>T | g.5215C>T | 083 | GATAGCAGGCC | intron 2 | | 48 | 3.1 |
| | | | | 084 | .....T..... | | | | |
| ch-v-048 | chyu | 206G>A | g.6986G>A | 146 | TTTCAGTATCT | Intron 3 | | 50 | 29.0 |
| | | | | 147 | .....A..... | | | | |
| ch-v-047 | chzu | 404T>C | g.12952T>C | 190 | GGAGGTATGAA | Intron 5 | splice defect | 50 | 1.0 |
| | | | | 191 | .....C..... | | | | |
| ch-v-061 | chzq | 194C>G | g.16993C>G | 194 | TCTGCCAAAGA | Intron 8 | | 49 | 1.0 |
| | | | | 195 | .....G..... | | | | |
| ch-v-011 | chzq | 364G>T | g.17163G>T | 097 | AGGAAGTATTC | Intron 9 | | 49 | 26.5 |
| | | | | 098 | .....T..... | | | | |
| ch-v-017 | chzm | 248-249insT | g.27131-27132insT | 111 | CACCT-ACCTA | Exon 11 | (D348.)¹ | 48 | 1.0 |
| | | | | 112 | .....T..... | | | | |
| ch-v-014 | chzm | 643C>T | g.27526C>T | 105 | CGAAACTACAT | Intron 11 | | 49 | 3.1 |
| | | | | 106 | .....T..... | | | | |
| ch-v-045 | chzn | 291T>C | g.31551T>C | 196 | ACCCATTGTTC | Exon 13 | l488T | 50 | 3.0 |
| | | | | 197 | .....C..... | | | | |
| ch-v-015 | chzn | 351T>C | g.31611T>C | 107 | AAGGATTTCTA | Exon 13 (3'UTR) | | 50 | 31.0 |
| | | | | 108 | .....C..... | | | | |

**Table 2E: CYP3A5 polymorphisms detected in samples of Korean origin.**

| **Variant ID** | **Reference sequence** | **Variant position on** | | **Sequence context** | | **Genetic element** | **Predicted effect** | **N** | **Variant allele frequency (%)** |
|---|---|---|---|---|---|---|---|---|---|
| | | **Reference sequence** | **gDNA** | **Seq ID** | **reference seq. variant seq.** | | | | |
| ch-v-020 | chzk | 254T>G | g.-20619T>G | 051 | TGGGCTTGCAA | 5' of PS2 | | 47 | 29.8 |
| | | | | 052 | ....G.... | | | | |
| ch-v-065 | chzk | 563T>C | g.-20310T>C | 198 | GCTACTGGCTG | 5' of PS2 | | 47 | 1.1 |
| | | | | 199 | .....C..... | | | | |
| ch-v-021 | chzk | 582A>G | g.-20291 A>G | 059 | GCCCCACCTCC | 5' of PS2 | | 47 | 29.8 |
| | | | | 060 | ....G..... | | | | |
| ch-v-040 | chzl | 206C>T | g.-6200C>T | 200 | GAAATCACCCG | 5' of Exon 1 | | 43 | 1.2 |
| | | | | 201 | .....T..... | | | | |
| ch-v-026 | chzl | 229A>G | g.-6177A>G | 061 | CTCACACTGGG | 5' of Exon 1 | | 43 | 31.4 |
| | | | | 062 | .....G..... | | | | |
| ch-v-034 | chyz | 328T>C | g.-1617T>C | 069 | CATCTTACCCC | 5' of Exon 1 | | 47 | 27.7 |
| | | | | 070 | .....C..... | | | | |
| ch-v-007 | chzw | 143C>T | g.5215C>T | 083 | GATAGCAGGCC | Intron 2 | | 47 | 2.1 |
| | | | | 084 | .....T..... | | | | |
| ch-v-048 | chyu | 206G>A | g.6986G>A | 146 | TTTCAGTATCT | Intron 3 | | 47 | 29.8 |
| | | | | 147 | .....A..... | | | | |
| ch-v-061 | chzq | 194C>G | g.16993C>G | 194 | TCTGCCAAAGA | Intron 8 | | 47 | 2.1 |
| | | | | 195 | .....G..... | | | | |
| ch-v-011 | chzq | 364G>T | g.17163G>T | 097 | AGGAAGTATTC | Intron 9 | | 47 | 27.7 |
| | | | | 098 | .....T..... | | | | |
| ch-v-014 | chzm | 643C>T | g.27526C>T | 105 | CGAAACTACAT | Intron 11 | | 47 | 2.1 |
| | | | | 106 | .....T..... | | | | |
| ch-v-015 | chzn | 351T>C | g.31611T>C | 107 | AAGGATTTCTA | Exon 13 (3'UTR) | | 43 | 36.1 |
| | | | | 108 | .....C..... | | | | |

Table 2A-E: Variants are listed according to their localisation along the gene, separately for each ethnic group. Polymorphism nomenclature is based on Antoarakis *et al.* (Antonarakis, Hum Mutat 11 (1998), 1-3) using the joined sequences AF280107.1 and AC005020.2 as genomic reference sequences wherein the A of the ATG at position 166220 in AF280107.1 is +1. *Sequence context:* local alignment at the polymorphic site with the reference allele sequence given at the top and the variant sequence given below. Dots indicate nucleotide identity at the respective position. *N:* number of samples analysed.

**Table 3: CYP3A5 genotypes and phenotypes.**

| | ch-v-021 | ch-v-026 | ch-v-015 | Phenotype | Livers |
|---|---|---|---|---|---|
| Genotype 1 | A/A | A/A | T/T | LE | 155 |
| Genotype 2 | A/G | A/G | T/T | LE | 9 |
| Genotype 3 | A/A | A/A | T/C | LE | 4 |
| Genotype 4 | A/G | A/G | T/C | HE | 17 |
| Genotype 5 | A/A | A/A | T/T | HE | 1 |

| | | | | | |
|---|---|---|---|---|---|
| All three variants were observed only in the heterozygous state. HE = high expressing livers, LE = low expressing livers. Numbers indicate LE and HE livers with each particular genotype. The increased CYP3A5. expression co-segregates with a distinct genotype. | | | | | |

**Table 4: Expected drug metabolism by CYP3A5**

| **Genotype No.** | **Allelic combination** | **Enzyme Activity** | **Dose Adjustment** | |
|---|---|---|---|---|
| | | | drug degradation | drug activation |
| I | high expressor allele/ high expressor allele | 190% | 1.90 | 0.53 |
| II, III | low expressor allele/ high expressor allele | 100 % | 1.00 | 1.00 |
| IV - VII | null allele/ high expressor allele | 95% | 0.95 | 1.05 |
| VIII, IX, X | low expressor allele/ low expressor allele | 10 % 10 % | 0.10 | 10 |
| XI - XVIII | null allele/ low expressor allele | 5% | 0.05 | 20 |
| XIX - XXV | null allele/ null allele | 0% | <0.05 | >20 |
| | | | | |

| **Genotype No.** | **Genotype (SeqID) at Locus** 1 - 2 - 3 - 4 - 5 - 6 - 7 - 8 | **Enzyme Activity** | **Dose Ad ustment** | |
|---|---|---|---|---|
| | | | drug degradation | drug activation |
| I | 060-062-079-081-087-111-103-108 / 060-062-079-081-087-111-103-108 | 190% | 1.90 | 0.53 |
| II | Oxx-06x-079-081-087-111-103-107 / 060-062-079-081-087-111-103-108 | 100% | 1.00 | 1.00 |
| III | 059-061-079-081-087-111-103-10x / 060-062-079-081-087-111-103-108 | 100% | 1.00 | 1.00 |
| IV | 0xx-06x-080-082-08x-11x-10x-10x / 060-062-079-081-087-111-103-108 | 95% | 0.95 | 1.05 |
| V | 0xx-06x-0xx-08x-088-11x-10x-10x / 060-062-079-081-087-111-103-108 | 95% | 0.95 | 1.05 |
| VI | 0xx-06x-0xx-08x-08x-112-10x-10x / 060-062-079-081-087-111-103-108 | 95% | 0.95 | 1.05 |
| VII | 0xx-06x-0xx-08x-08x-11x-104-10x / 060-062-079-081-087-111-103-108 | 95% | 0.95 | 1.05 |
| VIII | 060-062-079-081-087-111-103-107 / 059-061-079-081-087-111-103-108 | 10% | 0.10 | 10 |
| IX | 0xx-06x-079-081-087-111-103-107 / 0xx-06x-079-081-087-111-103-107 | 10% | 0.10 | 10 |
| X | 059-061-079-081-087-111-103-10x / 059-061-079-081-087-111-103-10x | 10% | 0.10 | 10 |
| XI | 059-061-079-081-087-111-103-10x / 0xx-06x-080-082-08x-11x-10x-10x | 5% | 0.05 | 20 |
| XII | 0xx-06x-079-081-087-111-103-107 / 0xx-06x-080-082-08x-11x-10x-10x | 5% | 0.05 | 20 |
| XIII | 059-061-079-081-087-111-103-10x / 0xx-06x-0xx-08x-088-11x-10x-10x | 5% | 0.05 | 20 |
| XIV | 0xx-06x-079-081-087-111-103-107 / 0xx-06x-0xx-08x-088-11x-10x-10x | 5% | 0.05 | 20 |
| XV | 059-061-079-081-087-111-103-10x / 0xx-06x-0xx-08x-08x-112-10x-10x | 5% | 0.05 | 20 |
| XVI | 0xx-06x-079-081-087-111-103-107 / 0xx-06x-0xx-08x-08x-112-10x-10x | 5% | 0.05 | 20 |
| XVII | 059-061-079-081-087-111-103-10x / 0xx-06x-0xx-08x-08x-11x-104-10x | 5% | 0.05 | 20 |
| XVIII | 0xx-06x-079-081-087-111-103-107 / 0xx-06x-0xx-08x-08x-112-104-10x | 5% | 0.05 | 20 |
| XIX | 0xx-06x-080-082-08x-11x-10x-10x / 0xx-06x-0xx-08x-088-11x-10x-10x | 0% | < 0.05 | > 20 |
| XX | 0xx-06x-080-082-08x-11x-10x-10x / 0xx-06x-0xx-08x-08x-112-10x-10x | 0% | < 0.05 | > 20 |
| XXI | 0xx-06x-080-082-08x-11x-10x-10x / 0xx-06x-0xx-08x-08x-11x-104-10x | 0% | < 0.05 | > 20 |
| XXII | 0xx-06x-080-082-08x-11x-10x-10x / 0xx-06x-080-082-08x-11x-10x-10x | 0% | < 0.05 | > 20 |
| XXIII | 0xx-06x-0xx-08x-088-11x-10x-10x / 0xx-06x-0xx-08x-088-11x-10x-10x | 0% | < 0.05 | > 20 |
| XXIV | 0xx-06x-0xx-08x-08x-112-10x-10x / 0xx-06x-0xx-08x-08x-112-10X-10x | 0% | <0.05 | > 20 |
| XXV | 0xx-06x-0xx-08x-08x-11x-104-10x / 0xx-06x-0xx-08x-08x-11x-104-10x | 0% | < 0.05 | > 20 |

| | | | | |
|---|---|---|---|---|
| *No*.: running genotype number. *Genotype:* Possible *CYP3A5* genotypes that result from combinations of alleles. At the top of the table concise allele names have been used to indicate the principle. The lower table lists the alleles in greater detail, giving all combinations of variants at 8 loci in the two homologous chromosomes (loci 1 -8 refer to positions corresponding to positions -20291, -6177, 3705, 3709/3710, 7303, 27131/27132, 27289 and 31611, respectively, of the *CYP3A5* gene (Accession No: AF280107.1, wherein position 166220 has been numbered +1 and position 174832 has been numbered +8613), respectively. Each variant is defined by a 3-digit Seq ID as listed in Table 2A-E. A wildcard (x) in Seq IDs indicates that the phenotype is independent from the variant at this locus in this chromosome. The possible variants for each locus that can be substituted for x can be extracted from Table 2A-E. For example, 08x at locus 4 stands for Seq IDs 081 or 082, whereas 08x at locus 5 indicates either Seq ID 087 or 088. *Enzyme Activity:* enzyme activity as calculated from protein concentration whereby the average protein concentration of genotype 059-061-079-081-111-107/060-062-079-081-111-108 was defined as 100 %. *Dose Adjustment:* dose adjustment factors for drugs that are degraded/activated by CYP3A5 relative to the dosis required for genotype 059-061-079-081-111-107/060-062-079-081-111-108. Factors may need to be weighted according to the activity share of the CYP3A5 enzyme for drugs which are not exclusively metabolised by CYP3A5. | | | | |

### SEQUENCE LISTING

<110> EPIDAUROS Biotechnologie AG
<120> Identification of the genetic determinants of the polymorhic CYP3A5 expression
<130> E 3103 PCT
<140>
   <141>
<150> EP 00 12 8627.7
   <151> 2000-12-28
<150> EP 01 10 0172.4
   <151> 2001-01-16
<150> EP 01 11 8884.4
   <151> 2001-08-16
<150> US 60/258,684
   <151> 2000-12-28
<150> US 60/258,952
   <151> 2000-12-29
<150> US 60/262,859
   <151> 2001-01-18
<150> US 60/312,825
   <151> 2001-08-16
<160> 239
<170> PatentIn Ver. 2.1
<210> 1
   <211> 21
   <212> DNA
   <213> Homo sapiens
<400> 1
   acaggcacag aaacccacaa g 21
<210> 2
   <211> 19
   <212> DNA
   <213> Homo sapiens
<400> 2
   atcgccactt gccttcttc 19
<210> 3
   <211> 19
   <212> DNA
   <213> Homo sapiens
<400> 3
   ccctgcttcg gcttgtgca 19
<210> 4
   <211> 24
   <212> DNA
   <213> Homo sapiens
<400> 4
   cacagcctgc tttatttgtc atga 24
<210> 5
   <211> 21
   <212> DNA
   <213> Homo sapiens
<400> 5
   gatccttggt aggacaagcc t 21
<210> 6
   <211> 24
   <212> DNA
   <213> Homo sapiens
<400> 6
   caagcactga tttggtcact tcct 24
<210> 7
   <211> 22
   <212> DNA
   <213> Homo sapiens
<400> 7
   gggatgggac cgtaagtgga ac 22
<210> 8
   <211> 26
   <212> DNA
   <213> Homo sapiens
<400> 8
   taatcacatt ggagttctga caaatg 26
<210> 9
   <211> 30
   <212> DNA
   <213> Homo sapiens
<400> 9
   aaaaacctct tacaaaagta tcatcggata 30
<210> 10
   <211> 27
   <212> DNA
   <213> Homo sapiens
<400> 10
   cctactaggt ctctgacttg gaaccat 27
<210> 11
   <211> 21
   <212> DNA
   <213> Homo sapiens
<400> 11
   gccgagacgc accattacac t 21
<210> 12
   <211> 21
   <212> DNA
   <213> Homo sapiens
<400> 12
   cacccatccc ttcccactca t 21
<210> 13
   <211> 28
   <212> DNA
   <213> Homo sapiens
<400> 13
   tgatggttcc aagtcagaga cctagtag 28
<210> 14
   <211> 35
   <212> DNA
   <213> Homo sapiens
<400> 14
   aattgtagac atctttctct taagttaatt cccag 35
<210> 15
   <211> 25
   <212> DNA
   <213> Homo sapiens
<400> 15
   tctgcatgcc aacagtgaac aatct 25
<210> 16
   <211> 19
   <212> DNA
   <213> Homo sapiens
<400> 16
   ggcacgcacc agcatgtcc 19
<210> 17
   <211> 19
   <212> DNA
   <213> Homo sapiens
<400> 17
   ctggctgagt gccgtggct 19
<210> 18
   <211> 25
   <212> DNA
   <213> Homo sapiens
<400> 18
   tgagcgcttc atgtattctg gctat 25
<210> 19
   <211> 31
   <212> DNA
   <213> Homo sapiens
<400> 19
   aaatattttc aaagtcacac tctgacaaca g 31
<210> 20
   <211> 29
   <212> DNA
   <213> Homo sapiens
<400> 20
   taacaggatc tcatgctttt ttcatggct 29
<210> 21
   <211> 30
   <212> DNA
   <213> Homo sapiens
<400> 21
   cactccaata ttcacaatag ccactattca 30
<210> 22
   <211> 24
   <212> DNA
   <213> Homo sapiens
<400> 22
   actcctacgt atccttccaa gccc 24
<210> 23
   <211> 28
   <212> DNA
   <213> Homo sapiens
<400> 23
   gctaagggaa acaggcatag aaacttac 28
<210> 24
   <211> 18
   <212> DNA
   <213> Homo sapiens
<400> 24
   ggagcttccc tgccctgc 18
<210> 25
   <211> 21
   <212> DNA
   <213> Homo sapiens
<400> 25
   tccttctcca gcacataaat c 21
<210> 26
   <211> 21
   <212> DNA
   <213> Homo sapiens
<400> 26
   aaattagaag gtggatggga g 21
<210> 27
   <211> 21
   <212> DNA
   <213> Homo sapiens
<400> 27
   gagtaactca ccagccctct g 21
<210> 28
   <211> 21
   <212> DNA
   <213> Homo sapiens
<400> 28
   aaacctcaga actccctccc a 21
<210> 29
   <211> 23
   <212> DNA
   <213> Homo sapiens
<400> 29
   gacatctctg aatagcttcc ttc 23
<210> 30
   <211> 21
   <212> DNA
   <213> Homo sapiens
<400> 30
   gcacatagtt tataacggca a 21
<210> 31
   <211> 23
   <212> DNA
   <213> Homo sapiens
<400> 31
   agaacctaag gttgctgtgt gtc 23
<210> 32
   <211> 21
   <212> DNA
   <213> Homo sapiens
<400> 32
   tgcaagatgt taccactggg c 21
<210> 33
   <211> 20
   <212> DNA
   <213> Homo sapiens
<400> 33
   cgccccacat acactcagaa 20
<210> 34
   <211> 21
   <212> DNA
   <213> Homo sapiens
<400> 34
   agaccatttt taggaagctc g 21
<210> 35
   <211> 22
   <212> DNA
   <213> Homo sapiens
<400> 35
   caaggggtag tccactgagt tc 22
<210> 36
   <211> 18
   <212> DNA
   <213> Homo sapiens
<400> 36
   ctctttggag ttgcagcg 18
<210> 37
   <211> 21
   <212> DNA
   <213> Homo sapiens
<400> 37
   aggtgagtct aactcagctt g 21
<210> 38
   <211> 21
   <212> DNA
   <213> Homo sapiens
<400> 38
   gacagctaaa gtgtgtgagg g 21
<210> 39
   <211> 21
   <212> DNA
   <213> Homo sapiens
<400> 39
   aatgggttcc agttgagaat c 21
<210> 40
   <211> 21
   <212> DNA
   <213> Homo sapiens
<400> 40
   attgttgtgc ctgatttcaa g 21
<210> 41
   <211> 19
   <212> DNA
   <213> Homo sapiens
<400> 41
   agaagccata gggaggttg 19
<210> 42
   <211> 20
   <212> DNA
   <213> Homo sapiens
<400> 42
   gactgtcctc caagcattct 20
<210> 43
   <211> 21
   <212> DNA
   <213> Homo sapiens
<400> 43
   gatgccatga tgaggagtgt g 21
<210> 44
   <211> 19
   <212> DNA
   <213> Homo sapiens
<400> 44
   accagggcca gcaatattg 19
<210> 45
   <211> 25
   <212> DNA
   <213> Homo sapiens
<400> 45
   aaatacttca cgaatactat gatca 25
<210> 46
   <211> 24
   <212> DNA
   <213> Homo sapiens
<400> 46
   cagggacata attgattatc tttg 24
<210> 47
   <211> 19
   <212> DNA
   <213> Homo sapiens
<400> 47
   tactggttgg gaggtggag 19
<210> 48
   <211> 23
   <212> DNA
   <213> Homo sapiens
<400> 48
   catgatgttc ttaatgctac agg 23
<210> 49
   <211> 25
   <212> DNA
   <213> Homo sapiens
<400> 49
   gaagagttca agatacatgg tgtta 25
<210> 50
   <211> 23
   <212> DNA
   <213> Homo sapiens
<400> 50
   tgcacaacac tctacacaga ctc 23
<210> 51
   <211> 11
   <212> DNA
   <213> Homo sapiens
<400> 51
   tgggcttgca a 11
<210> 52
   <211> 11
   <212> DNA
   <213> Homo sapiens
<400> 52
   tgggcgtgca a 11
<210> 53
   <211> 11
   <212> DNA
   <213> Homo sapiens
<400> 53
   gcatgggtaa a 1 1
<210> 54
   <211> 11
   <212> DNA
   <213> Homo sapiens
<400> 54
   gcatgagtaa a 11
<210> 55
   <211> 11
   <212> DNA
   <213> Homo sapiens
<400> 55
   ggggtgtgtg c 11
<210> 56
   <211> 11
   <212> DNA
   <213> Homo sapiens
<400> 56
   ggggtatgtg c 11
<210> 57
   <211> 11
   <212> DNA
   <213> Homo sapiens
<400> 57
   tgtgcgattc t 11
<210> 58
   <211> 11
   <212> DNA
   <213> Homo sapiens
<400> 58
   tgtgcaattc t 11
<210> 59
   <211> 11
   <212> DNA
   <213> Homo sapiens
<400> 59
   gccccacctc c 11
<210> 60
   <211> 11
   <212> DNA
   <213> Homo sapiens
<400> 60
   gccccgcctc c 11
<210> 61
   <211> 11
   <212> DNA
   <213> Homo sapiens
<400> 61
   ctcacactgg g 11
<210> 62
   <211> 11
   <212> DNA
   <213> Homo sapiens
<400> 62
   ctcacgctgg g 11
<210> 63
   <211> 11
   <212> DNA
   <213> Homo sapiens
<400> 63
   gagacgcacc a 11
<210> 64
   <211> 11
   <212> DNA
   <213> Homo sapiens
<400> 64
   gagacacacc a 11
<210> 65
   <211> 11
   <212> DNA
   <213> Homo sapiens
<400> 65
   tgtgtgtggg a 11
<210> 66
   <211> 11
   <212> DNA
   <213> Homo sapiens
<400> 66
   tgtgtatggg a 11
<210> 67
   <211> 11
   <212> DNA
   <213> Homo sapiens
<400> 67
   atccatgtat a 11
<210> 68
   <211> 11
   <212> DNA
   <213> Homo sapiens
<400> 68
   atccacgtat a 11
<210> 69
   <211> 11
   <212> DNA
   <213> Homo sapiens
<400> 69
   catcttaccc c 11
<210> 70
   <211> 11
   <212> DNA
   <213> Homo sapiens
<400> 70
   catctcaccc c 11
<210> 71
   <211> 11
   <212> DNA
   <213> Homo sapiens
<400> 71
   tctattgcta t 11
<210> 72
   <211> 11
   <212> DNA
   <213> Homo sapiens
<400> 72
   tctatagcta t 11
<210> 73
   <211> 11
   <212> DNA
   <213> Homo sapiens
<400> 73
   ggcagggaag c 11
<210> 74
   <211> 11
   <212> DNA
   <213> Homo sapiens
<400> 74
   ggcagagaag c 11
<210> 75
   <211> 11
   <212> DNA
   <213> Homo sapiens
<400> 75
   ccaggcaaac a 11
<210> 76
   <211> 11
   <212> DNA
   <213> Homo sapiens
<400> 76
   ccaggtaaac a 11
<210> 77
   <211> 11
   <212> DNA
   <213> Homo sapiens
<400> 77
   tcaaggagaa g 11
<210> 78
   <211> 11
   <212> DNA
   <213> Homo sapiens
<400> 78
   tcaagaagta g 11
<210> 79
   <211> 11
   <212> DNA
   <213> Homo sapiens
<400> 79
   gtacacatgg a 11
<210> 80
   <211> 11
   <212> DNA
   <213> Homo sapiens
<400> 80
   gtacatatgg a 11
<210> 81
   <211> 10
   <212> DNA
   <213> Homo sapiens
<400> 81
   catggacttt 10
<210> 82
   <211> 11
   <212> DNA
   <213> Homo sapiens
<400> 82
   catgggactt t 11
<210> 83
   <211> 11
   <212> DNA
   <213> Homo sapiens
<400> 83
   gatagcaggc c 11
<210> 84
   <211> 11
   <212> DNA
   <213> Homo sapiens
<400> 84
   gatagtaggc c 11
<210> 85
   <211> 11
   <212> DNA
   <213> Homo sapiens
<400> 85
   agaatcgggc t 11
<210> 86
   <211> 11
   <212> DNA
   <213> Homo sapiens
<400> 86
   agaatagggc t 11
<210> 87
   <211> 11
   <212> DNA
   <213> Homo sapiens
<400> 87
   ttattctgtc t 11
<210> 88
   <211> 11
   <212> DNA
   <213> Homo sapiens
<400> 88
   ttattatgtc t 11
<210> 89
   <211> 11
   <212> DNA
   <213> Homo sapiens
<400> 89
   gtacaggaag g 11
<210> 90
   <211> 11
   <212> DNA
   <213> Homo sapiens
<400> 90
   cctaaaaaat g 11
<210> 91
   <211> 11
   <212> DNA
   <213> Homo sapiens
<400> 91
   tcttttatct t 11
<210> 92
   <211> 11
   <212> DNA
   <213> Homo sapiens
<400> 92
   tctttgatct t 11
<210> 93
   <211> 11
   <212> DNA
   <213> Homo sapiens
<400> 93
   gagtctgcac a 11
<210> 94
   <211> 11
   <212> DNA
   <213> Homo sapiens
<400> 94
   gagtccgcac a 11
<210> 95
   <211> 11
   <212> DNA
   <213> Homo sapiens
<400> 95
   gaagtcaaga a 11
<210> 96
   <211> 11
   <212> DNA
   <213> Homo sapiens
<400> 96
   agtcgtcaag a 11
<210> 97
   <211> 11
   <212> DNA
   <213> Homo sapiens
<400> 97
   aggaagtatt c 11
<210> 98
   <211> 11
   <212> DNA
   <213> Homo sapiens
<400> 98
   aggaattatt c 11
<210> 99
   <211> 11
   <212> DNA
   <213> Homo sapiens
<400> 99
   agagagcttc a 11
<210> 100
   <211> 11
   <212> DNA
   <213> Homo sapiens
<400> 100
   agagaacttc a 11
<210> 101
   <211> 11
   <212> DNA
   <213> Homo sapiens
<400> 101
   cttcaatagt a 11
<210> 102
   <211> 11
   <212> DNA
   <213> Homo sapiens
<400> 102
   cttcagtagt a 11
<210> 103
   <211> 11
   <212> DNA
   <213> Homo sapiens
<400> 103
   tccaacttat g 11
<210> 104
   <211> 11
   <212> DNA
   <213> Homo sapiens
<400> 104 11
   tccaaattat g 11
<210> 105
   <211> 11
   <212> DNA
   <213> Homo sapiens
<400> 105
   cgaaactaca t 11
<210> 106
   <211> 11
   <212> DNA
   <213> Homo sapiens
<400> 106
   cgaaattaca t 11
<210> 107
   <211> 11
   <212> DNA
   <213> Homo sapiens
<400> 107
   aaggatttct a 11
<210> 108
   <211> 11
   <212> DNA
   <213> Homo sapiens
<400> 108
   aaggacttct a 11
<210> 109
   <211> 11
   <212> DNA
   <213> Homo sapiens
<400> 109
   agctccgttg t 11
<210> 110
   <211> 11
   <212> DNA
   <213> Homo sapiens
<400> 110
   agctctgttg t 11
<210> 111
   <211> 10
   <212> DNA
   <213> Homo sapiens
<400> 111
   cacctaccta 10
<210> 112
   <211> 11
   <212> DNA
   <213> Homo sapiens
<400> 112
   caccttacct a 11
<210> 113
   <211> 830
   <212> DNA
   <213> Homo sapiens
<400> 113
   tggtcaccca ccatgtgtac agtaccctgc tagggtccag ggtcatgaaa gtaaataata 60
   ccagactgtg cccttgagga actcacctct gctaagggaa acaggcacag aaacccacaa 120
   gggtggtaga gaggaaatag gacaatagga ctgtgtgagg gggataggag gcacccagag 180
   gaggaaatgg ttacatctgt gtgaggaggt tggtaaggaa agactttaat agaaggggtc 240
   tgtctggctg ggcgtgcaag gatgtgtagg agtcatctag ggggcacaag tacactccag 300
   gcagagggaa ttgcatgggt aaagatctgc agttgtggct tgtggggatg gatttcaagt 360
   attctggaat gaagacagcc atggaaacaa gggcaggtga gaggatattt aagaggcttc 420
   atgccaatgg ctccacttca gtttctgata agaactcagg ttccgtggac tccctgataa 480
   aactgattaa gttgtttatg attccccata gaatatgaac tcaaaggagg taagcaaagg 540
   ggtgtgtgcg attctttgct actggctgca gctgcagccc cgcctccttc tccagcacat 600
   aaacatttca gcagcttgac ctaagactgc tgtgcagggc agggatgctc caggcagaca 660
   gcccagcaaa caacagcaca cagctgaaag taagactcag aggagacagt tgaagaaggc 720
   aagtggcgat ggacctcatc ccaaatttgg cggtggaaac ctggcttctc ctggctgtca 780
   gcctggtgct cctctatctg tcagtaactg tccagattcc tctcctctgt            830
<210> 114
   <211> 830
   <212> DNA
   <213> Homo sapiens
<400> 114
   tggtcaccca ccatgtgtac agtaccctgc tagggtccag ggtcatgaaa gtaaataata 60
   ccagactgtg cccttgagga actcacctct gctaagggaa acaggcacag aaacccacaa 120
   gggtggtaga gaggaaatag gacaatagga ctgtgtgagg gggataggag gcacccagag 180
   gaggaaatgg ttacatctgt gtgaggaggt tggtaaggaa agactttaat agaaggggtc 240
   tgtctggctg ggcttgcaag gatgtgtagg agtcatctag ggggcacaag tacactccag 300
   gcagagggaa ttgcatgagt aaagatctgc agttgtggct tgtggggatg gatttcaagt 360
   attctggaat gaagacagcc atggaaacaa gggcaggtga gaggatattt aagaggcttc 420
   atgccaatgg ctccacttca gtttctgata agaactcagg ttccgtggac tccctgataa 480
   aactgattaa gttgtttatg attccccata gaatatgaac tcaaaggagg taagcaaagg 540
   ggtgtgtgcg attctttgct actggctgca gctgcagccc cacctccttc tccagcacat 600
   aaacatttca gcagcttgac ctaagactgc tgtgcagggc agggatgctc caggcagaca 660
   gcccagcaaa caacagcaca cagctgaaag taagactcag aggagacagt tgaagaaggc 720
   aagtggcgat ggacctcatc ccaaatttgg cggtggaaac ctggcttctc ctggctgtca 780
   gcctggtgct cctctatctg tcagtaactg tccagattcc tctcctctgt             830
<210> 115
   <211> 830
   <212> DNA
   <213> Homo sapiens
<400> 115
   tggtcaccca ccatgtgtac agtaccctgc tagggtccag ggtcatgaaa gtaaataata 60
   ccagactgtg cccttgagga actcacctct gctaagggaa acaggcacag aaacccacaa 120
   gggtggtaga gaggaaatag gacaatagga ctgtgtgagg gggataggag gcacccagag 180
   gaggaaatgg ttacatctgt gtgaggaggt tggtaaggaa agactttaat agaaggggtc 240
   tgtctggctg ggcttgcaag gatgtgtagg agtcatctag ggggcacaag tacactccag 300
   gcagagggaa ttgcatgggt aaagatctgc agttgtggct tgtggggatg gatttcaagt 360
   attctggaat gaagacagcc atggaaacaa gggcaggtga gaggatattt aagaggcttc 420
   atgccaatgg ctccacttca gtttctgata agaactcagg ttccgtggac tccctgataa 480
   aactgattaa gttgtttatg attccccata gaatatgaac tcaaaggagg taagcaaagg 540
   ggtatgtgcg attctttgct actggctgca gctgcagccc cacctccttc tccagcacat 600
   aaacatttca gcagcttgac ctaagactgc tgtgcagggc agggatgctc caggcagaca 660
   gcccagcaaa caacagcaca cagctgaaag taagactcag aggagacagt tgaagaaggc 720
   aagtggcgat ggacctcatc ccaaatttgg cggtggaaac ctggcttctc ctggctgtca 780
   gcctggtgct cctctatctg tcagtaactg tccagattcc tctcctctgt             830
<210> 116
   <211> 830
   <212> DNA
   <213> Homo sapiens
<400> 116
   tggtcaccca ccatgtgtac agtaccctgc tagggtccag ggtcatgaaa gtaaataata 60
   ccagactgtg cccttgagga actcacctct gctaagggaa acaggcacag aaacccacaa 120
   gggtggtaga gaggaaatag gacaatagga ctgtgtgagg gggataggag gcacccagag 180
   gaggaaatgg ttacatctgt gtgaggaggt tggtaaggaa agactttaat agaaggggtc 240
   tgtctggctg ggcttgcaag gatgtgtagg agtcatctag ggggcacaag tacactccag 300
   gcagagggaa ttgcatgggt aaagatctgc agttgtggct tgtggggatg gatttcaagt 360
   attctggaat gaagacagcc atggaaacaa gggcaggtga gaggatattt aagaggcttc 420
   atgccaatgg ctccacttca gtttctgata agaactcagg ttccgtggac tccctgataa 480
   aactgattaa gttgtttatg attccccata gaatatgaac tcaaaggagg taagcaaagg 540
   ggtgtgtgca attctttgct actggctgca gctgcagccc cacctccttc tccagcacat 600
   aaacatttca gcagcttgac ctaagactgc tgtgcagggc agggatgctc caggcagaca 660
   gcccagcaaa caacagcaca cagctgaaag taagactcag aggagacagt tgaagaaggc 720
   aagtggcgat ggacctcatc ccaaatttgg cggtggaaac ctggcttctc ctggctgtca 780
   gcctggtgct cctctatctg tcagtaactg tccagattcc tctcctctgt            830
<210> 117
   <211> 775
   <212> DNA
   <213> Homo sapiens
<400> 117
   ggagtgacct gattttccag gtgctgtctg tcaccccttt ctttgactag gaaagggaac 60
   tccttgaccc cttgcgcttc tcaagtgagg caatgcctcg ccctgcttcg gcttgtgcac 120
   agcacgctgc acccactgtc ctgcacccac tgtctggcac tccctagtga gatgaacccg 180
   gtacctcaga tgaaaatgca gaaatcaccc gtcttctgtg tcactcacgc tgggagctgt 240
   agaccggagc tgttcctatt cggccatctt ggctccaccc cccgagtttt ggcttttaat 300
   tgaaagtgta ttgatgtggg aaggagataa tgccatgcat ttatgagcac atattagagg 360
   gtctgagaca atgcatgtga taaaaggtca cctaaggaag aaaaaaagaa caagggaaga 420
   cactggaaag aacgtgatgc tgggagtccc tgggccacca aagtctggag aaaagtggta 480
   accacaaggc tcccagccta gtttcactga agacctcgac actaggtgac ttatgggatc 540
   cttggtagga caagccttga aaagttttac aatagcaaat gtggacgttg tcagaaccaa 600
   atgatgtcac gtgtgtattt gtgtgtgtgt gtcagtgtgt gtgtttaaaa atcatgacaa 660
   ataaagcagg ctgtgaagag gggattccca tgctcgtgtg cctgataaca caactatcac 720
   aaacgctttg cgaaaccaca agtttgcaca aaggcaatcc caaccttaca caaaa      775
<210> 118
   <211> 1110
   <212> DNA
   <213> Homo sapiens
<400> 118
   taatgaaaat aagaattatt ttgatggctc taacagtgac atttatatca tctgttttat 60
   ctggagcatt ctataataag tttatattaa gcaaatcaat aaaaacctct tacaaaagta 120
   tcatcggata ctttcctgaa cattaaggag aaatctatag aactgaatga atgagaacca 180
   acaagtaaat atatgtgatc attgtaacca ttgttggtgt ggggcatttg tcagaactcc 240
   aatgtgatta ttaacatagg tgagaattaa tccactgtga ctttgcccat tgcttagaaa 300
   gaacattcat agtttaatta tgcctttttt gaccaagcac agtggctcat gcctgtaatc 360
   ccagcacttt gggaggccga ggtgggtgga tcacctgagg tcaggagttc gagaccagcc 420
   tgaccaacat ggtgaaaccc catctctact aaaaatacaa aaattagcta ggtgtggtgg 480
   tatgcaccta taatctcagc tacccaggag gctgaggcag gagaatcact tgaacctgga 540
   ggcagaggtt gcagtgagcc gagacacacc attacactcc agcctgggtg acagagtgag 600
   attccatctc aaaaaaaaaa aaaaaaaatt atgccttttt gaagcacata cattttataa 660
   catacaactg aatcccttat tatattatta gttttgattt aatgttttca aaccatctcc 720
   cctgatattt ctgggagatg ggaaacatgt tttcttacac ctcttgcatt ccattctcaa 780
   ctcccaactg tcttactgca atgaacactt aataagaaac agtcaattgg tcaattgatt 840
   gggcaacagg ctaaacacac tcattccttg tctgttccca cttctttctt tactttccct 900
   tcctgagtaa cttatcctaa agtcattagg tgggtggcag ccagatggtg gccacacatt 960
   aaggtagaaa agagagtgtc atgatggttc caagtcagag acctagtagg gtgaggatca 1020
   agtaggtgtt cacgtggaga aacagcccgg cctgtgtgtg ggagtccaag caagcagaga 1080
   aaatgtcgac acagaggggt ggcctgaaaa 1110
<210> 119
   <211> 837
   <212> DNA
   <213> Homo sapiens
<400> 119
   caaaaattag ctaggtgtgg tggtatgcac ctataatctc agctacccag gaggctgagg 60
   caggagaatc acttgaacct ggaggcagag gttgcagtga gccgagacgc accattacac 120
   tccagcctgg gtgacagagt gagattccat ctcaaaaaaa aaaaaaaaaa attatgcctt 180
   tttgaagcac atacatttta taacatacaa ctgaatccct tattatatta ttagttttga 240
   tttaatgttt tcaaaccatc tcccctgata tttctgggag atgggaaaca tgttttctta 300
   cacctcttgc attccattct caactcccaa ctgtcttact gcaatgaaca cttaataaga 360
   aacagtcaat tggtcaattg attgggcaac aggctaaaca cactcattcc ttgtctgttc 420
   ccacttcttt ctttactttc ccttcctgag taacttatcc taaagtcatt aggtgggtgg 480
   cagccagatg gtggccacac attaaggtag aaaagagagt gtcatgatgg ttccaagtca 540
   gagacctagt agggtgagga tcaagtaggt gttcacgtgg agaaacagcc cggcctgtgt 600
   atgggagtcc aagcaagcag agaaaatgtc gacacagagg ggtggcctga aaaagcagcc 660
   agagcctaaa cagggcatgg agaacatatt tagggcatga ggtgaggagg gcatccatga 720
   gtgggaaggg atgggtgagg tttcactaca taaaggggat tgatgaaata agtaaataaa 780
   gtatactgga agccaggtgt gtcacttttg cagaaaagag tcatggattc agaaagg 837
<210> 120
   <211> 1197
   <212> DNA
   <213> Homo sapiens
<400> 120
   ttctttcttt actttccctt cctgagtaac ttatcctaaa gtcattaggt gggtggcagc 60
   cagatggtgg ccacacatta aggtagaaaa gagagtgtca tgatggttcc aagtcagaga 120
   cctagtaggg tgaggatcaa gtaggtgttc acgtggagaa acagcccggc ctgtgtgtgg 180
   gagtccaagc aagcagagaa aatgtcgaca cagaggggtg gcctgaaaaa gcagccagag 240
   cctaaacagg gcatggagaa catatttagg gcatgaggtg aggagggcat ccatgagtgg 300
   gaagggatgg gtgaggtttc actacataaa ggggattgat gaaataagta aataaagtat 360
   actggaagcc aggtgtgtca cttttgcaga aaagagtcat ggattcagaa agggagaaaa 420
   ctagcaggaa tcctatgaaa ttagattaaa atggatgtat ccacgtatat tcataccctt 480
   ctagatagat aaatggttag ataggtgata aaaagataac aagaggacaa gataattaga 540
   tagacataaa tgtatgtatg tgtttgtgtg tgtgtacaaa aaaacatata ctccctactt 600
   ctctccactg atagggctag gtaacaatgg catttcaata gcaatgagca cacttagtgg 660
   ccagatcttg gcttattaat accattttcc actgaaagga accagagctt tttagagaaa 720
   tggctgattc cagggccagg attaagaatg ttcaagataa gcctaggata cattttgtgc 780
   caggaagcaa gaagatgttc aaatgatttc caagtaatgt ttggaaatga tatttgaaaa 840
   tgatttccaa atgatatttc caaatgattt ccaaatgata tatggaaaca cttaaagact 900
   ccactaaaga actattagat ctgataaaca aattcagtaa tgttgctgga tacaaaatca 960
   acatacaaaa accagtagca tttctgcatg ccaacagtga acaatctggc aaaaataaaa 1020
   aatgtaatcc catttacaat aaccccaaat aaaactaaat acctgggaat taacttaaga 1080
   gaaagatgtc tacaattaat attgtaaaac actgatgaag gaaattgaag aagacacaaa 1140
   aaagaaggat attccatgtt tatatattgt aagcattaat attgttaaaa atgtcca    1197
<210> 121
   <211> 817
   <212> DNA
   <213> Homo sapiens
<400> 121
   caaagcaaaa atggacaaat ggatcagatc aagttaaaaa gcttctgtac cacaaagaaa 60
   gcaatcaaca aagtgaagac acaaaccaca gaatgggaga aaatattttc aaagtcacac 120
   tctgacaaca gattaatagc cagaatacat gaagcgctca aacaactctg taaggaaaaa 180
   tctaataatc caatcaaaaa atgggcaaaa tttgaataga catttttcaa aagaagacat 240
   acaaatgcca cataggcata tgataaggtg ctcaacatca ctggtcatta gagaaatgca 300
   aatcaaaacc acaatgagat atcatctcac cccagctaaa atggttttta tccaaaagac 360
   aggcaacaac aaatgccagc gagaatgtgg agaaaaggga acccttgtac actgttggtg 420
   taaattagtg caaccactat agagaacaat ttggaggttc ctcaaaacat taaaattaac 480
   attaaataga gctaccacaa tatccagaaa tccccatgct gggtatatac ctggaagaaa 540
   ggaaatcata tattgaagag ataacatcac tccaatattc acaatagcca ctattcacaa 600
   atgccaagat ttggaagcaa cctaagtgtc catcaacaga tgaatggata aagaaagtac 660
   tccaattata cacaatggag cacaattcag ccatgaaaaa agcatgagat cctgttatct 720
   gtaataatat ggatggaact ggaggtcatc atgttaagtg aaataagcca ggcacagaaa 780
   cacagatatt gcaagttctc acatacttgt gggatct                          817
<210> 122
   <211> 1126
   <212> DNA
   <213> Homo sapiens
<400> 122
   cattaaaatt aacattaaat agagctacca caatatccag aaatccccat gctgggtata 60
   tacctggaag aaaggaaatc atatattgaa gagataacat cactccaata ttcacaatag 120
   ccactattca caaatgccaa gatttggaag caacctaagt gtccatcaac agatgaatgg 180
   ataaagaaag tactccaatt atacacaatg gagcacaatt cagccatgaa aaaagcatga 240
   gatcctgtta tctgtaataa tatggatgga actggaggtc atcatgttaa gtgaaataag 300
   ccaggcacag aaacacagat attgcaagtt ctcacatact tgtgggatct acaaatcaaa 360
   acaactgagc taatgtctgg gccttagtca gtgttgtacc caagtactgg gagcacagct 420
   tttaaaatac atcatgaatg ctttaataca ggaatgaata gatgagaggc acaaactggt 480
   tgggtgttct tctgatacac agtatcttcc ttgacagatt cagtacaact ctcaacaggt 540
   aagtctcttc atgttatgtt accttatgag gaattaagtg gcagaacatg atttctatta 600
   ttttcctttg cagaacaaga ccaactttat tagttgggac acagtgtggc tgcatttgag 660
   tcccaagcaa ccattagtct atagctatca ccacagagtc agaggggatg agacgcccag 720
   caatctcacc caagacaact ccaccaacat tcctggttac ccaccatgtg tacagtaccc 780
   tgctaggaac cagggtcatg aaagtaaata ataccagact gtgcccttga ggagctcacc 840
   tctgctaagg gaaacaggca tagaaactta caatggtggt agagagaaaa gaggacaata 900
   ggactgtgtg agggggatag gaggcaccca gaggaggaaa tggttacatt tgtgtgagga 960
   ggttggtaag gaaaaatttt agcagaaggg gtctgtctgg ctgggcttgg aaggatacgt 1020
   aggagtcatc tagagggcac aggtacactc caggcagagg gaatttcgtg ggtaaagatg 1080
   tgtaggtgtg gcttgtgagg atggatttca attattctag aatgaa 1126
<210> 123
   <211> 624
   <212> DNA
   <213> Homo sapiens
<400> 123
   gattaagctt ttcatgattc ctcatagaac atgaactcaa aagaggtcag caaaggggtg 60
   tgtgcgattc tttgctattg gctgcagcta tagccctgcc tccttctcca gcacataaat 120
   ctttcagcag cttggctgaa gactgctgtg cagggcagag aagctccagg caaacagccc 180
   agcaaacagc agcactcagc taaaaggaag actcacagaa cacagttgaa gaaggaaagt 240
   ggcgatggac ctcatcccaa atttggcggt ggaaacctgg cttctcctgg ctgtcagcct 300
   ggtgctcctc tatctgtgag taactgtcca aactcctctc tttgtttcct tggacttggg 360
   gtgctaatcg ggcccctttt cccttatctg ttttgaagat caaaagagat gttcaaggag 420
   aagtagctga agtgttggac gctacaaacg catagaagtt attattatct tatgcagatc 480
   tatgaatgaa taaataagca tttctcccat ccaccttcta attttggtga ctaggagggt 540
   ttagggacag catttggtag tgggaatgat ttgattagct tagatctgac gaagactaat 600
   caatgaaaac atggcagcgg caga 624
<210> 124
   <211> 624
   <212> DNA
   <213> Homo sapiens
<400> 124
   gattaagctt ttcatgattc ctcatagaac atgaactcaa aagaggtcag caaaggggtg 60
   tgtgcgattc tttgctattg gctgcagcta tagccctgcc tccttctcca gcacataaat 120
   ctttcagcag cttggctgaa gactgctgtg cagggcaggg aagctccagg taaacagccc 180
   agcaaacagc agcactcagc taaaaggaag actcacagaa cacagttgaa gaaggaaagt 240
   ggcgatggac ctcatcccaa atttggcggt ggaaacctgg cttctcctgg ctgtcagcct 300
   ggtgctcctc tatctgtgag taactgtcca aactcctctc tttgtttcct tggacttggg 360
   gtgctaatcg ggcccctttt cccttatctg ttttgaagat caaaagagat gttcaaggag 420
   aagtagctga agtgttggac gctacaaacg catagaagtt attattatct tatgcagatc 480
   tatgaatgaa taaataagca tttctcccat ccaccttcta attttggtga ctaggagggt 540
   ttagggacag catttggtag tgggaatgat ttgattagct tagatctgac gaagactaat 600
   caatgaaaac atggcagcgg caga 624
<210> 125
   <211> 621
   <212> DNA
   <213> Homo sapiens
<400> 125
   gattaagctt ttcatgattc ctcatagaac atgaactcaa aagaggtcag caaaggggtg 60
   tgtgcgattc tttgctattg gctgcagcta tagccctgcc tccttctcca gcacataaat 120
   ctttcagcag cttggctgaa gactgctgtg cagggcaggg aagctccagg caaacagccc 180
   agcaaacagc agcactcagc taaaaggaag actcacagaa cacagttgaa gaaggaaagt 240
   ggcgatggac ctcatcccaa atttggcggt ggaaacctgg cttctcctgg ctgtcagcct 300
   ggtgctcctc tatctgtgag taactgtcca aactcctctc tttgtttcct tggacttggg 360
   gtgctaatcg ggcccctttt cccttatctg ttttgaagat caaaagagat gttcaagaag 420
   tagctgaagt gttggacgct acaaacgcat agaagttatt attatcttat gcagatctat 480
   gaatgaataa ataagcattt ctcccatcca ccttctaatt ttggtgacta ggagggttta 540
   gggacagcat ttggtagtgg gaatgatttg attagcttag atctgacgaa gactaatcaa 600
   tgaaaacatg gcagcggcag a                                            621
<210> 126
   <211> 465
   <212> DNA
   <213> Homo sapiens
<400> 126
   aggaaaggac ctgatgagtg aatgcaatta ctgatgttgg agttgctgtt attatttatc 60
   gtgtacatat tacctccctc tcttgaccat tccagttcct gagtaactca ccagccctct 120
   gatctataaa gtcacaatcc ctgtgacctg atttctgttt cactttgtag atatgggacc 180
   cgtacatatg ggacttttta agagactggg aattccaggg cccacacctc tgcctttgtt 240
   gggaaatgtt ttgtcctatc gtcaggtgag ttgcttgagc ttcctctttt gcttcttatg 300
   gttgcaaaca tcagcttagt tccatcagta aaaatgcccc tccttgggag ggagttctga 360
   ggtttcacat tttcagaaat ggtgggactg ggtgcagtgg atcatgcctg taatctcagc 420
   ctctgtgagg ccaagactgg caaattgctt gagcccagga gtttg                  465
<210> 127
   <211> 33
   <212> PRT
   <213> Homo sapiens
<400> 127
   Met Asp Leu Ile Pro Asn Leu Ala Val Glu Thr Trp Leu Leu Leu Ala
   1               5                   10                  15
   Val Ser Leu Val Leu Leu Tyr Leu Tyr Gly Thr Arg Thr Tyr Gly Thr
               20                  25                  30
   Phe
<210> 128
   <211> 602
   <212> DNA
   <213> Homo sapiens
<400> 128
   catagacaag ggtgagtcct tcagtactta gagaaaattc aagagtgact ttaaattccc 60
   cacttcaaat atattctctg ttttcttgtc tttcccttaa gacatctctg aatagcttcc 120
   ttcaactgcc agtgaaagat agtaggcctg atttcattgg acgcaactgt tttcagcccc 180
   aattagaggt agggtttatt ctatttaaaa taataatcaa cttgtatttt gtttcctctc 240
   ccagggtctc tggaaatttg acacagagtg ctataaaaag tatggaaaaa tgtgggggtg 300
   agtattctga aaacctccat tggatagacc tgctactgtg aggaggttac cccactgcag 360
   gatagtctct gcccaggtct tcatgggatg aagctcttgt caacctaaat acaaacagag 420
   agaggttctc tgaaagaaga ggataattac ttgggagtag aatattgcaa tgggaatctg 480
   cttgccgtta taaactatgt gcaaattcag ggaggtaaac aagacaaaga tgctccatag 540
   aaaatatgag aagaatctca taactgtttt gagataatta ttgttagcta caaagatcaa 600
   ta                                                                602
<210> 129
   <211> 594
   <212> DNA
   <213> Homo sapiens
<400> 129
   cagtatctct tccctgtttg gaccacatta cccttcatca tatgaagcct tgggtggctc 60
   ctgtgtgaga ctcttgctgt gtgtcacacc ctaatgaact agaacctaag gttgctgtgt 120
   gtcgtacaac taggggtatg gattacataa cataatgatc aaagtctggc ttcctgggtg 180
   tggctccagc tgcagaatag ggctagtgaa gtttaatcag ctccgttgtc cccacacaga 240
   acgtatgaag gtcaactccc tgtgctggcc atcacagatc ccgacgtgat cagaacagtg 300
   ctagtgaaag aatgttattc tgtcttcaca aatcgaaggg taagcatcca ttttttgaaa 360
   tttaaataat gattgatcca ctgattaaat ttttattttg aaaaaaacat atattcacag 420
   aaggttacct aaaaaatgta caggaaggtt ccatgtactc ttcatcctgt cccgcccagt 480
   ggtaacatct tgcaatcttg tatattgcaa tatatatcta gtatattcat attatcaggt 540
   tggcacaaaa gttaaaatgg caaactacag gctgggcata atggctcatg cctg       594
<210> 130
   <211> 594
   <212> DNA
   <213> Homo sapiens
<400> 130
   cagtatctct tccctgtttg gaccacatta cccttcatca tatgaagcct tgggtggctc 60
   ctgtgtgaga ctcttgctgt gtgtcacacc ctaatgaact agaacctaag gttgctgtgt 120
   gtcgtacaac taggggtatg gattacataa cataatgatc aaagtctggc ttcctgggtg 180
   tggctccagc tgcagaatcg ggctagtgaa gtttaatcag ctctgttgtc cccacacaga 240
   acgtatgaag gtcaactccc tgtgctggcc atcacagatc ccgacgtgat cagaacagtg 300
   ctagtgaaag aatgttattc tgtcttcaca aatcgaaggg taagcatcca ttttttgaaa 360
   tttaaataat gattgatcca ctgattaaat ttttattttg aaaaaaacat atattcacag 420
   aaggttacct aaaaaatgta caggaaggtt ccatgtactc ttcatcctgt cccgcccagt 480
   ggtaacatct tgcaatcttg tatattgcaa tatatatcta gtatattcat attatcaggt 540
   tggcacaaaa gttaaaatgg caaactacag gctgggcata atggctcatg cctg       594
<210> 131
   <211> 594
   <212> DNA
   <213> Homo sapiens
<400> 131
   cagtatctct tccctgtttg gaccacatta cccttcatca tatgaagcct tgggtggctc 60
   ctgtgtgaga ctcttgctgt gtgtcacacc ctaatgaact agaacctaag gttgctgtgt 120
   gtcgtacaac taggggtatg gattacataa cataatgatc aaagtctggc ttcctgggtg 180
   tggctccagc tgcagaatcg ggctagtgaa gtttaatcag ctccgttgtc cccacacaga 240
   acgtatgaag gtcaactccc tgtgctggcc atcacagatc ccgacgtgat cagaacagtg 300
   ctagtgaaag aatgttatta tgtcttcaca aatcgaaggg taagcatcca ttttttgaaa 360
   tttaaataat gattgatcca ctgattaaat ttttattttg aaaaaaacat atattcacag 420
   aaggttacct aaaaaatgta caggaaggtt ccatgtactc ttcatcctgt cccgcccagt 480
   ggtaacatct tgcaatcttg tatattgcaa tatatatcta gtatattcat attatcaggt 540
   tggcacaaaa gttaaaatgg caaactacag gctgggcata atggctcatg cctg 594
<210> 132
   <211> 60
   <212> PRT
   <213> Homo sapiens
<400> 132
   Met Trp Gly Thr Tyr Glu Gly Gln Leu Pro Val Leu Ala Ile Thr Asp
   1 5 10 15
   Pro Asp Val Ile Arg Thr Val Leu Val Lys Glu Cys Tyr Tyr Val Phe
   20 25 30
   Thr Asn Arg Arg Ser Leu Gly Pro Val Gly Phe Met Lys Ser Ala Ile
   35 40 45
   Ser Leu Ala Glu Asp Glu Glu Trp Lys Arg Ile Arg
   50 55 60
<210> 133
   <211> 601
   <212> DNA
   <213> Homo sapiens
<400> 133
   cagtatctct tccctgtttg gaccacatta cccttcatca tatgaagcct tgggtggctc 60
   ctgtgtgaga ctcttgctgt gtgtcacacc ctaatgaact agaacctaag gttgctgtgt 120
   gtcgtacaac taggggtatg gattacataa cataatgatc aaagtctggc ttcctgggtg 180
   tggctccagc tgcagaatcg ggctagtgaa gtttaatcag ctccgttgtc cccacacaga 240
   acgtatgaag gtcaactccc tgtgctggcc atcacagatc ccgacgtgat cagaacagtg 300
   ctagtgaaag aatgttattc tgtcttcaca aatcgaaggg taagcatcca ttttttgaaa 360
   tttaaataat gattgatcca ctgattaaat ttttattttg aaaaaaacat atattcacag 420
   aaggttacct aaaaaatgta cctaaaaaat gaaggttcca tgtactcttc atcctgtccc 480
   gcccagtggt aacatcttgc aatcttgtat attgcaatat atatctagta tattcatatt 540
   atcaggttgg cacaaaagtt aaaatggcaa actacaggct gggcataatg gctcatgcct 600
   g 601
<210> 134
   <211> 603
   <212> DNA
   <213> Homo sapiens
<400> 134
   agcggaaaac tcaaggaggt atgaaaataa gatgagtctt aattagaaat gtaaagaatg 60
   aatctgggga caggtagaaa gtaagatcac agtccgtttc caaggggtag tccactgagt 120
   tcgagcttcc taaaaatggt ctttgatctt tatgtacaga aaagacatca caaaattcat 180
   tacaaaatgt cacttactgc tccatgctgg agaaagccat atccttctgg gacttgagtc 240
   tgcacattta actacaggta ctgatctgtt ttgtgcttag atgttcccca tcattgccca 300
   gtatggagat gtattggtga gaaacttgag gcgggaagca gagaaaggca agcctgtcac 360
   cttgaaagag taagtaggag cacagccatg gggttctgag ctgtcatgag cccttccagc 420
   tgcctgccat ggagtcgaca gtcgcactgt tgggttactc cagtgaccag acaaaagcag 480
   ggcagcgctg caactccaaa gagccaccta agagggagtg gctcccatga ggcggcaagt 540
   cagcaaggga aaagggcctt ctctcctgtg cacaggagcc aggatttact tatctgttaa 600
   ctt                                                               603
<210> 135
   <211> 603
   <212> DNA
   <213> Homo sapiens
<400> 135
   agcggaaaac tcaaggaggt atgaaaataa gatgagtctt aattagaaat gtaaagaatg 60
   aatctgggga caggtagaaa gtaagatcac agtccgtttc caaggggtag tccactgagt 120
   tcgagcttcc taaaaatggt cttttatctt tatgtacaga aaagacatca caaaattcat 180
   tacaaaatgt cacttactgc tccatgctgg agaaagccat atccttctgg gacttgagtc 240
   cgcacattta actacaggta ctgatctgtt ttgtgcttag atgttcccca tcattgccca 300
   gtatggagat gtattggtga gaaacttgag gcgggaagca gagaaaggca agcctgtcac 360
   cttgaaagag taagtaggag cacagccatg gggttctgag ctgtcatgag cccttccagc 420
   tgcctgccat ggagtcgaca gtcgcactgt tgggttactc cagtgaccag acaaaagcag 480
   ggcagcgctg caactccaaa gagccaccta agagggagtg gctcccatga ggcggcaagt 540
   cagcaaggga aaagggcctt ctctcctgtg cacaggagcc aggatttact tatctgttaa 600
   ctt                                                               603
<210> 136
   <211> 626
   <212> DNA
   <213> Homo sapiens
<400> 136
   attggacatg atagctagat ttgtttcagg aaaacatcct gctttccaag gatttagatg 60
   aatgtttttg ttcactggtg actcaggtaa cacgtcttca agaagccata gggaggttga 120
   gggagggaag tcgtcaagaa gggaggttga ggactgcact tttgatttac ttctgacttc 180
   acgagtcact ttctgccaaa gaaatctctc cttttgcttc tagcaccgac tagatttcct 240
   tcagctgatg attgactccc agaattcgaa agaaactgag tcccacaaag gtaaccaagg 300
   agtgcttctg agggctactg gcggggacac taagagggag ggccttgttc tgaaaatgtg 360
   caggaagtat tccaggaaga tgagaatttt tgccacatag cagaacaaca cacatttaga 420
   tgttataaat ggtagctgga ggcactttcc agaagcccac aggtatagcc atgttccagg 480
   ctgaaagggc aaccctaagc aaacctagaa tgcttggagg acagtcagtg gtttgtggat 540
   cacctacatg agatcaaatg ccagttctca gcctcctcca gatccaccaa gtgagaacct 600
   ctacttggaa atttatatca aacata                                      626
<210> 137
   <211> 623
   <212> DNA
   <213> Homo sapiens
<400> 137
   attggacatg atagctagat ttgtttcagg aaaacatcct gctttccaag gatttagatg 60
   aatgtttttg ttcactggtg actcaggtaa cacgtcttca agaagccata gggaggttga 120
   gggagggaag tcaagaaggg aggttgagga ctgcactttt gatttacttc tgacttcacg 180
   agtcactttc tgccaaagaa atctctcctt ttgcttctag caccgactag atttccttca 240
   gctgatgatt gactcccaga attcgaaaga aactgagtcc cacaaaggta accaaggagt 300
   gcttctgagg gctactggcg gggacactaa gagggagggc cttgttctga aaatgtgcag 360
   gaattattcc aggaagatga gaatttttgc cacatagcag aacaacacac atttagatgt 420
   tataaatggt agctggaggc actttccaga agcccacagg tatagccatg ttccaggctg 480
   aaagggcaac cctaagcaaa cctagaatgc ttggaggaca gtcagtggtt tgtggatcac 540
   ctacatgaga tcaaatgcca gttctcagcc tcctccagat ccaccaagtg agaacctcta 600
   cttggaaatt tatatcaaac ata 623
<210> 138
   <211> 826
   <212> DNA
   <213> Homo sapiens
<400> 138
   agataaagta cttttaggat cattcaaggc acacacccat aacactgagt atgtaagaca 60
   gaaatgctct ctctggaaat tacagcagtg ctggtgctgg gatgccatga tgaggagtgt 120
   gtggcccaca atcatgtaga ccttgggaaa acctggatta aaatgatttt gcgtcatcct 180
   ggccctgtat aagatacata tcagaatgaa aaccactccc agtgtgactt tgaattgctt 240
   ttccattttt tcttcttggg attagagaac ttcacttaga tttcatctaa gctgtgatgt 300
   tgtacgttga cctgatttac ctaaaatgtc tttcctctcc tttcagctct gtctgatctg 360
   gagctcgcag cccagtcaat aatcttcatt tttgctggct atgaaaccac cagcagtgtt 420
   ctttccttca ctttatatga actggccact caccctgatg tccagcagaa actgcaaaag 480
   gagattgatg cagttttgcc caataaggtg aggggatgac ccctggagat gaagggaaga 540
   ggtgaagcct tagcaaaaat gcctcctcac cactccccag gagaattttt ataaaaagca 600
   taatcactga ttccttcact gacataatgt aggaagcctc tgaggagaaa aacaaaggga 660
   gaaacataga gaacggttgc tactggcaga agcataagat ctttgtacaa tattgctggc 720
   cctggttcac ctgtttactg ttatcacaat aatgctaagt aaaaaaaaaa aaaaaaaaaa 780
   aaaaaaaaaa aggagtgtgg cgagaagatg gccaaacagg aacagc 826
<210> 139
   <211> 795
   <212> DNA
   <213> Homo sapiens
<400> 139
   gtccctgggg tgaggatggt cttgaatatc tcctacattc ataactcctc cacacatctc 60
   agtaggtcac tgagcacatc aatggacatg ccagttatta aaatacttca cgaatactat 120
   gatcatttac cagtatgagt tattctctgg agcttctaat acttcagtag tactgcatgg 180
   actcagttga gagttaattc aaaatctcag attatccaat tctgtttctt tccttccagg 240
   caccacctac ctatgatgcc gtggtacaga tggagtacct tgacatggtg gtgaatgaaa 300
   cactcagatt attcccagtt gctattagac ttgagaggac ttgcaagaaa gatgttgaaa 360
   tcaatggggt attcattccc aaagggtcaa tggtggtgat tccaacttat gctcttcacc 420
   atgacccaaa gtactggaca gagcctgagg agttccgccc tgaaaggtac aagtctccag 480
   ggaaatggag ctcaccctga cccaggctgg ttcaagcata ttctgcctct cttaatctac 540
   atgacaatcg tgtggttgta caatcatttg cttgtaagtc tttttatcac aaaaaagtga 600
   taattatcaa actttacaaa ccacagacta gaaaaaacga aactacatcc atccacagtc 660
   ccagcacaag acaaagataa tcaattatgt ccctgtgggc atttttctac gcctatatag 720
   atttttaaaa attagaatgg tatcactttt tatttggttt gaattgctgc ttacttgatt 780
   taacaggaaa ctatc                                                  795
<210> 140
   <211> 796
   <212> DNA
   <213> Homo sapiens
<400> 140
   gtccctgggg tgaggatggt cttgaatatc tcctacattc ataactcctc cacacatctc 60
   agtaggtcac tgagcacatc aatggacatg ccagttatta aaatacttca cgaatactat 120
   gatcatttac cagtatgagt tattctctgg agcttctaat acttcaatag tactgcatgg 180
   actcagttga gagttaattc aaaatctcag attatccaat tctgtttctt tccttccagg 240
   caccacctta cctatgatgc cgtggtacag atggagtacc ttgacatggt ggtgaatgaa 300
   acactcagat tattcccagt tgctattaga cttgagagga cttgcaagaa agatgttgaa 360
   atcaatgggg tattcattcc caaagggtca atggtggtga ttccaactta tgctcttcac 420
   catgacccaa agtactggac agagcctgag gagttccgcc ctgaaaggta caagtctcca 480
   gggaaatgga gctcaccctg acccaggctg gttcaagcat attctgcctc tcttaatcta 540
   catgacaatc gtgtggttgt acaatcattt gcttgtaagt ctttttatca caaaaaagtg 600
   ataattatca aactttacaa accacagact agaaaaaacg aaactacatc catccacagt 660
   cccagcacaa gacaaagata atcaattatg tccctgtggg catttttcta cgcctatata 720
   gatttttaaa aattagaatg gtatcacttt ttatttggtt tgaattgctg cttacttgat 780
   ttaacaggaa actatc                                                 796
<210> 141
   <211> 59
   <212> PRT
   <213> Homo sapiens
<400> 141
   Ala Leu Ser Asp Leu Glu Leu Ala Ala Gln Ser Ile Ile Phe Ile Phe
   1               5                   10                  15
   Ala Gly Tyr Glu Thr Thr Ser Ser Val Leu Ser Phe Thr Leu Tyr Glu
               20                  25                  30
   Leu Ala Thr His Pro Asp Val Gln Gln Lys Leu Gln Lys Glu Ile Asp
           35                  40                  45
   Ala Val Leu Pro Asn Lys Ala Pro Pro Tyr Leu
<210> 142
   <211> 795
   <212> DNA
   <213> Homo sapiens
<400> 142
   gtccctgggg tgaggatggt cttgaatatc tcctacattc ataactcctc cacacatctc 60
   agtaggtcac tgagcacatc aatggacatg ccagttatta aaatacttca cgaatactat 120
   gatcatttac cagtatgagt tattctctgg agcttctaat acttcaatag tactgcatgg 180
   actcagttga gagttaattc aaaatctcag attatccaat tctgtttctt tccttccagg 240
   caccacctac ctatgatgcc gtggtacaga tggagtacct tgacatggtg gtgaatgaaa 300
   cactcagatt attcccagtt gctattagac ttgagaggac ttgcaagaaa gatgttgaaa 360
   tcaatggggt attcattccc aaagggtcaa tggtggtgat tccaacttat gctcttcacc 420
   atgacccaaa gtactggaca gagcctgagg agttccgccc tgaaaggtac aagtctccag 480
   ggaaatggag ctcaccctga cccaggctgg ttcaagcata ttctgcctct cttaatctac 540
   atgacaatcg tgtggttgta caatcatttg cttgtaagtc tttttatcac aaaaaagtga 600
   taattatcaa actttacaaa ccacagacta gaaaaaacga aattacatcc atccacagtc 660
   ccagcacaag acaaagataa tcaattatgt ccctgtgggc atttttctac gcctatatag 720
   atttttaaaa attagaatgg tatcactttt tatttggttt gaattgctgc ttacttgatt 780
   taacaggaaa ctatc                                                  795
<210> 143
   <211> 616
   <212> DNA
   <213> Homo sapiens
<400> 143
   caggcctggc acagagtcag tgctccataa atattttgtt aaacgatgga tggtgagtgc 60
   ttttactatc cagtatttac ccagcttata gattaagtat gaagagttca agatacatgg 120
   tgttaagagt cgtttttata tgcttgcaaa gcatttttgt catatttttt ctactttgct 180
   tccatctttt cttctttcac ttcatttatt aattctccat atgcttgttt aactattgta 240
   gatccccttg aaattagaca cgcaaggact tcttcaacca gaaaaaccca ttgttctaaa 300
   ggtggattca agagatggaa ccctaagtgg agaatgagtt attctaagga cttctacttt 360
   ggtcttcaag aaagctgtgc cccagaacac cagagatttc aacttagtca ataaaacctt 420
   gaaataaaga tgggcttaat ctaatgtact gcatgagtag ttggtgattt tgtacattca 480
   ttgagctctc ccagagtctg tgtagagtgt tgtgcattat gtagtataaa ggaggtgacc 540
   aggtaagtga cagataggta gactcagctt ctctgcttct cataggacta cctctaccca 600
   cctctagtta gcatta                                                 616
<210> 144
   <211> 1508
   <212> DNA
   <213> Homo sapiens
<400> 144
   ccatggctct gttattagca gtttttctgg tgctcctcta tctatatggg acccgtacac 60
   atggactttt taagagactg ggaattccag ggcccacacc tctgcctttg ttgggaaatg 120
   ttttgtccta tcgtcagggt ctctggaaat ttgacacaga gtgctataaa aagtatggaa 180
   aaatgtgggg aacgtatgaa ggtcaactcc ctgtgctggc catcacagat cccgacgtga 240
   tcagaacagt gctagtgaaa gaatgttatt ctgtcttcac aaatcgaagg tctttaggcc 300
   cagtgggatt tatgaaaagt gccatctctt tagctgagga tgaagaatgg aagagaatac 360
   ggtcattgct gtctccaacc ttcaccagcg gaaaactcaa ggagatgttc cccatcattg 420
   cccagtatgg agatgtattg gtgagaaact tgaggcggga agcagagaaa ggcaagcctg 480
   tcaccttgaa agacatcttt ggggcctaca gcatggatgt gattactggc acatcatttg 540
   gagtgaacat cgactctctc aacaatccac aagacccctt tgtggagagc actaagaagt 600
   tcctaaaatt tggtttctta gatccattat ttctctcaat aatactcttt ccattcctta 660
   ccccagtttt tgaagcatta aatgtctctc tgtttccaaa agataccata aattttttaa 720
   gtaaatctgt aaacagaatg aagaaaagtc gcctcaacga caaacaaaag caccgactag 780
   atttccttca gctgatgatt gactcccaga attcgaaaga aactgagtcc cacaaagctc 840
   tgtctgatct ggagctcgca gcccagtcaa taatcttcat ttttgctggc tatgaaacca 900
   ccagcagtgt tctttccttc actttatatg aactggccac tcaccctgat gtccagcaga 960
   aactgcaaaa ggagattgat gcagttttgc ccaataaggc accacctacc tatgatgccg 1020
   tggtacagat ggagtacctt gacatggtgg tgaatgaaac actcagatta ttcccagttg 1080
   ctattagact tgagaggact tgcaagaaag atgttgaaat caatggggta ttcattccca 1140
   aagggtcaat ggtggtgatt ccaacttatg ctcttcacca tgacccaaag tactggacag 1200
   agcctgagga gttccgccct gaaaggttca gtaagaagaa ggacagcata gatccttaca 1260
   tatacacacc ctttggaact ggacccagaa actgcattgg catgaggttt gctctcatga 1320
   acatgaaact tgctctaatc agagtccttc agaacttctc cttcaaacct tgtaaagaaa 1380
   cacagatccc cttgaaatta gacacgcaag gacttcttca accagaaaaa cccattgttc 1440
   taaaggtgga ttcaagagat ggaaccctaa gtggagaaca tcaccatcac catcactgag 1500
   atctgcag                                                          1508
<210> 145
   <211> 60
   <212> PRT
   <213> Homo sapiens
<400> 145
   Pro Val Ala Ile Arg Leu Glu Arg Thr Cys Lys Lys Asp Val Glu Ile
   1               5                   10                  15
   Asn Gly Val Phe Ile Pro Lys Gly Ser Met Val Val Ile Pro Asn Tyr
               20                  25                  30
   Ala Leu His His Asp Pro Lys Tyr Trp Thr Glu Pro Glu Glu Phe Arg
           35                  40                  45
   Pro Glu Arg Phe Ser Lys Lys Lys Asp Ser Ile Asp
       50                  55                  60
<210> 146
   <211> 11
   <212> DNA
   <213> Homo sapiens
<400> 146
   tttcagtatc t 11
<210> 147
   <211> 11
   <212> DNA
   <213> Homo sapiens
<400> 147
   tttcaatatc t 11
<210> 148
   <211> 11
   <212> DNA
   <213> Homo sapiens
<400> 148
   tttaatagaa g 11
<210> 149
   <211> 11
   <212> DNA
   <213> Homo sapiens
<400> 149
   tttaacagaa g 11
<210> 150
   <211> 11
   <212> DNA
   <213> Homo sapiens
<400> 150
   attccccata g 11
<210> 151
   <211> 11
   <212> DNA
   <213> Homo sapiens
<400> 151
   attcctcata g 11
<210> 152
   <211> 11
   <212> DNA
   <213> Homo sapiens
<400> 152
   tagaatatga a 11
<210> 153
   <211> 11
   <212> DNA
   <213> Homo sapiens
<400> 153
   tagaacatga a 11
<210> 154
   <211> 11
   <212> DNA
   <213> Homo sapiens
<400> 154
   gtaacttatc c 11
<210> 155
   <211> 11
   <212> DNA
   <213> Homo sapiens
<400> 155
   gtaacgtatc c 11
<210> 156
   <211> 11
   <212> DNA
   <213> Homo sapiens
<400> 156
   ttcacgtgga g 11
<210> 157
   <211> 11
   <212> DNA
   <213> Homo sapiens
<400> 157
   ttcacatgga g 11
<210> 158
   <211> 11
   <212> DNA
   <213> Homo sapiens
<400> 158
   tggacgcaac t 11
<210> 159
   <211> 11
   <212> DNA
   <213> Homo sapiens
<400> 159
   tggacacaac t 11
<210> 160
   <211> 11
   <212> DNA
   <213> Homo sapiens
<400> 160
   gaggataatt a 11
<210> 161
   <211> 11
   <212> DNA
   <213> Homo sapiens
<400> 161
   gaggaaaatt a 11
<210> 162
   <211> 11
   <212> DNA
   <213> Homo sapiens
<400> 162
   catcattgcc c 11
<210> 163
   <211> 11
   <212> DNA
   <213> Homo sapiens
<400> 163
   catcactgcc c 11
<210> 164
   <211> 11
   <212> DNA
   <213> Homo sapiens
<400> 164
   cagtcgcact g 11
<210> 165
   <211> 11
   <212> DNA
   <213> Homo sapiens
<400> 165
   cagtcacact g 11
<210> 166
   <211> 11
   <212> DNA
   <213> Homo sapiens
<400> 166
   actaagaagt t 11
<210> 167
   <211> 11
   <212> DNA
   <213> Homo sapiens
<400> 167
   actaaaaagt t 11
<210> 168
   <211> 11
   <212> DNA
   <213> Homo sapiens
<400> 168
   gatccattat t 11
<210> 169
   <211> 11
   <212> DNA
   <213> Homo sapiens
<400> 169
   gatccgttat t 11
<210> 170
   <211> 11
   <212> DNA
   <213> Homo sapiens
<400> 170
   caattccatt g 11
<210> 171
   <211> 11
   <212> DNA
   <213> Homo sapiens
<400> 171
   caatttcatt g 11
<210> 172
   <211> 11
   <212> DNA
   <213> Homo sapiens
<400> 172
   tgtcaatcta g 11
<210> 173
   <211> 11
   <212> DNA
   <213> Homo sapiens
<400> 173
   tgtcagtcta g 11
<210> 174
   <211> 11
   <212> DNA
   <213> Homo sapiens
<400> 174
   ttgtttgttt t 11
<210> 175
   <211> 11
   <212> DNA
   <213> Homo sapiens
<400> 175
   ttgttcgttt t 11
<210> 176
   <211> 11
   <212> DNA
   <213> Homo sapiens
<400> 176
   aaataaagaa g 11
<210> 177
   <211> 11
   <212> DNA
   <213> Homo sapiens
<400> 177
   aaatacagaa g 11
<210> 178
   <211> 11
   <212> DNA
   <213> Homo sapiens
<400> 178
   tttgcgtcat c 11
<210> 179
   <211> 11
   <212> DNA
   <213> Homo sapiens
<400> 179
   tttgcatcat c 11
<210> 180
   <211> 11
   <212> DNA
   <213> Homo sapiens
<400> 180
   ttgacctgat t 11
<210> 181
   <211> 11
   <212> DNA
   <213> Homo sapiens
<400> 181
   ttgacttgat t 11
<210> 182
   <211> 11
   <212> DNA
   <213> Homo sapiens
<400> 182
   tattgtagat c 11
<210> 183
   <211> 11
   <212> DNA
   <213> Homo sapiens
<400> 183
   tattgcagat c 11
<210> 184
   <211> 11
   <212> DNA
   <213> Homo sapiens
<400> 184
   ccttttccct t 11
<210> 185
   <211> 11
   <212> DNA
   <213> Homo sapiens
<400> 185
   cctttcccct t 11
<210> 186
   <211> 11
   <212> DNA
   <213> Homo sapiens
<400> 186
   cttatgcaga t 11
<210> 187
   <211> 11
   <212> DNA
   <213> Homo sapiens
<400> 187
   cttatacaga t 11
<210> 188
   <211> 11
   <212> DNA
   <213> Homo sapiens
<400> 188
   aatacggtca t 11
<210> 189
   <211> 11
   <212> DNA
   <213> Homo sapiens
<400> 189
   aatacagtca t 11
<210> 190
   <211> 11
   <212> DNA
   <213> Homo sapiens
<400> 190
   ggaggtatga a 11
<210> 191
   <211> 11
   <212> DNA
   <213> Homo sapiens
<400> 191
   ggaggcatga a 11
<210> 192
   <211> 11
   <212> DNA
   <213> Homo sapiens
<400> 192
   agtccgtttc c 11
<210> 193
   <211> 11
   <212> DNA
   <213> Homo sapiens
<400> 193
   agtccatttc c 11
<210> 194
   <211> 11
   <212> DNA
   <213> Homo sapiens
<400> 194
   tctgccaaag a 11
<210> 195
   <211> 11
   <212> DNA
   <213> Homo sapiens
<400> 195
   tctgcgaaag a 11
<210> 196
   <211> 11
   <212> DNA
   <213> Homo sapiens
<400> 196
   acccattgtt c 11
<210> 197
   <211> 11
   <212> DNA
   <213> Homo sapiens
<400> 197
   acccactgtt c 11
<210> 198
   <211> 11
   <212> DNA
   <213> Homo sapiens
<400> 198
   gctactggct g 11
<210> 199
   <211> 11
   <212> DNA
   <213> Homo sapiens
<400> 199
   gctaccggct g 11
<210> 200
   <211> 11
   <212> DNA
   <213> Homo sapiens
<400> 200
   gaaatcaccc g 11
<210> 201
   <211> 11
   <212> DNA
   <213> Homo sapiens
<400> 201
   gaaattaccc g 11
<210> 202
   <211> 33
   <212> DNA
   <213> Homo sapiens
<400> 202
   gctctactgt catttctaac cataatctct tta 33
<210> 203
   <211> 27
   <212> DNA
   <213> Homo sapiens
<400> 203
   gcttcatatg atgaagggta atgtggt 27
<210> 204
   <211> 18
   <212> DNA
   <213> Homo sapiens
<400> 204
   tgtctttcag tatctctt 18
<210> 205
   <211> 19
   <212> DNA
   <213> Homo sapiens
<400> 205
   tgtctttcaa tatctcttc 19
<210> 206
   <211> 444
   <212> DNA
   <213> Homo sapiens
<400> 206
   catttagtcc ttgtgagcac ttgatgattt acctgccttc aatttttcac tgacctaata 60
   ttctttttga taatgaagta ttttaaacat ataaaacatt atggagagtg gcataggaga 120
   tacccacgta tgtaccaccc agcttaacga atgctctact gtcatttcta accataatct 180
   ctttaaagag ctcttttgtc tttcaatatc tcttccctgt ttggaccaca ttacccttca 240
   tcatatgaag ccttgggtgg ctcctgtgtg agactcttgc tgtgtgtcac accctaatga 300
   actagaacct aaggttgctg tgtgtcgtac aactaggggt atggattaca taacataatg 360
   atcaaagtct ggcttcctgg gtgtggctcc agctgcagaa tcgggctagt gaagtttaat 420
   cagctccgtt gtccccacac agaa 444
<210> 207
   <211> 830
   <212> DNA
   <213> Homo sapiens
<400> 207
   tggtcaccca ccatgtgtac agtaccctgc tagggtccag ggtcatgaaa gtaaataata 60
   ccagactgtg cccttgagga actcacctct gctaagggaa acaggcacag aaacccacaa 120
   gggtggtaga gaggaaatag gacaatagga ctgtgtgagg gggataggag gcacccagag 180
   gaggaaatgg ttacatctgt gtgaggaggt tggtaaggaa agactttaac agaaggggtc 240
   tgtctggctg ggcttgcaag gatgtgtagg agtcatctag ggggcacaag tacactccag 300
   gcagagggaa ttgcatgggt aaagatctgc agttgtggct tgtggggatg gatttcaagt 360
   attctggaat gaagacagcc atggaaacaa gggcaggtga gaggatattt aagaggcttc 420
   atgccaatgg ctccacttca gtttctgata agaactcagg ttccgtggac tccctgataa 480
   aactgattaa gttgtttatg attccccata gaatatgaac tcaaaggagg taagcaaagg 540
   ggtgtgtgcg attctttgct actggctgca gctgcagccc cacctccttc tccagcacat 600
   aaacatttca gcagcttgac ctaagactgc tgtgcagggc agggatgctc caggcagaca 660
   gcccagcaaa caacagcaca cagctgaaag taagactcag aggagacagt tgaagaaggc 720
   aagtggcgat ggacctcatc ccaaatttgg cggtggaaac ctggcttctc ctggctgtca 780
   gcctggtgct cctctatctg tcagtaactg tccagattcc tctcctctgt 830
<210> 208
   <211> 830
   <212> DNA
   <213> Homo sapiens
<400> 208
   tggtcaccca ccatgtgtac agtaccctgc tagggtccag ggtcatgaaa gtaaataata 60
   ccagactgtg cccttgagga actcacctct gctaagggaa acaggcacag aaacccacaa 120
   gggtggtaga gaggaaatag gacaatagga ctgtgtgagg gggataggag gcacccagag 180
   gaggaaatgg ttacatctgt gtgaggaggt tggtaaggaa agactttaat agaaggggtc 240
   tgtctggctg ggcttgcaag gatgtgtagg agtcatctag ggggcacaag tacactccag 300
   gcagagggaa ttgcatgggt aaagatctgc agttgtggct tgtggggatg gatttcaagt 360
   attctggaat gaagacagcc atggaaacaa gggcaggtga gaggatattt aagaggcttc 420
   atgccaatgg ctccacttca gtttctgata agaactcagg ttccgtggac tccctgataa 480
   aactgattaa gttgtttatg attcctcata gaatatgaac tcaaaggagg taagcaaagg 540
   ggtgtgtgcg attctttgct actggctgca gctgcagccc cacctccttc tccagcacat 600
   aaacatttca gcagcttgac ctaagactgc tgtgcagggc agggatgctc caggcagaca 660
   gcccagcaaa caacagcaca cagctgaaag taagactcag aggagacagt tgaagaaggc 720
   aagtggcgat ggacctcatc ccaaatttgg cggtggaaac ctggcttctc ctggctgtca 780
   gcctggtgct cctctatctg tcagtaactg tccagattcc tctcctctgt            830
<210> 209
   <211> 830
   <212> DNA
   <213> Homo sapiens
<400> 209
   tggtcaccca ccatgtgtac agtaccctgc tagggtccag ggtcatgaaa gtaaataata 60
   ccagactgtg cccttgagga actcacctct gctaagggaa acaggcacag aaacccacaa 120
   gggtggtaga gaggaaatag gacaatagga ctgtgtgagg gggataggag gcacccagag 180
   gaggaaatgg ttacatctgt gtgaggaggt tggtaaggaa agactttaat agaaggggtc 240
   tgtctggctg ggcttgcaag gatgtgtagg agtcatctag ggggcacaag tacactccag 300
   gcagagggaa ttgcatgggt aaagatctgc agttgtggct tgtggggatg gatttcaagt 360
   attctggaat gaagacagcc atggaaacaa gggcaggtga gaggatattt aagaggcttc 420
   atgccaatgg ctccacttca gtttctgata agaactcagg ttccgtggac tccctgataa 480
   aactgattaa gttgtttatg attccccata gaacatgaac tcaaaggagg taagcaaagg 540
   ggtgtgtgcg attctttgct actggctgca gctgcagccc cacctccttc tccagcacat 600
   aaacatttca gcagcttgac ctaagactgc tgtgcagggc agggatgctc caggcagaca 660
   gcccagcaaa caacagcaca cagctgaaag taagactcag aggagacagt tgaagaaggc 720
   aagtggcgat ggacctcatc ccaaatttgg cggtggaaac ctggcttctc ctggctgtca 780
   gcctggtgct cctctatctg tcagtaactg tccagattcc tctcctctgt            830
<210> 210
   <211> 837
   <212> DNA
   <213> Homo sapiens
<400> 210
   caaaaattag ctaggtgtgg tggtatgcac ctataatctc agctacccag gaggctgagg 60
   caggagaatc acttgaacct ggaggcagag gttgcagtga gccgagacgc accattacac 120
   tccagcctgg gtgacagagt gagattccat ctcaaaaaaa aaaaaaaaaa attatgcctt 180
   tttgaagcac atacatttta taacatacaa ctgaatccct tattatatta ttagttttga 240
   tttaatgttt tcaaaccatc tcccctgata tttctgggag atgggaaaca tgttttctta 300
   cacctcttgc attccattct caactcccaa ctgtcttact gcaatgaaca cttaataaga 360
   aacagtcaat tggtcaattg attgggcaac aggctaaaca cactcattcc ttgtctgttc 420
   ccacttcttt ctttactttc ccttcctgag taacgtatcc taaagtcatt aggtgggtgg 480
   cagccagatg gtggccacac attaaggtag aaaagagagt gtcatgatgg ttccaagtca 540
   gagacctagt agggtgagga tcaagtaggt gttcacgtgg agaaacagcc cggcctgtgt 600
   gtgggagtcc aagcaagcag agaaaatgtc gacacagagg ggtggcctga aaaagcagcc 660
   agagcctaaa cagggcatgg agaacatatt tagggcatga ggtgaggagg gcatccatga 720
   gtgggaaggg atgggtgagg tttcactaca taaaggggat tgatgaaata agtaaataaa 780
   gtatactgga agccaggtgt gtcacttttg cagaaaagag tcatggattc agaaagg    837
<210> 211
   <211> 837
   <212> DNA
   <213> Homo sapiens
<400> 211
   caaaaattag ctaggtgtgg tggtatgcac ctataatctc agctacccag gaggctgagg 60
   caggagaatc acttgaacct ggaggcagag gttgcagtga gccgagacgc accattacac 120
   tccagcctgg gtgacagagt gagattccat ctcaaaaaaa aaaaaaaaaa attatgcctt 180
   tttgaagcac atacatttta taacatacaa ctgaatccct tattatatta ttagttttga 240
   tttaatgttt tcaaaccatc tcccctgata tttctgggag atgggaaaca tgttttctta 300
   cacctcttgc attccattct caactcccaa ctgtcttact gcaatgaaca cttaataaga 360
   aacagtcaat tggtcaattg attgggcaac aggctaaaca cactcattcc ttgtctgttc 420
   ccacttcttt ctttactttc ccttcctgag taacttatcc taaagtcatt aggtgggtgg 480
   cagccagatg gtggccacac attaaggtag aaaagagagt gtcatgatgg ttccaagtca 540
   gagacctagt agggtgagga tcaagtaggt gttcacatgg agaaacagcc cggcctgtgt 600
   gtgggagtcc aagcaagcag agaaaatgtc gacacagagg ggtggcctga aaaagcagcc 660
   agagcctaaa cagggcatgg agaacatatt tagggcatga ggtgaggagg gcatccatga 720
   gtgggaaggg atgggtgagg tttcactaca taaaggggat tgatgaaata agtaaataaa 780
   gtatactgga agccaggtgt gtcacttttg cagaaaagag tcatggattc agaaagg    837
<210> 212
   <211> 602
   <212> DNA
   <213> Homo sapiens
<400> 212
   catagacaag ggtgagtcct tcagtactta gagaaaattc aagagtgact ttaaattccc 60
   cacttcaaat atattctctg ttttcttgtc tttcccttaa gacatctctg aatagcttcc 120
   ttcaactgcc agtgaaagat agcaggcctg atttcattgg acacaactgt tttcagcccc 180
   aattagaggt agggtttatt ctatttaaaa taataatcaa cttgtatttt gtttcctctc 240
   ccagggtctc tggaaatttg acacagagtg ctataaaaag tatggaaaaa tgtgggggtg 300
   agtattctga aaacctccat tggatagacc tgctactgtg aggaggttac cccactgcag 360
   gatagtctct gcccaggtct tcatgggatg aagctcttgt caacctaaat acaaacagag 420
   agaggttctc tgaaagaaga ggataattac ttgggagtag aatattgcaa tgggaatctg 480
   cttgccgtta taaactatgt gcaaattcag ggaggtaaac aagacaaaga tgctccatag 540
   aaaatatgag aagaatctca taactgtttt gagataatta ttgttagcta caaagatcaa 600
   ta                                                                602
<210> 213
   <211> 602
   <212> DNA
   <213> Homo sapiens
<400> 213
   catagacaag ggtgagtcct tcagtactta gagaaaattc aagagtgact ttaaattccc 60
   cacttcaaat atattctctg ttttcttgtc tttcccttaa gacatctctg aatagcttcc 120
   ttcaactgcc agtgaaagat agcaggcctg atttcattgg acgcaactgt tttcagcccc 180
   aattagaggt agggtttatt ctatttaaaa taataatcaa cttgtatttt gtttcctctc 240
   ccagggtctc tggaaatttg acacagagtg ctataaaaag tatggaaaaa tgtgggggtg 300
   agtattctga aaacctccat tggatagacc tgctactgtg aggaggttac cccactgcag 360
   gatagtctct gcccaggtct tcatgggatg aagctcttgt caacctaaat acaaacagag 420
   agaggttctc tgaaagaaga ggaaaattac ttgggagtag aatattgcaa tgggaatctg 480
   cttgccgtta taaactatgt gcaaattcag ggaggtaaac aagacaaaga tgctccatag 540
   aaaatatgag aagaatctca taactgtttt gagataatta ttgttagcta caaagatcaa 600
   ta                                                                602
<210> 214
   <211> 603
   <212> DNA
   <213> Homo sapiens
<400> 214
   agcggaaaac tcaaggaggt atgaaaataa gatgagtctt aattagaaat gtaaagaatg 60
   aatctgggga caggtagaaa gtaagatcac agtccgtttc caaggggtag tccactgagt 120
   tcgagcttcc taaaaatggt cttttatctt tatgtacaga aaagacatca caaaattcat 180
   tacaaaatgt cacttactgc tccatgctgg agaaagccat atccttctgg gacttgagtc 240
   tgcacattta actacaggta ctgatctgtt ttgtgcttag atgttcccca tcactgccca 300
   gtatggagat gtattggtga gaaacttgag gcgggaagca gagaaaggca agcctgtcac 360
   cttgaaagag taagtaggag cacagccatg gggttctgag ctgtcatgag cccttccagc 420
   tgcctgccat ggagtcgaca gtcgcactgt tgggttactc cagtgaccag acaaaagcag 480
   ggcagcgctg caactccaaa gagccaccta agagggagtg gctcccatga ggcggcaagt 540
   cagcaaggga aaagggcctt ctctcctgtg cacaggagcc aggatttact tatctgttaa 600
   ctt                                                               603
<210> 215
   <211> 60
   <212> PRT
   <213> Homo sapiens
<400> 215
   Ser Leu Ala Glu Asp Glu Glu Trp Lys Arg Ile Arg Ser Leu Leu Ser
    1               5                  10                  15
   Pro Thr Phe Thr Ser Gly Lys Leu Lys Glu Met Phe Pro Ile Thr Ala
               20                  25                  30
   Gln Tyr Gly Asp Val Leu Val Arg Asn Leu Arg Arg Glu Ala Glu Lys
           35                  40                  45
   Gly Lys Pro Val Thr Leu Lys Asp Ile Phe Gly Ala
       50                  55                  60
<210> 216
   <211> 603
   <212> DNA
   <213> Homo sapiens
<400> 216
   agcggaaaac tcaaggaggt atgaaaataa gatgagtctt aattagaaat gtaaagaatg 60
   aatctgggga caggtagaaa gtaagatcac agtccgtttc caaggggtag tccactgagt 120
   tcgagcttcc taaaaatggt cttttatctt tatgtacaga aaagacatca caaaattcat 180
   tacaaaatgt cacttactgc tccatgctgg agaaagccat atccttctgg gacttgagtc 240
   tgcacattta actacaggta ctgatctgtt ttgtgcttag atgttcccca tcattgccca 300
   gtatggagat gtattggtga gaaacttgag gcgggaagca gagaaaggca agcctgtcac 360
   cttgaaagag taagtaggag cacagccatg gggttctgag ctgtcatgag cccttccagc 420
   tgcctgccat ggagtcgaca gtcacactgt tgggttactc cagtgaccag acaaaagcag 480
   ggcagcgctg caactccaaa gagccaccta agagggagtg gctcccatga ggcggcaagt 540
   cagcaaggga aaagggcctt ctctcctgtg cacaggagcc aggatttact tatctgttaa 600
   ctt                                                               603
<210> 217
   <211> 778
   <212> DNA
   <213> Homo sapiens
<400> 217
   cagagcttac atatcttata tcatccacac tcaacacatg ctactgtagt tgtctgataa 60
   tgggtctctg tcttcctatg actgggctcc ttgacctcag aggtgagtct aactcagctt 120
   ggtgtctcca tcacccccag catagggcca gctccatcac tggcaccaga taaccacctt 180
   ctgagggagt agatggaaga tgattcagca gatagttctg aaagtctgtg gctctttatg 240
   tgtcttgact ggatatgtgg gtttcttgct gcatgtatag tggaaggacg gtaagaggtg 300
   ctgattttaa ttttccatat ctttctccac tcagcatctt tggggcctac agcatggatg 360
   tgattactgg cacatcattt ggagtgaaca tcgactctct caacaatcca caagacccct 420
   ttgtggagag cactaaaaag ttcctaaaat ttggtttctt agatccatta tttctctcaa 480
   taagtatgtg ggctattatt tctttctctc tttttaaaaa taactgcttt cttgacatat 540
   aattcacata tcgtataatt catccactta aaaggtacaa ttccattgtt tttaagataa 600
   tcaaaaatat gtatgaccat tactattgta aactaaaatg tttttgtcaa tctagagccc 660
   tcacacactt tagctgtcaa caccccacca caaaccccac tgccctaagc atccaataat 720
   caactttctg cctctataga tttgcctatt ctggacactt catagaaata atatcatt   778
<210> 218
   <211> 778
   <212> DNA
   <213> Homo sapiens
<400> 218
   cagagcttac atatcttata tcatccacac tcaacacatg ctactgtagt tgtctgataa 60
   tgggtctctg tcttcctatg actgggctcc ttgacctcag aggtgagtct aactcagctt 120
   ggtgtctcca tcacccccag catagggcca gctccatcac tggcaccaga taaccacctt 180
   ctgagggagt agatggaaga tgattcagca gatagttctg aaagtctgtg gctctttatg 240
   tgtcttgact ggatatgtgg gtttcttgct gcatgtatag tggaaggacg gtaagaggtg 300
   ctgattttaa ttttccatat ctttctccac tcagcatctt tggggcctac agcatggatg 360
   tgattactgg cacatcattt ggagtgaaca tcgactctct caacaatcca caagacccct 420
   ttgtggagag cactaagaag ttcctaaaat ttggtttctt agatccgtta tttctctcaa 480
   taagtatgtg ggctattatt tctttctctc tttttaaaaa taactgcttt cttgacatat 540
   aattcacata tcgtataatt catccactta aaaggtacaa ttccattgtt tttaagataa 600
   tcaaaaatat gtatgaccat tactattgta aactaaaatg tttttgtcaa tctagagccc 660
   tcacacactt tagctgtcaa caccccacca caaaccccac tgccctaagc atccaataat 720
   caactttctg cctctataga tttgcctatt ctggacactt catagaaata atatcatt   778
<210> 219
   <211> 778
   <212> DNA
   <213> Homo sapiens
<400> 219
   cagagcttac atatcttata tcatccacac tcaacacatg ctactgtagt tgtctgataa 60
   tgggtctctg tcttcctatg actgggctcc ttgacctcag aggtgagtct aactcagctt 120
   ggtgtctcca tcacccccag catagggcca gctccatcac tggcaccaga taaccacctt 180
   ctgagggagt agatggaaga tgattcagca gatagttctg aaagtctgtg gctctttatg 240
   tgtcttgact ggatatgtgg gtttcttgct gcatgtatag tggaaggacg gtaagaggtg 300
   ctgattttaa ttttccatat ctttctccac tcagcatctt tggggcctac agcatggatg 360
   tgattactgg cacatcattt ggagtgaaca tcgactctct caacaatcca caagacccct 420
   ttgtggagag cactaagaag ttcctaaaat ttggtttctt agatccatta tttctctcaa 480
   taagtatgtg ggctattatt tctttctctc tttttaaaaa taactgcttt cttgacatat 540
   aattcacata tcgtataatt catccactta aaaggtacaa tttcattgtt tttaagataa 600
   tcaaaaatat gtatgaccat tactattgta aactaaaatg tttttgtcaa tctagagccc 660
   tcacacactt tagctgtcaa caccccacca caaaccccac tgccctaagc atccaataat 720
   caactttctg cctctataga tttgcctatt ctggacactt catagaaata atatcatt   778
<210> 220
   <211> 778
   <212> DNA
   <213> Homo sapiens
<400> 220
   cagagcttac atatcttata tcatccacac tcaacacatg ctactgtagt tgtctgataa 60
   tgggtctctg tcttcctatg actgggctcc ttgacctcag aggtgagtct aactcagctt 120
   ggtgtctcca tcacccccag catagggcca gctccatcac tggcaccaga taaccacctt 180
   ctgagggagt agatggaaga tgattcagca gatagttctg aaagtctgtg gctctttatg 240
   tgtcttgact ggatatgtgg gtttcttgct gcatgtatag tggaaggacg gtaagaggtg 300
   ctgattttaa ttttccatat ctttctccac tcagcatctt tggggcctac agcatggatg 360
   tgattactgg cacatcattt ggagtgaaca tcgactctct caacaatcca caagacccct 420
   ttgtggagag cactaagaag ttcctaaaat ttggtttctt agatccatta tttctctcaa 480
   taagtatgtg ggctattatt tctttctctc tttttaaaaa taactgcttt cttgacatat 540
   aattcacata tcgtataatt catccactta aaaggtacaa ttccattgtt tttaagataa 600
   tcaaaaatat gtatgaccat tactattgta aactaaaatg tttttgtcag tctagagccc 660
   tcacacactt tagctgtcaa caccccacca caaaccccac tgccctaagc atccaataat 720
   caactttctg cctctataga tttgcctatt ctggacactt catagaaata atatcatt   778
<210> 221
   <211> 670
   <212> DNA
   <213> Homo sapiens
<400> 221
   agaaggtgcc attgatctca ctgctgtagt ggtgtttcct atgtatagac ctgcccttgc 60
   tcagtcgccg gcctgaaaga agggcaaaca tgataaaagg aatgggttcc agttgagaat 120
   catgatgttc ttattcttat tactggtaga gaaaattata attgctccag gtaaagtttg 180
   cattttcaat gatttccttt tgttcgtttt gtttttccca cagtactctt tccattcctt 240
   accccagttt ttgaagcatt aaatgtctct ctgtttccaa aagataccat aaatttttta 300
   agtaaatctg taaacagaat gaagaaaagt cgcctcaacg acaaacaaaa ggtaaaatct 360
   gatggtggtt aaatgacgat gtttaggttt tgataaattt agattttata cacatgatag 420
   agcatgtatc tgtattttta aaaataaaga cagagaactt atgtttagaa caagagaagc 480
   catttggtag aaataaagaa ggagattggg gaaggagatg agaatgagtc agagagatag 540
   catttaaaac ttgaaatcag gcacaacaat tagtatgtca tgatataaac agtattgaga 600
   taaaatttta ccacttctct tccctttaat aaattgtcaa aggataaagt ttcctgtttg 660
   aaaatatatt                                                        670
<210> 222
   <211> 670
   <212> DNA
   <213> Homo sapiens
<400> 222
   agaaggtgcc attgatctca ctgctgtagt ggtgtttcct atgtatagac ctgcccttgc 60
   tcagtcgccg gcctgaaaga agggcaaaca tgataaaagg aatgggttcc agttgagaat 120
   catgatgttc ttattcttat tactggtaga gaaaattata attgctccag gtaaagtttg 180
   cattttcaat gatttccttt tgtttgtttt gtttttccca cagtactctt tccattcctt 240
   accccagttt ttgaagcatt aaatgtctct ctgtttccaa aagataccat aaatttttta 300
   agtaaatctg taaacagaat gaagaaaagt cgcctcaacg acaaacaaaa ggtaaaatct 360
   gatggtggtt aaatgacgat gtttaggttt tgataaattt agattttata cacatgatag 420
   agcatgtatc tgtattttta aaaataaaga cagagaactt atgtttagaa caagagaagc 480
   catttggtag aaatacagaa ggagattggg gaaggagatg agaatgagtc agagagatag 540
   catttaaaac ttgaaatcag gcacaacaat tagtatgtca tgatataaac agtattgaga 600
   taaaatttta ccacttctct tccctttaat aaattgtcaa aggataaagt ttcctgtttg 660
   aaaatatatt                                                        670
<210> 223
   <211> 826
   <212> DNA
   <213> Homo sapiens
<400> 223
   agataaagta cttttaggat cattcaaggc acacacccat aacactgagt atgtaagaca 60
   gaaatgctct ctctggaaat tacagcagtg ctggtgctgg gatgccatga tgaggagtgt 120
   gtggcccaca atcatgtaga ccttgggaaa acctggatta aaatgatttt gcatcatcct 180
   ggccctgtat aagatacata tcagaatgaa aaccactccc agtgtgactt tgaattgctt 240
   ttccattttt tcttcttggg attagagagc ttcacttaga tttcatctaa gctgtgatgt 300
   tgtacgttga cctgatttac ctaaaatgtc tttcctctcc tttcagctct gtctgatctg 360
   gagctcgcag cccagtcaat aatcttcatt tttgctggct atgaaaccac cagcagtgtt 420
   ctttccttca ctttatatga actggccact caccctgatg tccagcagaa actgcaaaag 480
   gagattgatg cagttttgcc caataaggtg aggggatgac ccctggagat gaagggaaga 540
   ggtgaagcct tagcaaaaat gcctcctcac cactccccag gagaattttt ataaaaagca 600
   taatcactga ttccttcact gacataatgt aggaagcctc tgaggagaaa aacaaaggga 660
   gaaacataga gaacggttgc tactggcaga agcataagat ctttgtacaa tattgctggc 720
   cctggttcac ctgtttactg ttatcacaat aatgctaagt aaaaaaaaaa aaaaaaaaaa 780
   aaaaaaaaaa aggagtgtgg cgagaagatg gccaaacagg aacagc 826
<210> 224
   <211> 826
   <212> DNA
   <213> Homo sapiens
<400> 224
   agataaagta cttttaggat cattcaaggc acacacccat aacactgagt atgtaagaca 60
   gaaatgctct ctctggaaat tacagcagtg ctggtgctgg gatgccatga tgaggagtgt 120
   gtggcccaca atcatgtaga ccttgggaaa acctggatta aaatgatttt gcgtcatcct 180
   ggccctgtat aagatacata tcagaatgaa aaccactccc agtgtgactt tgaattgctt 240
   ttccattttt tcttcttggg attagagagc ttcacttaga tttcatctaa gctgtgatgt 300
   tgtacgttga cttgatttac ctaaaatgtc tttcctctcc tttcagctct gtctgatctg 360
   gagctcgcag cccagtcaat aatcttcatt tttgctggct atgaaaccac cagcagtgtt 420
   ctttccttca ctttatatga actggccact caccctgatg tccagcagaa actgcaaaag 480
   gagattgatg cagttttgcc caataaggtg aggggatgac ccctggagat gaagggaaga 540
   ggtgaagcct tagcaaaaat gcctcctcac cactccccag gagaattttt ataaaaagca 600
   taatcactga ttccttcact gacataatgt aggaagcctc tgaggagaaa aacaaaggga 660
   gaaacataga gaacggttgc tactggcaga agcataagat ctttgtacaa tattgctggc 720
   cctggttcac ctgtttactg ttatcacaat aatgctaagt aaaaaaaaaa aaaaaaaaaa 780
   aaaaaaaaaa aggagtgtgg cgagaagatg gccaaacagg aacagc 826
<210> 225
   <211> 616
   <212> DNA
   <213> Homo sapiens
<400> 225
   caggcctggc acagagtcag tgctccataa atattttgtt aaacgatgga tggtgagtgc 60
   ttttactatc cagtatttac ccagcttata gattaagtat gaagagttca agatacatgg 120
   tgttaagagt cgtttttata tgcttgcaaa gcatttttgt catatttttt ctactttgct 180
   tccatctttt cttctttcac ttcatttatt aattctccat atgcttgttt aactattgca 240
   gatccccttg aaattagaca cgcaaggact tcttcaacca gaaaaaccca ttgttctaaa 300
   ggtggattca agagatggaa ccctaagtgg agaatgagtt attctaagga tttctacttt 360
   ggtcttcaag aaagctgtgc cccagaacac cagagatttc aacttagtca ataaaacctt 420
   gaaataaaga tgggcttaat ctaatgtact gcatgagtag ttggtgattt tgtacattca 480
   ttgagctctc ccagagtctg tgtagagtgt tgtgcattat gtagtataaa ggaggtgacc 540
   aggtaagtga cagataggta gactcagctt ctctgcttct cataggacta cctctaccca 600
   cctctagtta gcatta 616
<210> 226
   <211> 624
   <212> DNA
   <213> Homo sapiens
<400> 226
   gattaagctt ttcatgattc ctcatagaac atgaactcaa aagaggtcag caaaggggtg 60
   tgtgcgattc tttgctattg gctgcagcta tagccctgcc tccttctcca gcacataaat 120
   ctttcagcag cttggctgaa gactgctgtg cagggcaggg aagctccagg caaacagccc 180
   agcaaacagc agcactcagc taaaaggaag actcacagaa cacagttgaa gaaggaaagt 240
   ggcgatggac ctcatcccaa atttggcggt ggaaacctgg cttctcctgg ctgtcagcct 300
   ggtgctcctc tatctgtgag taactgtcca aactcctctc tttgtttcct tggacttggg 360
   gtgctaatcg ggcccctttc cccttatctg ttttgaagat caaaagagat gttcaaggag 420
   aagtagctga agtgttggac gctacaaacg catagaagtt attattatct tatgcagatc 480
   tatgaatgaa taaataagca tttctcccat ccaccttcta attttggtga ctaggagggt 540
   ttagggacag catttggtag tgggaatgat ttgattagct tagatctgac gaagactaat 600
   caatgaaaac atggcagcgg caga 624
<210> 227
   <211> 624
   <212> DNA
   <213> Homo sapiens
<400> 227
   gattaagctt ttcatgattc ctcatagaac atgaactcaa aagaggtcag caaaggggtg 60
   tgtgcgattc tttgctattg gctgcagcta tagccctgcc tccttctcca gcacataaat 120
   ctttcagcag cttggctgaa gactgctgtg cagggcaggg aagctccagg caaacagccc 180
   agcaaacagc agcactcagc taaaaggaag actcacagaa cacagttgaa gaaggaaagt 240
   ggcgatggac ctcatcccaa atttggcggt ggaaacctgg cttctcctgg ctgtcagcct 300
   ggtgctcctc tatctgtgag taactgtcca aactcctctc tttgtttcct tggacttggg 360
   gtgctaatcg ggcccctttt cccttatctg ttttgaagat caaaagagat gttcaaggag 420
   aagtagctga agtgttggac gctacaaacg catagaagtt attattatct tatacagatc 480
   tatgaatgaa taaataagca tttctcccat ccaccttcta attttggtga ctaggagggt 540
   ttagggacag catttggtag tgggaatgat ttgattagct tagatctgac gaagactaat 600
   caatgaaaac atggcagcgg caga 624
<210> 228
   <211> 626
   <212> DNA
   <213> Homo sapiens
<400> 228
   gaacagttcc ctaccacgtg gagcatttgc aattaaaagg agactgagat atagaggcag 60
   gagaccacac cagatggctg ggtctcccca ctcccacccc cgccccacat acactcagaa 120
   gaggctaggc atctaggatc tccattgagc atcttgaata tggcttgcca taatatcata 180
   tacagtcaat aaatatttgt taaataagga tgcctcttca atatattttg tgcaaccatg 240
   aagatcacca caactaatgt gagaaaaaat gtttctgttg aactctagtc tttaggccca 300
   gtgggattta tgaaaagtgc catctcttta gctgaggatg aagaatggaa gagaatacag 360
   tcattgctgt ctccaacctt caccagcgga aaactcaagg aggtatgaaa ataagatgag 420
   tcttaattag aaatgtaaag aatgaatctg gggacaggta gaaagtaaga tcacagtccg 480
   tttccaaggg gtagtccact gagttcgagc ttcctaaaaa tggtctttta tctttatgta 540
   cagaaaagac atcacaaaat tcattacaaa atgtcactta ctgctccatg ctggagaaag 600
   ccatatcctt ctgggacttg agtctg                                      626
<210> 229
   <211> 60
   <212> PRT
   <213> Homo sapiens
<400> 229
   Ser Val Phe Thr Asn Arg Arg Ser Leu Gly Pro Val Gly Phe Met Lys
   1               5                   10                  15
   Ser Ala Ile Ser Leu Ala Glu Asp Glu Glu Trp Lys Arg Ile Gln Ser
               20                  25                  30
   Leu Leu Ser Pro Thr Phe Thr Ser Gly Lys Leu Lys Glu Met Phe Pro
           35                  40                  45
   Ile Ile Ala Gln Tyr Gly Asp Val Leu Val Arg Asn
       50                  55                  60
<210> 230
   <211> 626
   <212> DNA
   <213> Homo sapiens
<400> 230
   gaacagttcc ctaccacgtg gagcatttgc aattaaaagg agactgagat atagaggcag 60
   gagaccacac cagatggctg ggtctcccca ctcccacccc cgccccacat acactcagaa 120
   gaggctaggc atctaggatc tccattgagc atcttgaata tggcttgcca taatatcata 180
   tacagtcaat aaatatttgt taaataagga tgcctcttca atatattttg tgcaaccatg 240
   aagatcacca caactaatgt gagaaaaaat gtttctgttg aactctagtc tttaggccca 300
   gtgggattta tgaaaagtgc catctcttta gctgaggatg aagaatggaa gagaatacgg 360
   tcattgctgt ctccaacctt caccagcgga aaactcaagg aggcatgaaa ataagatgag 420
   tcttaattag aaatgtaaag aatgaatctg gggacaggta gaaagtaaga tcacagtccg 480
   tttccaaggg gtagtccact gagttcgagc ttcctaaaaa tggtctttta tctttatgta 540
   cagaaaagac atcacaaaat tcattacaaa atgtcactta ctgctccatg ctggagaaag 600
   ccatatcctt ctgggacttg agtctg                                      626
<210> 231
   <211> 626
   <212> DNA
   <213> Homo sapiens
<400> 231
   gaacagttcc ctaccacgtg gagcatttgc aattaaaagg agactgagat atagaggcag 60
   gagaccacac cagatggctg ggtctcccca ctcccacccc cgccccacat acactcagaa 120
   gaggctaggc atctaggatc tccattgagc atcttgaata tggcttgcca taatatcata 180
   tacagtcaat aaatatttgt taaataagga tgcctcttca atatattttg tgcaaccatg 240
   aagatcacca caactaatgt gagaaaaaat gtttctgttg aactctagtc tttaggccca 300
   gtgggattta tgaaaagtgc catctcttta gctgaggatg aagaatggaa gagaatacgg 360
   tcattgctgt ctccaacctt caccagcgga aaactcaagg aggtatgaaa ataagatgag 420
   tcttaattag aaatgtaaag aatgaatctg gggacaggta gaaagtaaga tcacagtcca 480
   tttccaaggg gtagtccact gagttcgagc ttcctaaaaa tggtctttta tctttatgta 540
   cagaaaagac atcacaaaat tcattacaaa atgtcactta ctgctccatg ctggagaaag 600
   ccatatcctt ctgggacttg agtctg                                      626
<210> 232
   <211> 623
   <212> DNA
   <213> Homo sapiens
<400> 232
   attggacatg atagctagat ttgtttcagg aaaacatcct gctttccaag gatttagatg 60
   aatgtttttg ttcactggtg actcaggtaa cacgtcttca agaagccata gggaggttga 120
   gggagggaag tcaagaaggg aggttgagga ctgcactttt gatttacttc tgacttcacg 180
   agtcactttc tgcgaaagaa atctctcctt ttgcttctag caccgactag atttccttca 240
   gctgatgatt gactcccaga attcgaaaga aactgagtcc cacaaaggta accaaggagt 300
   gcttctgagg gctactggcg gggacactaa gagggagggc cttgttctga aaatgtgcag 360
   gaagtattcc aggaagatga gaatttttgc cacatagcag aacaacacac atttagatgt 420
   tataaatggt agctggaggc actttccaga agcccacagg tatagccatg ttccaggctg 480
   aaagggcaac cctaagcaaa cctagaatgc ttggaggaca gtcagtggtt tgtggatcac 540
   ctacatgaga tcaaatgcca gttctcagcc tcctccagat ccaccaagtg agaacctcta 600
   cttggaaatt tatatcaaac ata                                         623
<210> 233
   <211> 616
   <212> DNA
   <213> Homo sapiens
<400> 233
   caggcctggc acagagtcag tgctccataa atattttgtt aaacgatgga tggtgagtgc 60
   ttttactatc cagtatttac ccagcttata gattaagtat gaagagttca agatacatgg 120
   tgttaagagt cgtttttata tgcttgcaaa gcatttttgt catatttttt ctactttgct 180
   tccatctttt cttctttcac ttcatttatt aattctccat atgcttgttt aactattgta 240
   gatccccttg aaattagaca cgcaaggact tcttcaacca gaaaaaccca ctgttctaaa 300
   ggtggattca agagatggaa ccctaagtgg agaatgagtt attctaagga tttctacttt 360
   ggtcttcaag aaagctgtgc cccagaacac cagagatttc aacttagtca ataaaacctt 420
   gaaataaaga tgggcttaat ctaatgtact gcatgagtag ttggtgattt tgtacattca 480
   ttgagctctc ccagagtctg tgtagagtgt tgtgcattat gtagtataaa ggaggtgacc 540
   aggtaagtga cagataggta gactcagctt ctctgcttct cataggacta cctctaccca 600
   cctctagtta gcatta                                                 616
<210> 234
   <211> 59
   <212> PRT
   <213> Homo sapiens
<400> 234
   Met Arg Phe Ala Leu Met Asn Met Lys Leu Ala Leu Ile Arg Val Leu
   1               5                   10                  15
   Gln Asn Phe Ser Phe Lys Pro Cys Lys Glu Thr Gln Ile Pro Leu Lys
               20                  25                  30
   Leu Asp Thr Gln Gly Leu Leu Gln Pro Glu Lys Pro Thr Val Leu Lys
           35                  40                  45
   Val Asp Ser Arg Asp Gly Thr Leu Ser Gly Glu
       50                  55
<210> 235
   <211> 830
   <212> DNA
   <213> Homo sapiens
<400> 235
   tggtcaccca ccatgtgtac agtaccctgc tagggtccag ggtcatgaaa gtaaataata 60
   ccagactgtg cccttgagga actcacctct gctaagggaa acaggcacag aaacccacaa 120
   gggtggtaga gaggaaatag gacaatagga ctgtgtgagg gggataggag gcacccagag 180
   gaggaaatgg ttacatctgt gtgaggaggt tggtaaggaa agactttaat agaaggggtc 240
   tgtctggctg ggcttgcaag gatgtgtagg agtcatctag ggggcacaag tacactccag 300
   gcagagggaa ttgcatgggt aaagatctgc agttgtggct tgtggggatg gatttcaagt 360
   attctggaat gaagacagcc atggaaacaa gggcaggtga gaggatattt aagaggcttc 420
   atgccaatgg ctccacttca gtttctgata agaactcagg ttccgtggac tccctgataa 480
   aactgattaa gttgtttatg attccccata gaatatgaac tcaaaggagg taagcaaagg 540
   ggtgtgtgcg attctttgct accggctgca gctgcagccc cacctccttc tccagcacat 600
   aaacatttca gcagcttgac ctaagactgc tgtgcagggc agggatgctc caggcagaca 660
   gcccagcaaa caacagcaca cagctgaaag taagactcag aggagacagt tgaagaaggc 720
   aagtggcgat ggacctcatc ccaaatttgg cggtggaaac ctggcttctc ctggctgtca 780
   gcctggtgct cctctatctg tcagtaactg tccagattcc tctcctctgt            830
<210> 236
   <211> 775
   <212> DNA
   <213> Homo sapiens
<400> 236
   ggagtgacct gattttccag gtgctgtctg tcaccccttt ctttgactag gaaagggaac 60
   tccttgaccc cttgcgcttc tcaagtgagg caatgcctcg ccctgcttcg gcttgtgcac 120
   agcacgctgc acccactgtc ctgcacccac tgtctggcac tccctagtga gatgaacccg 180
   gtacctcaga tgaaaatgca gaaattaccc gtcttctgtg tcactcacac tgggagctgt 240
   agaccggagc tgttcctatt cggccatctt ggctccaccc cccgagtttt ggcttttaat 300
   tgaaagtgta ttgatgtggg aaggagataa tgccatgcat ttatgagcac atattagagg 360
   gtctgagaca atgcatgtga taaaaggtca cctaaggaag aaaaaaagaa caagggaaga 420
   cactggaaag aacgtgatgc tgggagtccc tgggccacca aagtctggag aaaagtggta 480
   accacaaggc tcccagccta gtttcactga agacctcgac actaggtgac ttatgggatc 540
   cttggtagga caagccttga aaagttttac aatagcaaat gtggacgttg tcagaaccaa 600
   atgatgtcac gtgtgtattt gtgtgtgtgt gtcagtgtgt gtgtttaaaa atcatgacaa 660
   ataaagcagg ctgtgaagag gggattccca tgctcgtgtg cctgataaca caactatcac 720
   aaacgctttg cgaaaccaca agtttgcaca aaggcaatcc caaccttaca caaaa      775
<210> 237
   <211> 25
   <212> DNA
   <213> Homo sapiens
<400> 237
   ttgttgggaa atgttttgtc ctatc 25
<210> 238
   <211> 24
   <212> DNA
   <213> Homo sapiens
<400> 238
   acagggagtt gaccttcata cgtt 24
<210> 239
   <211> 33
   <212> DNA
   <213> Homo sapiens
<400> 239
   tcagggtctc tggaaatttg acacagagtg cta 33
1
   49/49
   \\Ntvossius1\Allgemein\Daten-1\sg\sequencelisting\E3103PCT\E3103PCT.APP
   1
   47/47
   1
   4/47

## Claims

1. A polynucleotide selected from the group consisting of:
(a) a polynucleotide hybridising under stringent conditions to a CYP3A5 gene and having the nucleic acid sequence of SEQ ID NO: 112;
(b) a polynucleotide hybridising under stringent conditions to a CYP3A5 gene and encoding a polypeptide having the amino acid sequence of SEQ ID NO: 141; and
(c) a polynucleotide hybridizing under stringent conditions to a part of a CYP3A5 gene comprising SEQ ID NO:111, wherein said polynucleotide is having an additional nucleotide at a position corresponding to position 27131/27132 of the CYP3A5 gene as defined by the joined sequences of Accession No: AF280107.1, wherein position 166220 has been numbered +1 and position 174832 has been numbered +8613, and Accession No: AC005020.2, wherein position 27341 has been numbered +8614.

2. A polynucleotide of claim 1 which is DNA or RNA.

3. A vector comprising a polynucleotide of claim 1 or 2.

4. The vector of claim 3, wherein the polynucleotide is operatively linked to expression control sequences allowing expression in prokaryotic or eukaryotic cells or isolated fractions thereof.

5. A host cell genetically engineered with the polynucleotide of claim 1 or 2, or the vector of claim 3 or 4.

6. A method for producing a molecular variant CYP3A5 polypeptide comprising
(a) culturing the host cell of claim 5; and
(b) recovering said protein from the culture.

7. A method for producing cells capable of expressing a molecular variant CYP3A5 polypeptide comprising genetically engineering cells with the polynucleotide of claim 1 or 2, or the vector of claim 3 or 4.

8. A polypeptide encoded by the polynucleotide of claim 1 or 2 or obtainable by the method of claim 6 from cells produced by the method of claim 7.

9. An antibody which binds specifically to the polypeptide of claim 8.

10. The antibody of claim 9 which is monoclonal or polyclonal.

11. A transgenic non-human animal comprising at least one polynucleotide of claim 1 or 2, or the vector of claim 3 or 4.

12. The transgenic non-human animal of claim 11 which is a mouse, a rat or a zebrafish.

13. A solid support comprising one or a plurality of the polynucleotide of claim 1 or 2, the vector of claim 3 or 4, the polypeptide of claim 8, the antibody of claim 9 or the host cell of claim 5 in immobilized form.

14. The solid support of claim 13, wherein said solid support is a membrane, a glass-, polypropylene- or silicon-chip, are oligonucleotide conjugated beads or bead array, which is assembled on an optical filter substrate.

15. An in vitro method for identifying a polymorphism said method comprising the step of identifying a polymorphism by comparing the nucleic acid sequence of a polynucleotide of claim 1 or 2 obtained from a subgroup of individuals, wherein the subgroup has no prevalence for cancer with the nucleic acid sequence of a polynucleotide of claim 1 or 2 obtained from a plurality of subgroups of individuals, wherein at least one or more further subgroup(s) do have prevalence for cancer.

16. A method for identifying and obtaining a pro-drug or a drug capable of modulating the activity of a molecular variant of a CYP3A5 polypeptide comprising the steps of:
(a) contacting the polypeptide of claim 8, the solid support of claim 13 or 14, a cell expressing a molecular variant gene comprising a polynucleotide of claim 1 or 2 or the vector of claim 3 or 4 in the presence of components capable of providing a detectable signal in response to drug activity with a compound to be screened for pro-drug or drug activity; and
(b) detecting the presence or absence of a signal or increase or decrease of a signal generated from the pro-drug or the drug activity, wherein the absence, presence, increase or decrease of the signal is indicative for a putative pro-drug or drug.

17. The method of claim 16, wherein said cell is a cell of claim 6, obtained by the method of claim 7 or can be obtained by the transgenic non-human animal of claim 11 or 12.

18. A method of identifying and obtaining a pro-drug or drug capable of modulating the activity of a molecular variant of a CYP3A5 polypeptide comprising the steps of:
(a) contacting the host cell of claim 5, the cell obtained by the method of claim 7, the polypeptide of claim 8 or the solid support of claim 13 or 14 with the first molecule known to be bound by a CYP3A5 polypeptide to form a first complex of said polypeptide and said first molecule;
(b) contacting said first complex with a compound to be screened; and
(c) measuring whether said compound displaces said first molecule from said first complex.

19. The method of claim 18, wherein said measuring step comprises measuring the formation of a second complex of said protein and said compound.

20. The method of claim 18 or 19, wherein said measuring step comprises measuring the amount of said first molecule that is not bound to said protein.

21. The method of any one of claims 18 to 20, wherein said first molecule is labeled.

22. An in vitro method of diagnosing liver cancer or a susceptibility to liver cancer related to the presence of a molecular variant of a CYP3A5 gene comprising determining the presence of
(a) a polynucleotide selected from the group consisting of:
(i) a polynucleotide of claim 1 or 2; and
(ii) a polynucleotide having an additional nucleotide at a position corresponding to position 27131/27132 of the CYP3A5 gene as defined by the joined sequences of Accession No: AF280107.1, wherein position 166220 has been numbered +1 and position 174832 has been numbered +8613, and Accession No: AC005020.2, wherein position 27341 has been numbered +8614; or
(b) the polypeptide of claim 8
in a sample from a subject.

23. The method of claim 22 comprising PCR, ligase chain reaction, restriction digestion, direct sequencing, nucleic acid amplification techniques, hybridization techniques, mass spectroscopy or immunoassays.

24. A method of detection of the polynucleotide of claim 1 or 2 in a sample comprising the steps of
(a) contacting the solid support of claim 13 or 14 with the sample under conditions allowing interaction of the polynucleotide of claim 1 or 2 with the immobilized targets on a solid support; and
(b) determining the binding of said polynucleotide or said gene to said immobilized targets on a solid support.

25. A diagnostic composition comprising the polynucleotide of claim 1 or 2, the vector of claim 3 or 4, the polypeptide of claim 8 or the antibody of claim 9.

26. A pharmaceutical composition comprising the polynucleotide of claim 1 or 2, the vector of claim 3 or 4, the polypeptide of claim 8 or the antibody of claim 9.

27. Use of the polynucleotide of claim 1 or 2, the polypeptide of claim 8 or the antibody of claim 9 for the preparation of a diagnostic composition for diagnosing liver cancer in a subject.

28. A diagnostic kit for detection of a single nucleotide polymorphism comprising the polynucleotide of claim 1 or 2, the vector of claim 3 or 4, the polypeptide of claim 8, the antibody of claim 9, the host cell of claim 5, the transgenic non-human animal of claim 11 or 12 or the solid support of claim 13 or 14.

## Patentansprüche

1. Polynucleotid, ausgewählt aus der Gruppe bestehend aus:
(a) einem Polynucleotid, das unter stringenten Bedingungen mit einem CYP3A5-Gen hybridisiert und die Nucleinsäuresequenz von SEQ ID NO:112 hat;
(b) einem Polynucleotid, das unter stringenten Bedingungen mit einem CYP3A5-Gen hybridisiert und ein Polypeptid codiert, das die Aminosäuresequenz von SEQ ID NO:141 hat; und
(c) einem Polynucleotid, das unter stringenten Bedingungen mit einem Teil eines CYP3A5-Gens hybridisiert, das die SEQ ID NO:111 umfasst, wobei das Polynucleotid ein zusätzliches Nucleotid an einer Position hat, die der Position 27131/27132 des CYP3A5-Gens entspricht, das definiert ist durch die verknüpften Sequenzen von Zugangsnr. AF280107.1, wobei Position 166220 als +1 nummeriert worden ist und Position 174832 als + 8613 nummeriert worden ist, und Zugangsnr. AC005020.2, wobei Position 27341 als +8614 nummeriert worden ist.

2. Polynucleotid nach Anspruch 1, das DNA oder RNA ist.

3. Vektor, der ein Polynucleotid nach Anspruch 1 oder 2 umfasst.

4. Vektor nach Anspruch 3, wobei das Polynucleotid funktionell mit einer Expressionskontrollsequenz verbunden ist, die Expression in prokaryontischen oder eukaryontischen Zellen oder isolierten Fraktionen davon erlaubt.

5. Wirtszelle, die mit dem Polynucleotid nach Anspruch 1 oder 2 oder dem Vektor nach Anspruch 3 oder 4 genetisch verändert ist.

6. Verfahren zur Herstellung einer molekularen Variante des CYP3A5-Polypeptids, umfassend
(a) das Züchten der Wirtszelle nach Anspruch 5; und
(b) das Ernten des Proteins aus der Kultur.

7. Verfahren zur Herstellung von Zellen, die eine molekulare Variante des CYP3A5-Polypeptids exprimieren können, umfassend das genetische Verändern der Zellen mit dem Polynucleotid nach Anspruch 1 oder 2 oder dem Vektor nach Anspruch 3 oder 4.

8. Polypeptid, das von dem Polynucleotid nach Anspruch 1 oder 2 codiert wird, oder erhältlich ist durch das Verfahren nach Anspruch 6 von den Zellen, die gemäß dem Verfahren nach Anspruch 7 hergestellt wurden.

9. Antikörper, der spezifisch an das Polypeptid nach Anspruch 8 bindet.

10. Antikörper nach Anspruch 9, der monoclonal oder polyclonal ist.

11. Transgenes nicht-menschliches Tier, umfassend zumindest ein Polynucleotid nach Anspruch 1 oder 2 oder den Vektor nach Anspruch 3 oder 4.

12. Transgenes nicht-menschliches Tier nach Anspruch 11, das eine Maus, Ratte oder ein Zebrafisch ist.

13. Fester Träger, umfassend ein/einen/eine oder eine Vielzahl der Polynucleotide nach Anspruch 1 oder 2, der Vektoren nach Anspruch 3 oder 4, der Polypeptide nach Anspruch 8, der Antikörper nach Anspruch 9 oder der Wirtszellen nach Anspruch 5, in immobilisierter Form.

14. Fester Träger nach Anspruch 13, wobei der feste Träger eine Membran, ein Glas-, Polypropylen- oder ein Silikonchip ist, Oligonucleotid-konjugierte Kügelchen sind oder ein Kügelchenarray, der auf einem optischen Filtersubstrat angeordnet ist.

15. In vitro-Verfahren zum Auffinden eines Polymorphismus, umfassend das Auffinden eines Polymorphismus durch Vergleichen der Nucleinsäuresequenz eines Polynucleotids nach Anspruch 1 oder 2, erhalten von einer Untergruppe von Individuen, wobei die Untergruppe keine Prävalenz für Krebs hat, mit der Nucleinsäuresequenz eines Polynucleotids nach Anspruch 1 oder 2 erhalten von einer Vielzahl von Untergruppen von Individuen, wobei zumindest eine oder mehrere weitere Untergruppe(n) eine Prävalenz für Krebs hat/haben.

16. Verfahren zum Auffinden und Erhalten einer Arzneistoffvorstufe oder eines Arzneistoffs, die/der die Aktivität einer molekularen Variante eines CYP3A5-Polypeptids modulieren kann, umfassend
(a) das Inkontaktbringen des Polypeptids nach Anspruch 8, des festen Trägers nach Anspruch 13 oder 14, einer Zelle, die eine molekulare Variante eines Gens exprimiert, das ein Polynucleotid nach Anspruch 1 oder 2 umfasst, oder des Vektors nach Anspruch 3 oder 4 in der Anwesenheit von Stoffen, die ein nachweisbares Signal als Antwort auf die Arzneistoffaktivität liefern können, mit einer Verbindung, die auf Arzneistoffvorstufen- oder Arzneistoffaktivität zu durchmustern ist; und
(b) das Nachweisen der Anwesenheit oder Abwesenheit eines Signals oder der Zunahme oder Abnahme eines Signals, das von der Arzneistoffvorstufen- oder der Arzneistoffaktivität erzeugt wurde, wobei die Abwesenheit, Anwesenheit, Zunahme oder Abnahme des Signals auf eine mögliche Arzneistoffvorstufe oder einen Arzneistoff hinweist.

17. Verfahren nach Anspruch 16, wobei die Zelle eine Zelle nach Anspruch 6 ist, erhalten durch das Verfahren nach Anspruch 7, oder von dem transgenen nicht-menschlichen Tier nach Anspruch 11 oder 12 erhalten werden kann.

18. Verfahren zum Auffinden und Erhalten einer Arzneistoffvorstufe oder eines Arzneistoffs, die/der die Aktivität einer molekularen Variante eines CYP3A5-Polypeptids modulieren kann, umfassend die Schritte
(a) des Inkontaktbringens der Wirtszelle nach Anspruch 5, der Zelle erhalten durch das Verfahren nach Anspruch 7, des Polypeptids nach Anspruch 8 oder des festen Trägers nach Anspruch 13 oder 14 mit einem ersten Molekül, von dem bekannt ist, dass es von einem CYP3A5-Polypeptid gebunden wird, um einen ersten Komplex zwischen dem Polypeptid und dem ersten Molekül zu bilden;
(b) des Inkontaktbringens mit einer zu durchmusternden Verbindung; und
(c) des Messens, ob die Verbindung das erste Molekül aus dem ersten Komplex verdrängt.

19. Verfahren nach Anspruch 18, wobei der Messschritt das Messen der Bildung eines zweiten Komplexes zwischen dem Protein und der Verbindung umfasst.

20. Verfahren nach Anspruch 18 oder 19, wobei der Messschritt das Messen der Menge des ersten Moleküls, das nicht an das Protein gebunden ist, umfasst.

21. Verfahren nach einem der Ansprüche 18 bis 20, wobei das erste Molekül markiert ist.

22. In vitro-Verfahren zur Diagnose von Leberkrebs oder einer Anfälligkeit für Leberkrebs im Zusammenhang mit der Anwesenheit einer molekularen Variante eines CYP3A5-Gens, umfassend das Bestimmen der Anwesenheit
(a) eines Polynucleotids, ausgewählt aus der Gruppe bestehend aus
(i) einem Polynucleotid nach Anspruch 1 oder 2; und
(ii) einem Polynucleotid, das ein zusätzliches Nucleotid an einer Position hat, die der Position 27131/27132 des CYP3A5-Gens entspricht, das durch die verknüpften Sequenzen von Zugangsnr. AF280107.1 definiert ist, wobei Position 166220 als +1 nummeriert worden ist und Position 174832 als +8613 nummeriert worden ist, und Zugangsnr. AC005020.2, wobei Position 27341 als +8614 nummeriert worden ist; oder
(b) des Polypeptids nach Anspruch 8
in einer Probe eines Individuums.

23. Verfahren nach Anspruch 22, umfassend PCR, Ligase-Kettenreaktion, Restriktionsverdau, direktes Sequenzieren, Nucleinsäure-Amplifikationstechniken, Hybridisierungstechniken, Massenspektroskopie oder Immun-Assays.

24. Verfahren zum Nachweis des Polynucleotids nach Anspruch 1 oder 2 in einer Probe, umfassend
(a) das Inkontaktbringen des festen Trägers nach Anspruch 13 oder 14 mit der Probe unter Bedingungen, die eine Interaktion des Polynucleotids nach Anspruch 1 oder 2 mit den immobilisierten Zielen auf einem festen Träger erlauben; und
(b) das Bestimmen der Bindung des Polynucleotids oder des Gens an die immobilisierten Ziele auf dem festen Träger.

25. Diagnostische Zusammensetzung, umfassend das Polynucleotid nach Anspruch 1 oder 2, den Vektor nach Anspruch 3 oder 4, das Polypeptid nach Anspruch 8 oder den Antikörper nach Anspruch 9.

26. Arzneimittel, umfassend das Polynucleotid nach Anspruch 1 oder 2, den Vektor nach Anspruch 3 oder 4, das Polypeptid nach Anspruch 8 oder den Antikörper nach Anspruch 9.

27. Verwendung des Polynucleotids nach Anspruch 1 oder 2, des Polypeptids nach Anspruch 8 oder des Antikörpers nach Anspruch 9 für die Herstellung einer diagnostischen Zusammensetzung zum Diagnostizieren von Leberkrebs in einem Individuum.

28. Diagnostischer Kit zum Nachweis eines Einzelnucleotid-Polymorphismus, umfassend das Polynucleotid nach Anspruch 1 oder 2, den Vektor nach Anspruch 3 oder 4, das Polypeptid nach Anspruch 8, den Antikörper nach Anspruch 9, die Wirtszelle nach Anspruch 5, das transgene nicht-menschliche Tier nach Anspruch 11 oder 12 oder den festen Träger nach Anspruch 13 oder 14.

## Revendications

1. Polynucléotide choisi dans le groupe constitué de :
(a) un polynucléotide s'hybridant dans des conditions stringentes avec un gène CYP3A5 et ayant la séquence d'acide nucléique SEQ ID NO : 112 ;
(b) un polynucléotide s'hybridant dans des conditions stringentes avec un gène CYP3A5 et codant un polypeptide ayant la séquence d'acides aminés SEQ ID NO : 141 ; et
(c) un polynucléotide s'hybridant dans des conditions stringentes avec une partie d'un gène CYP3A5 comprenant SEQ ID NO : 111, ledit polynucléotide ayant un nucléotide supplémentaire au niveau d'une position correspondant à la position 27131/27132 du gène CYP3A5 comme défini par les séquences jointes du numéro d'enregistrement : AF280107.1, où la position 166220 a été numérotée +1 et la position 174832 +8613, et numéro d'enregistrement : AC005020.2, où la position 27341 a été numérotée +8614.

2. Polynucléotide selon la revendication 1 qui est de l'ADN ou à de l'ARN.

3. Vecteur comprenant un polynucléotide selon la revendication 1 ou 2.

4. Vecteur selon la revendication 3, dans lequel le polynucléotide est lié de manière opérationnelle à des séquences de contrôle de l'expression permettant l'expression dans des cellules procaryotes ou eucaryotes ou dans des fractions isolées de celles-ci.

5. Cellule hôte génétiquement modifiée avec le polynucléotide selon la revendication 1 ou 2, ou le vecteur selon la revendication 3 ou 4.

6. Procédé pour produire un variant moléculaire du polypeptide CYP3A5 comprenant :
(a) la culture de la cellule hôte selon la revendication 5 ; et
(b) la récupération de ladite protéine à partir de la culture.

7. Procédé pour produire des cellules capables d'exprimer un variant moléculaire du polypeptide CYP3A5 comprenant la modification génétique de cellules avec le polynucléotide selon la revendication 1 ou 2, ou le vecteur selon la revendication 3 ou 4.

8. Polypeptide codé par le polynucléotide selon la revendication 1 ou 2 ou susceptible d'être obtenu par le procédé selon la revendication 6 à partir de cellules produites par le procédé selon la revendication 7.

9. Anticorps se liant de manière spécifique au polypeptide selon la revendication 8.

10. Anticorps selon la revendication 9 qui est monoclonal ou polyclonal.

11. Animal transgénique non humain comprenant au moins un polynucléotide selon la revendication 1 ou 2, ou le vecteur selon la revendication 3 ou 4.

12. Animal transgénique non humain selon la revendication 11 qui est une souris, un rat ou à un dard-perche.

13. Support solide comprenant un ou une pluralité du polynucléotide selon la revendication 1 ou 2, le vecteur selon la revendication 3 ou 4, le polypeptide selon la revendication 8, l'anticorps selon la revendication 9 ou la cellule hôte selon la revendication 5 sous une forme immobilisée.

14. Support solide selon la revendication 13, ledit support solide étant une membrane, une puce de verre, de polypropylène ou de silicium, des billes ou à un ensemble de billes conjugués à des oligonucléotides, assemblé sur un substrat filtre optique.

15. Procédé *in vitro* pour identifier un polymorphisme, ledit procédé comprenant l'étape d'identification d'un polymorphisme en comparant la séquence d'acide nucléique d'un polynucléotide selon la revendication 1 ou 2 obtenu à partir d'un sous-groupe d'individus, le sous-groupe ne présentant pas de prévalence de cancer, avec la séquence d'acide nucléique d'un polynucléotide selon la revendication 1 ou 2 obtenu à partir d'une pluralité de sous-groupes d'individus, au moins un ou plusieurs sous-groupe(s) supplémentaires présentant une prévalence de cancer.

16. Procédé pour identifier et obtenir un promédicament ou un médicament capable de moduler l'activité d'un variant moléculaire d'un polypeptide CYP3A5 comprenant les étapes de:
(a) mise en contact du polypeptide selon la revendication 8, du support solide selon la revendication 13 ou 14, d'une cellule exprimant un gène de variant moléculaire comprenant un polynucléotide selon la revendication 1 ou 2 ou le vecteur selon la revendication 3 ou 4 en présence de composants capables de fournir un signal détectable en réponse à l'activité médicamenteuse, avec un composé à cribler vis-à-vis d'une activité de médicament ou de promédicament ; et
(b) détection de la présence ou l'absence d'un signal ou de l'augmentation ou de la diminution d'un signal généré à partir de l'activité du médicament ou du promédicament, l'absence, la présence, l'augmentation ou la diminution du signal étant indicative d'un médicament ou d'un promédicament putatif.

17. Procédé selon la revendication 16, dans lequel ladite cellule est une cellule selon la revendication 6, obtenue selon le procédé de la revendication 7 ou susceptible d'être obtenue grâce à l'animal transgénique non humain selon la revendication 11 ou 12.

18. Procédé pour identifier et obtenir un promédicament ou un médicament capable de moduler l'activité d'un variant moléculaire d'un polypeptide CYP3A5 comprenant les étapes suivantes :
(a) mettre en contact la cellule hôte selon la revendication 5, la cellule obtenue selon le procédé de la revendication 7, le polypeptide selon la revendication 8 ou le support solide selon la revendication 13 ou 14, avec la première molécule connue pour être liée par un polypeptide CYP3A5 et former un premier complexe dudit polypeptide et de ladite première molécule ;
(b) mettre en contact ledit premier complexe avec un composé devant être criblé, et
(c) mesurer si ledit composé déplace ladite première molécule à partir dudit premier complexe.

19. Procédé selon la revendication 18, dans lequel ladite étape de mesure comprend la mesure de la formation d'un second complexe de ladite protéine et dudit composé.

20. Procédé selon la revendication 18 ou 19, dans lequel ladite étape de mesure comprend la mesure de la quantité de ladite première molécule qui n'est pas liée à ladite protéine.

21. Procédé selon l'une quelconque des revendications 18 à 20, dans lequel ladite première molécule est marquée.

22. Procédé *in vitro* de diagnostic du cancer du foie ou d'une susceptibilité au cancer du foie, lié à la présence d'un variant moléculaire d'un gène CYP3A5 comprenant la détermination de la présence
(a) d'un polynucléotide choisi dans le groupe constitué de :
(i) un polynucléotide selon la revendication 1 ou 2 ;
(ii) un polynucléotide ayant un nucléotide supplémentaire au niveau d'une position correspondant à la position 27131/27132 du gène CYP3A5 comme défini par les séquences jointes numéro d'enregistrement : AF280107.1, où la position 166220 a été numérotée +1 et la position 174832 a été numérotée +8613, et numéro d'enregistrement : AC005020.2, où la position 27341 a été numérotée +8614 ; ou
(b) du polypeptide selon la revendication 8, dans un échantillon provenant d'un sujet.

23. Procédé selon la revendication 22, comprenant une PCR, une réaction de ligature en chaîne, une digestion de restriction, un séquençage direct, des techniques d'amplification des acides nucléiques, des techniques d'hybridation, une spectroscopie de masse ou des immunodosages.

24. Procédé pour détecter le polynucléotide selon la revendication 1 ou 2 dans un échantillon, comprenant les étapes de :
(a) mise en contact du support solide selon la revendication 13 ou 14 avec l'échantillon dans des conditions permettant l'interaction du polynucléotide selon la revendication 1 ou 2 avec les cibles immobilisées sur un support solide et ;
(b) détermination de la liaison dudit polynucléotide ou dudit gène auxdites cibles immobilisées sur un support solide.

25. Composition diagnostique comprenant le polynucléotide selon la revendication 1 ou 2, le vecteur selon la revendication 3 ou 4, le polypeptide selon la revendication 8 ou l'anticorps selon la revendication 9.

26. Composition pharmaceutique comprenant le polynucléotide selon la revendication 1 ou 2, le vecteur selon la revendication 3 ou 4, le polypeptide selon la revendication 8 ou l'anticorps selon la revendication 9.

27. Utilisation du polynucléotide selon la revendication 1 ou 2, du polypeptide selon la revendication 8 ou de l'anticorps selon la revendication 9 pour la préparation d'une composition diagnostique pour le diagnostic d'un cancer du foie chez un sujet.

28. Trousse de diagnostic pour la détection d'un polymorphisme d'un nucléotide unique, comprenant le polynucléotide selon la revendication 1 ou 2, le vecteur selon la revendication 3 ou 4, le polypeptide selon la revendication 8, l'anticorps selon la revendication 9, la cellule hôte selon la revendication 5, l'animal transgénique non humain selon la revendication 11 ou 12 ou le support solide selon la revendication 13 ou 14.
